(19)

Europäisches
Patentamt
European
Patent Office
Office européen
des brevets

(11) **EP 3 944 877 A1**

(12) **EUROPEAN PATENT APPLICATION**

(43) Date of publication:
**02.02.2022 Bulletin 2022/05**

(21) Application number: **20188451.7**

(22) Date of filing: **29.07.2020**

(51) International Patent Classification (IPC):
**A61P 31/14** *(2006.01)* **C07K 16/10** *(2006.01)*

(52) Cooperative Patent Classification (CPC):
**C07K 16/10; A61P 31/14;** C07K 2317/22;
C07K 2317/35; C07K 2317/569; C07K 2317/76;
C07K 2317/92; C07K 2319/70

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.
80539 München (DE)**

(72) Inventor: **The designation of the inventor has not yet been filed**

(74) Representative: **Weiss, Wolfgang
Weickmann & Weickmann
Patent- und Rechtsanwälte PartmbB
Postfach 860 820
81635 München (DE)**

(54) **NANOBODIES SARS-COV2**

(57) The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2, a strategy for an enhanced block of the homotrimeric viral spike proteins by symmetry-matching VHH-fusions, implementations of this strategy, as well as VHH antibodies for sensitive detection of SARS-CoV2-infections.

## Description

### Field of the invention

The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2, a strategy for an enhanced block of the homotrimeric viral spike proteins by symmetry-matching VHH-fusions, implementations of this strategy, as well as VHH antibodies for sensitive detection of SARS-CoV2-infections.

### Background of the invention

The COVID-19 pandemics has plunged the world into a nearly unprecedented medical and economic crisis - mainly because only insufficient means for treatment are available. COVID-19 is caused by SARS-CoV-2 - a recently evolved virus belonging to the *Coronaviridae.* The virus infects the upper airways initially but can also cause pneumonia and more systemic infections with damages to lungs, kidneys and/or the circulatory system. Approximately 5% of the WHO-documented infections had a lethal outcome so far, whereby the probability of a severe course increases with the age of the individuals and with risk factors such as obesity, diabetes, and chronic lung or kidney diseases.

SARS-CoV-2 enters cells with the help of its homotrimeric spike protein, which docks to the ACE2 receptor of the host and subsequently mediates fusion between the viral and host cell membranes to allow entry of the viral genomic RNA into the now infected host cell. The host cell is then re-programmed to produce viral proteins, to replicate the viral RNA, and package replicated RNA into the next generation of viral particles, which are then shed in large numbers off the host cell to infect further cells.

The adaptive immune system can control viral infections by a T-cell mediated cytotoxic response that eliminates infected cells and/ or by B-cells producing antibodies that bind to the virus's surface and block infection (for a textbook view see: Murphy and Weaver, 2016). Such infection-blocking antibodies are referred to as neutralizing ones. Typically, however, only a small fraction of produced anti-viral antibodies also has neutralizing properties.

Immunoglobulin G (IgG) is not only the most abundant class of antibodies but also the most important one in terms of long-term immunity. IgGs are built from four polypeptide chains, two light and two heavy ones. They contain two antigen-binding sites formed each by one heavy and one light chain. Such a composite binding site can confer a virus-neutralizing activity. In addition, IgGs comprise an Fc fragment, formed only by the two heavy chains. The Fc fragment is a prototypic immune effector domain that can interact with cell surface receptors (called Fc receptors) as well as with components of the complement system.

In some cases, however, antibodies might even help viral entry by an effect called ADE (for antibody-dependent enhancement) that is mediated by Fc-receptor interactions (Kliks et al., 1989; Littaua et al., 1990). Likewise, the immune system might aggravate the condition of patients with COVID-19 by a systemic over-reaction that manifests itself as a cytokine storm (Chen et al., 2020b; Mehta et al., 2020) or an inappropriate complement activation (Magro et al., 2020).

An effective immune response will either prevent a virus from establishing an infection in the first place or reduce, after an initial infection, the virus-load to non-pathogenic levels. An insufficiently controlled SARS-CoV-2 infection, however, might have a lethal outcome. In survivors, it often causes permanent damage (e.g. lung dysfunction) and impairs quality of life. An efficient anti-viral therapy would be highly desirable, ideally with orthogonal lines of treatment that target distinct steps of the viral infection cycle independently.

Remdesivir represents so far one, still experimental line of treatment. This nucleoside analogue inhibits the SARS-CoV-2 RNA polymerase. As of May 2020, clinical studies gave mixed outcomes: from no decrease in mortality to a modest reduction in hospitalization time (Chen et al., 2020a).

Another treatment option for severely infected patients is passive immunization with polyclonal antibodies/blood plasma from individuals who have already recovered from COVD-19 (Duan et al., 2020). This approach is considered as a last resort and generally has several drawbacks, such as the risk of ADE in case of an insufficient neutralization, limited availability of sera from convalescent individuals, inevitable batch-to-batch variability of plasma samples, the risk of transmitting other infectious diseases, and all other potential adverse effects when large amounts of antibodies of mixed specificity are transferred from one person to another.

A more advanced approach is the isolation of monoclonal antibodies from SARS-CoV-2 infected or convalescent patients (Wang et al., 2020). These can be characterized in-depth and produced reproducibly in a recombinant form. This solves some of the issues raised for the plasma-therapy mentioned above. However, monoclonal antibodies are extremely costly to produce, making it hard to imagine that it will be feasible to scale up production to levels needed for the treatment of large numbers of patients. In addition, immunological side effects, such as aggravating cytokine storms, inappropriate complement activation, or ADE, cannot be ruled out either, mainly due to the Fc region present in human/ humanized antibodies.

Virus diagnostics is another important line for dealing with local, epidemic or even pandemic virus infections. Once infected individuals have been identified, they can be isolated from the still healthy part of the population and thereby a spread of the disease be contained. Also, a proper diagnosis is essential for a targeted treatment, for monitoring the course of an infection as well as for monitoring the success of a treatment. A SARS-CoV-2 infection can be diagnosed by three principles: (1) by a detection of viral RNA e.g. through RT PCR. This is potentially very sensitive and reports acute infections; (2) by a detection of anti-viral antibodies that patients have produced in response to an infection. This allows potentially higher throughputs but yields false negative results at an early stage of infection, allows no conclusions as to the current viral load and cannot distinguish between past and ongoing infections. (3) The third possibility is to detect viral proteins by means of anti-viral antibodies. This way of detection reports on acute infections but is likely of limited sensitivity, at least when bulk detection in ELISA or lateral flow setups is being used, and when patient's antibodies mask the respective epitopes.

Camelids are unusual in that they contain heavy chain-only antibodies, whose far simpler antigen-binding domain can easily be cloned and recombinantly expressed in bacteria or yeast (Arbabi Ghahroudi et al., 1997). Such isolated antigen-binding domains are then referred to as nanobodies or VHH antibodies (variable domain of heavy homodimer antibodies). They are just ~14kDa in size - about one-tenth of a traditional IgG molecule. They have found numerous useful applications, for example as crystallization chaperones (Rasmussen et al., 2011; Chug et al., 2015), as tools in affinity chromatography, for localizing proteins in cells or as an animal-friendly substitute for secondary antibodies (Pleiner et al., 2015; Pleiner et al., 2018).

**Summary of the invention**

A first aspect of the invention relates to an VHH antibody recognizing the SARS-CoV-2 spike protein S1 domain, particularly the receptor-binding domain (RBD) of the SARS-CoV-2 S1 domain.

The VHH antibody is selected from the group of VHH antibodies comprising a CDR3 sequence as disclosed herein or a related CDR3 sequence. In certain embodiments, the VHH antibody is selected from the group of VHH antibodies comprising a combination of CDR1, CDR2 and CDR3 sequences as disclosed herein or a related combination of CDR1, CDR2 and CDR3 sequences. In certain embodiments, the VHH antibody is selected from the group of antibodies comprising a VHH sequence as disclosed herein or a VHH sequence.

In certain embodiments, the VHH antibody as described above is covalently or non-covalently conjugated to a heterologous moiety, which may be selected from a labeling group, a capture group or an effector group.

In certain embodiments, the VHH antibody as described above is fused to a heterologous polypeptide moiety, which may be selected from a multimerization module, e.g. dimerization, trimerization or tetramerization module. In particular embodiments, the multimerization module is trimerization module.

In certain embodiments, the VHH antibody is conjugated to one or several polymer moieties, preferably hydrophilic polymer moieties, such as polyethylene glycol (PEG), to increase the molecular weight of the antibody conjugate and, thus, delay renal clearance. The molecular weight of a polymer moiety may vary over a broad range, for example in the range of about 5 kDa to about 80 kDa. Such coupling may be performed through e.g. amino or carboxyl groups already present in the VHHs and/or through the side chains of engineered lysine, aspartic acid, glutamic acid or cysteine residues and involve known chemistries for forming amide bonds, secondary amine bonds or thioether bonds.

In certain embodiments, the VHH antibody as described above is directed against the SARS-CoV-2 spike protein receptor-binding domain (RBD). In certain embodiments, the VHH antibody is capable of virus neutralization.

A further aspect of the present invention relates to a set of two or more different VHH antibodies as described above, wherein the different VHH antibodies may recognize different epitopes, particularly different epitopes on the RBD, and more particularly non-overlapping epitopes on the RBD.

A VHH antibody as described above is suitable for use in medicine, e.g. human medicine, particularly for use in therapy, e.g. in the prevention or treatment of a disorder caused by and/or associated with an infection with SARS-CoV-2, or in diagnostics, e.g. for detecting SARS-CoV-2 virus or viral components in a patient sample, e.g. in a body fluid or tissue sample.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for recombinant production of a VHH antibody as described above, comprising cultivating a cell or an organism as described above in a suitable medium and obtaining the VHH antibody from the cell or organism or from the medium.

Still a further aspect of the invention relates to a method for the prevention or treatment of a disorder caused by and/or associated with an infection with SARS-CoV-2, comprising administering an effective dose of the VHH antibody as described above or the set of at least two different VHH antibodies as described above to a subject in need thereof, particularly to a human subject infected with SARS-CoV-2 or at risk from being infected with SARS-CoV-2.

A list of VHH antibodies of the present invention and their utility for virus detection and virus neutralization is shown in the following Table 1:

Table 1

| VHH | Anti | Spike protein detection in | | Virus neutralization |
|---|---|---|---|---|
| | | transfected cells* | infected cells** | |
| Re5A08 | RBD | Yes | Yes | Yes |
| Re5B06 | RBD | Yes | Yes | Yes |
| Re5C01 | RBD | Yes | Yes | Yes |
| Re5C08 | RBD | Yes | weak | Yes |
| Re5D06 | RBD | Yes | Yes | Yes |
| Re5E03 | RBD | ND | ND | Yes |
| Re5E11 | RBD | ND | ND | Yes |
| Re5F10 | RBD | ND | ND | Yes |
| Re5F11 | RBD | ND | ND | Yes |
| Re5G05 | RBD | ND | ND | Yes |
| Re6A11 | RBD | Yes*** | ND | Yes |
| Re6B02 | RBD | Yes | Yes | Yes |
| Re6B06 | RBD | Yes | weak | Yes |
| Re6B07 | RBD | Yes | weak | Yes |
| Re6D06 | RBD | Yes | Yes | Yes |
| Re6D08 | RBD | Yes | ND | Yes |
| Re6D09 | RBD | Yes | Yes | Yes |
| Re6E11 | RBD | ND | ND | Yes |
| Re6F06 | RBD | ND | ND | Yes |
| Re6G03 | RBD | ND | ND | Yes |
| Re6H06 | RBD | ND | ND | Yes |
| Re6H10 | RBD | ND | ND | Yes |
| Re7A01 | RBD | Yes | Yes | Yes |
| Re7B01 | RBD | Yes | Yes | Yes |
| Re7D05 | RBD | Yes | weak | No |
| Re7E02 | RBD | Yes | Yes | Yes |
| Re7H02 | RBD | Yes | Yes | Yes |
| Re8A03 | S1ΔRBD | Yes | weak | ND |
| Re8A06 | S1ΔRBD | Yes | weak | ND |
| Re8C06 | S1ΔRBD | Yes | Yes | ND |
| Re8E12 | S1ΔRBD | Yes | Yes | ND |
| Re8F03 | S1ΔRBD | Yes | Yes | ND |
| Re9B09 | RBD | Yes | Yes | Yes |
| Re9B10 | RBD | Yes | Yes | Yes |

(continued)

| VHH | Anti | Spike protein detection in | | Virus neutralization |
|---|---|---|---|---|
| | | transfected cells* | infected cells** | |
| Re9C07 | RBD | Yes | Yes | Yes |
| Re9C08 | RBD | Yes | Yes | Yes |
| Re9D02 | RBD | weak | weak | Yes |
| Re9G05 | RBD | Yes | Yes | Yes |
| Re9G12 | RBD | weak | weak | Yes |
| Re9H01 | RBD | Yes | Yes | Yes |
| Re10B02 | RBD | ND | ND | Yes |
| Re10B10 | RBD | Yes | Yes | Yes |
| Re10F10 | RBD | Yes | Yes | Yes |
| Re11C10 | RBD | Yes | Yes | Yes |
| Re11E11 | RBD | Yes | Yes | Yes |
| Re11F07 | RBD | Yes | Yes | Yes |
| Re11F11 | RBD | Yes | Yes | Yes |
| Re11G09 | RBD | Yes | Yes | Yes |
| Re11H04 | RBD | weak | weak | Yes |
| * SARS-CoV2 spike protein detection in transiently transfected HeLa cells with 5 minutes fixation with 4% PFA; ** in SARS-CoV-2 infected Vero E6 cells with 120 minutes fixation with 4% PFA. *** Sensitive detection only with trimerized VHH. ND, not determined. | | | | |

The variable regions (CDRs) of each VHH antibody of Table 1 as well as assignment of SEQ ID NO.s for VHHs and CDRs are listed in the following Table 2:

**Table 2**

| VHH | SEQ ID NO. | CDR1 | SEQ ID NO. | CDR2 | SEQ ID NO. | CDR3 | SEQ ID NO. |
|---|---|---|---|---|---|---|---|
| Re5A08 | 1 | HTFTANRMG | 2 | FVAAINWGGDSTNYV | 3 | AARNHVTGEFDSW | 4 |
| Re5B06 | 5 | SIRSIYATV | 6 | WVGSITSSNVTTYA | 7 | NVHFASEYSDYAQ IQ | 8 |
| Re5C01 | 9 | RTFSSYAMG | 10 | FVATISWSGGTTNYA | 11 | YAVSSGSDYDGGMDYW | 12 |
| Re5C08 | 13 | RTFNDYNMV | 14 | FVAAIKWNGGNTSYA | 15 | YTVGPEGDYW | 16 |
| Re5D06 | 17 | ITLDYYAIG | 18 | GVSRIRSSDGSTNYA | 19 | AYGPLTKYGSSWYWPYEYDYW | 20 |
| Re5E03 | 21 | FTLDYYAIG | 22 | GVSCISNSDGSTRYA | 23 | AGGPQTYYSGSYYYTCAEGAMDYW | 24 |
| Re5E11 | 25 | FTLDYYAIG | 26 | GVSCISSDGRTYYA | 27 | ATAPLTYYSGSWYLTCNSDAMDYW | 28 |
| Re5F10 | 29 | FTFSSFAMG | 30 | WVATITITGGSTNYA | 31 | NPDPGCRR | 32 |
| Re5F11 | 33 | SISSYRMG | 34 | LVAFITIGGITDYI | 35 | NADPPLFNW | 36 |
| Re5G05 | 37 | FTATSYAMG | 38 | WVATITSTGGNTNYA | 39 | NPDPGCDW | 40 |
| Re6A11 | 41 | RTFSNDALG | 42 | FVAAINWNSGTYYA | 43 | AAASDYGLPREDFLYDYW | 44 |
| Re6B02 | 45 | FTLDYYAIG | 46 | GVSRIRSSDGSTNYA | 47 | AYGPLTKYGSSWYWPYEYDYW | 48 |
| Re6B06 | 49 | RAFSSAPMS | 50 | FVASVSWSGDSTNYA | 51 | KRGPYW | 52 |
| Re6B07 | 53 | FTLDYYAIG | 54 | GVSYIRSSDGTTYYA | 55 | AADEAYYSELGWESPWGWSYW | 56 |
| Re6D06 | 57 | RMFGVYRMG | 58 | FVAGISTSVGTTNYA | 59 | AARDPTTYEYDYW | 60 |
| Re6D08 | 61 | RTFSSYAMG | 62 | FVATINWSGDSTYYA | 63 | AAVVDPSPTYYSGKYYPPRVEYW | 64 |

(continued)

| VHH | SEQ ID NO. | CDR1 | SEQ ID NO. | CDR2 | SEQ ID NO. | CDR3 | SEQ ID NO. |
|---|---|---|---|---|---|---|---|
| Re6D09 | 65 | RTFNNYNMV | 66 | FVAAINWNGGSTSYA | 67 | YTVGPEGDYW | 68 |
| Re6E11 | 69 | LTLDYYAIG | 70 | GVSCISSRDGSTMYA | 71 | AATPTTYYSGSYYYTCSPEGYDYW | 72 |
| Re6F06 | 73 | FTFSNYAMG | 74 | FVAVITITGSNTNYA | 75 | NPDPGCESQ | 76 |
| Re6G03 | 77 | RTFSTYRMA | 78 | FVAGINWSDGTTSYK | 79 | NAHLSTGQEGPGEYFGMDYW | 80 |
| Re6H06 | 81 | VTLDYYAIG | 82 | GVSCTSSSDGSTYYA | 83 | AVVPQTYYGGKYYSQCTANGMDYW | 84 |
| Re6H10 | 85 | FTFSSYAMG | 86 | FVAVITITGGSTNYA | 87 | NPDPGCRGG | 88 |
| Re7A01 | 89 | RTFSSYAMG | 90 | FVATISFSGSTSYA | 91 | HAVTRASDQDGGMDYW | 92 |
| Re7B01 | 93 | FTLDYYAIG | 94 | GVSRIRSNDGSTDYA | 95 | AYGPLTKYGSSWYWPYEYDYW | 96 |
| Re7D05 | 97 | RTFSSYAMG | 98 | FVATISWSGGSTSYA | 99 | NAVTHHSDQDGGMDYW | 100 |
| Re7E02 | 101 | FTLDYYAIG | 102 | GVSYIRSSDGTTYYA | 103 | AADEAYYSELGWESPWGWSYW | 104 |
| Re7H02 | 105 | RAFESAPMS | 106 | FVASVSWSGDSTNYA | 107 | KRGPYW | 108 |
| Re8A03 | 109 | RITGFNGMG | 110 | LVASITNGGITKYA | 111 | YFWRPEFPNLYW | 112 |
| Re8A06 | 113 | SIFSINAMG | 114 | LVAAMGSSGWINYA | 115 | RGTGGVGPTSADYW | 116 |
| Re8C06 | 117 | RTDTIYNMG | 118 | FVAAISWSDGKTTFA | 119 | AAKAFLVAGRSLEEYDYS | 120 |
| Re8E12 | 121 | FTSDVDLRNYLVS | 122 | LVAAITPTVISGGNTNYA | 123 | KVGVYW | 124 |
| Re8F03 | 125 | LTSYVDLRNYLVS | 126 | LVAAITPTAITGGSTNYA | 127 | KVGVYW | 128 |
| Re9B09 | 129 | FTLDYYAIG | 130 | GVSRISSSDGSTDYA | 131 | ATVPGTYYSGNWYYTWHPEAVDYW | 132 |

(continued)

| VHH | SEQ ID NO. | CDR1 | SEQ ID NO. | CDR2 | SEQ ID NO. | CDR3 | SEQ ID NO. |
|---|---|---|---|---|---|---|---|
| Re9B10 | 133 | RMFGVYRMG | 134 | FVAGISTSVGTTNYA | 135 | AARDPTTYEYDYW | 136 |
| Re9C07 | 137 | RTFSRYAMG | 138 | FVAAITWNADTTYYA | 139 | AAGGNHYYSRSYYSSLEYDHW | 140 |
| Re9C08 | 141 | NIFGITAWD | 142 | LVAHITSRGDTYYL | 143 | YLRTFGPPNDHW | 144 |
| Re9D02 | 145 | RTFSNYAMG | 146 | FVAAISWGGDTTYYA | 147 | AADRGLSYYYDRVTEYDYW | 148 |
| Re9G05 | 149 | NISSITAWD | 150 | LVAHITSRGDTMYL | 151 | YLRTFGPPYDYW | 152 |
| Re9G12 | 153 | RTFSSYVMG | 154 | FVAHISWSGDSTYYA | 155 | AADRGASYYYTWASEYNYW | 156 |
| Re9H01 | 157 | NIFSINAMG | 158 | LVAFITSRGSTNYT | 159 | RGGYSDYDIYFGSW | 160 |
| Re10B02 | 161 | SISSYRMG | 162 | LVAFITIGGITDYI | 163 | NADPPLFNW | 164 |
| Re10B10 | 165 | FTLDYYAIG | 166 | GVSRIRSSDGSTTYA | 167 | AYGPLTKYGSSVWWPYEYDYW | 168 |
| Re10F10 | 169 | FTLDYYAIG | 170 | GVSRIRNNDGSTDYA | 171 | AYGPLTKYGSSVWWPYEYDYW | 172 |
| Re11C10 | 173 | RTLDNYNAV | 174 | FVAAINWNGSNTSYG | 175 | YTVGPEGDYW | 176 |
| Re11E11 | 177 | RTFNNYNIV | 178 | FVAAINWNGGSTSYA | 179 | YTVGPEGDYW | 180 |
| Re11F07 | 181 | RAFSSGTMG | 182 | FVATISWSGGSTSYA | 183 | YAVSSGSDYDGGMDYW | 184 |
| Re11F11 | 185 | FTFSNYHMS | 186 | LVADITSGGDYTHYA | 187 | HVRIFGPGFPVDYR | 188 |
| Re11G09 | 189 | SIFNIYRMA | 190 | KVAIITTYGLTDYA | 191 | NTDPPDLGPGYW | 192 |
| Re11H04 | 193 | FTLDYYTIA | 194 | GVSCISGNDGSTYYA | 195 | AADRGESYYPFRPSEYHYW | 196 |

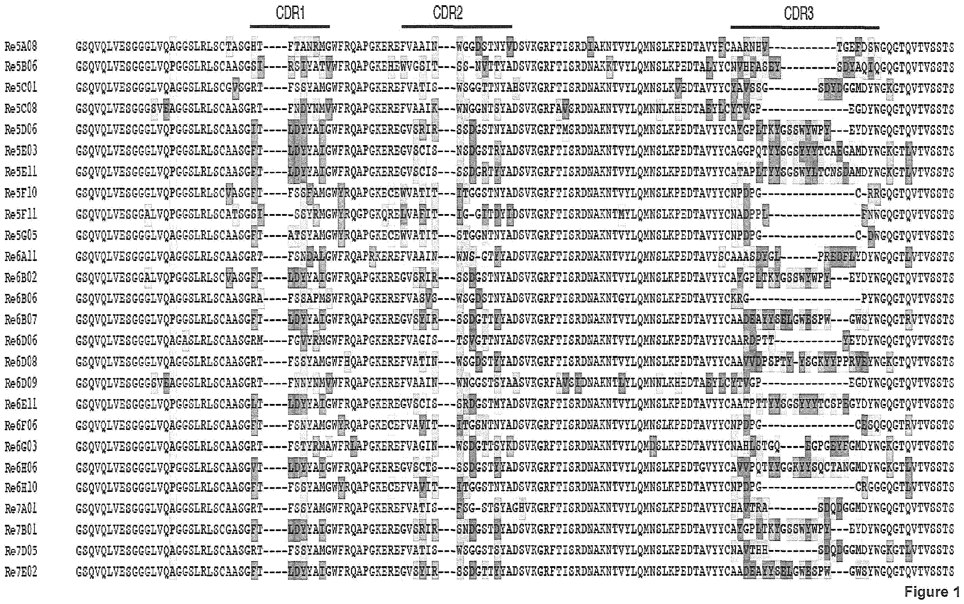

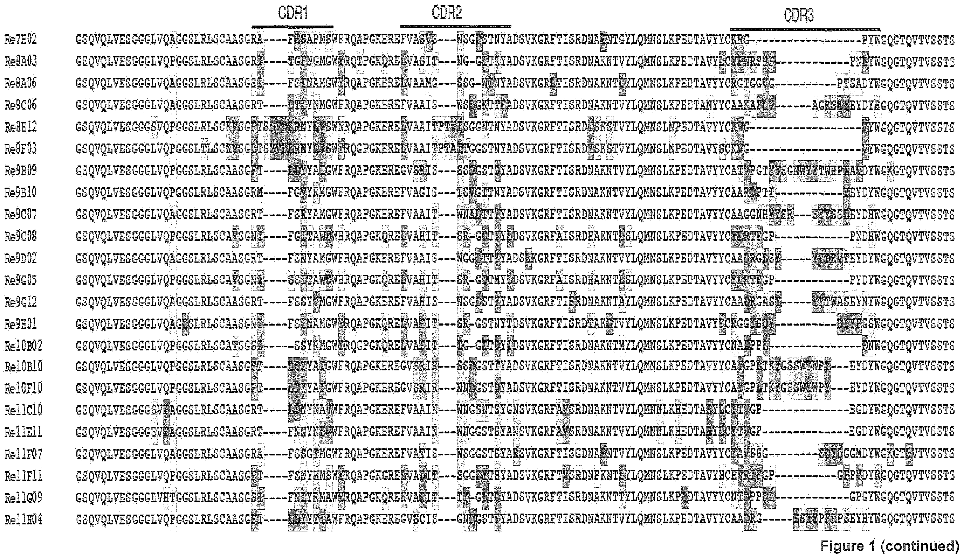

A list of complete sequences of VHH antibodies is shown at the end of the specification and in Figure 1.

A further independent aspect of the present invention relates to a homomultimeric VHH antibody, particularly a homotrimeric VHH antibody. The homomultimeric VHH antibody comprises a plurality of individual subunits, particularly 3 individual subunits. An individual subunit may be a fusion polypeptide comprising a VHH antibody, e.g. a VHH antibody as described above, directly linked or linked via a spacer to a multimerization module, particularly to a trimerization module.

In certain embodiments, an individual subunit may comprise at its N-terminus the VHH antibody and at its C-terminus the multimerization module, optionally linked via a peptide spacer. In other embodiments, an individual subunit may comprise the multimerization module at its N-terminus and the VHH antibody at its C-terminus, optionally linked via spacer.

In certain embodiments, the spacer is a peptide spacer having a length of about 5 to about 100 amino acids, particularly of about 10 to about 50 amino acids.

In certain embodiments, the peptide spacer comprises amino acids selected from glycine, serine, threonine, alanine, proline, glutamate and aspartate. In certain embodiments, the spacer is composed to at least about 50%, about 60%, about 70%, about 80%, about 90% or completely of these amino acids.

In certain embodiments, the spacer is composed of a succession of tripeptides, whose first two residues are selected from glycine, serine, threonine, alanine and proline, and the third amino acid is selected from glutamate and aspartate. In certain embodiments, each tripeptide differs in at least one amino acid from adjacent tripeptides.

In certain embodiments, the linker is substantially free or free from amino acids selected from basic amino acids lysine and arginine, as well as from amino acids selected from tryptophan, tyrosine, phenylalanine, methionine, leucine, isoleucine, valine, histidine and cysteine.

In certain embodiments, the homomultimeric VHH antibody is a homotrimeric VHH antibody.

In certain embodiments of this aspect, the VHH antibody portion of the homomultimeric VHH antibody is directed to the SARS-CoV-2 spike protein S1 domain, particularly the receptor-binding domain (RBD) of the SARS-CoV-2 S1 domain. In particular embodiments, the VHH antibody portion of the homomultimeric VHH antibody is a VHH antibody as described above, i.e. an antibody comprising a CDR3 sequence as shown in Table 2 or a sequence related thereto, a combination of CDR1, CDR2 and CDR3 sequences, as shown in Table 2 or a related sequence, or a VHH sequence as shown in Figure 1 or a related sequence.

According to this aspect, the VHH antibody portion may be directed to another target structure, particularly a target structure having a threefold-rotational symmetry, e.g. viral or non-viral target structure comprising a homotrimeric protein or protein assembly.

**Embodiments of the invention**

In the following, specific embodiments of the invention are disclosed as follows:

1. A VHH antibody recognizing the SARS-CoV-2 spike protein S1 domain, particularly the receptor-binding domain (RBD) of the SARS-CoV-2 S1 domain comprising

(a) a CDR3 sequence as shown in SEQ ID NO. 20, 4, 8, 12, 16, 24, 28, 32, 36, 40, 44, 48, 52, 56, 60, 64, 68, 72, 76, 80, 84, 88, 92, 96, 100, 104, 108, 112, 116, 120, 124, 128, 132, 136, 140, 144, 148, 152, 156, 160, 164, 168, 172, 176, 180, 184, 188, 192 or 196,

(b) a CDR3 sequence, which has an identity of at least 80% or at least 90% to a CDR3 sequence of (a), or

(c) a VHH antibody, which competes with a VHH antibody of (a) for the binding to the SARS-CoV-2 spike protein S1 domain, particularly the receptor-binding domain (RBD) of the SARS-CoV-2 S1 domain.

2. The VHH antibody according to embodiment 1 comprising

(a) a combination of CDR1, CDR2 and CDR3 sequences as shown in SEQ ID NO. 18-20, 2-4, 6-8, 10-12, 14-16, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108, 110-112, 114-116, 118-120, 122-124, 126-128, 130-132, 134-136, 138-140, 142-144, 146-148, 150-152, 154-156, 158-160, 162-164, 166-168, 170-172, 174-176, 178-180, 182-184, 186-188, 190-192 or 194-196,

(b) a combination of CDR1, CDR2 and CDR3 sequences which has an identity of at least 80%, at least 90% or at least 95% to a combination of CDR1, CDR2 and CDR3 sequences of (a), or

(c) a VHH antibody, which competes with a VHH antibody of (a) for the binding to the SARS-CoV-2 spike protein S1 domain, particularly the receptor-binding domain (RBD) of the SARS-CoV-2 S1 domain.

3. The VHH antibody according to embodiment 1 or 2 comprising

(a) a VHH sequence as shown in SEQ ID NO. 17, 1, 5, 9, 13, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 177, 181, 185, 189 or 193,
(b) a sequence which has an identity of at least 70%, at least 80%, at least 90%, at least 95% or at least 99% to a VHH sequence of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

4. The VHH antibody of any one of embodiments 1-3, which recognizes the receptor-binding domain (RBD) of the SARS-CoV-2 S1 domain.

5. The VHH antibody of any one of embodiments 1-4, which is capable of virus neutralization.

6. The VHH antibody of any one of embodiments 1-5, which is covalently or non-covalently conjugated to a heterologous moiety.

7. The VHH antibody of embodiment 6, which is conjugated to a labeling group, a capture group or an effector group.

8. The VHH antibody of embodiment 6 or 7, wherein the heterologous moiety is selected from a fluorescence group, biotin, an enzyme such as a peroxidase, phosphatase or luciferase, a hapten, an affinity tag, or a nucleic acid such as an oligonucleotide.

9. The VHH antibody of any one of embodiments 1-5, which is fused to a heterologous polypeptide moiety.

10. The VHH antibody of embodiment 9, wherein the heterologous polypeptide moiety is a multimerization module, e.g. dimerization, trimerization or tetramerization module.

11. The VHH antibody of embodiment 10, which is a homo-trimerized VHH antibody fused to a trimerization module, e.g. a collagen trimerization moiety, particularly a human collagen moiety, a lung surfactant protein D moiety.

12. The VHH antibody of any of embodiments 9-11, which is fused to a heterologous polypeptide moiety directly or via a spacer, e.g. a spacer having a chain length of 1-50 amino acids, particularly selected from Gly, Ser, Glu and/or Asp.

13. The VHH antibody of any one of embodiments 1-12, which is non-glycosylated.

14. The VHH antibody of any of embodiments 1-13, which is conjugated to one or several polymer moieties, preferably hydrophilic polymer moieties, such as polyethylene glycol (PEG).

15. A set of two or more different VHH antibodies of any of embodiments 1-14.

16. The set of embodiment 15, wherein the different VHH antibodies recognize different epitopes on the RBD, particularly non-overlapping epitopes on the RBD.

17. The VHH antibody of any one of embodiments 1-14 or the set of embodiment 15 or 16 for use in medicine, e.g. human medicine, particularly for use in therapy or diagnostics.

18. The VHH antibody of any one of embodiments 1-14 or the set of embodiment 15 or 16 for the prevention or treatment of a disorder caused by and/or associated with an infection with SARS-CoV-2.

19. The VHH antibody of any one of embodiments 1-14 or the set of embodiment 15 or 16 for the prevention or treatment of Covid-19 in a human subject.

20. The VHH antibody of any one of embodiments 1-14 or the set of embodiment 15 or 16 for the detection of SARS-CoV-2 virus comprising

(i) a set of at least two VHH antibodies recognizing different, particularly non-overlapping epitopes on the RBD,

(ii) a set of at least two VHH antibodies comprising a set of capturing antibodies conjugated to a capturing moiety and a set of labeling antibodies conjugated to a labeling moiety, and/or

(iii) a set of at least two VHH antibodies comprising a first set of labeling antibodies conjugated to a first labeling moiety, and a second set of labeling antibodies conjugated to a second labeling moiety, wherein the first labeling moiety is different from the second labeling moiety, wherein the first and the second labeling moieties are particularly selected from two spectrally different fluorescence labeling moieties.

21. Use of the VHH antibody of any one of embodiments 1-14 or the set of embodiment 15 or 16 for detecting SARS-CoV-2 virus or viral components in a patient sample, e.g. in a body fluid or tissue sample.

22. Use of the VHH antibody of any one of embodiments 1-14 or the set of embodiment 15 or 16 for detecting SARS-CoV-2 virus or viral components in a virus culture, e.g. in a culture of SARS-CoV-2 or variants thereof including pseudo typed variants, or in a genetically modified organism, e.g. in an organism producing viruses or viral components.

23. The use of embodiment 22 for monitoring, quantification and/or quality control during production of viruses or viral components.

24. A nucleic acid molecule encoding a VHH antibody according to any one of embodiments 1-14, preferably in operative linkage with a heterologous expression control sequence.

25. A vector comprising a nucleic acid molecule according to embodiment 24.

26. A recombinant cell or non-human organism transformed or transfected with a nucleic acid molecule according to embodiment 24 or a vector according to 25.

27. The cell or organism of embodiment 26 which is selected from a bacterium such as *E. coli Bacillus sp.,* a unicellular eukaryotic organism such as yeast or *Leishmania,* an insect cell, a mammalian cell or a plant cell.

28. A method for recombinant production of a VHH antibody of any one of embodiments 1-14, comprising cultivating a cell or an organism of embodiment 26 or 27 in a suitable medium and obtaining the VHH antibody from the cell or organism or from the medium.

29. A method for the prevention or treatment of a disorder caused by and/or associated with an infection with SARS-CoV-2, comprising administering an effective dose of the VHH antibody of any one of embodiments 1-14 or the set of embodiment 15 or 16 to a subject in need thereof, particularly to a human subject.

30. A homotrimeric VHH antibody comprising 3 VHH antibody subunits recognizing the same target structure, wherein each VHH antibody subunit comprises a VHH antibody directly linked or linked via a spacer to a trimerization module.

31. The homotrimeric VHH antibody of embodiment 30, which comprises three identical VHH antibody subunits.

32. The homotrimeric VHH antibody of embodiment 30 or 31 wherein the VHH antibody is directed to a target structure having a threefold-rotationally symmetric subunit structure.

33. The homotrimeric VHH antibody of any one of embodiments 30-32 wherein the VHH antibody is directed to a target structure comprising a homo-trimeric protein or protein assembly.

34. The homotrimeric VHH antibody of any one of embodiments 30-33 wherein the target structure is a viral protein or protein assembly, particularly a homo-trimeric viral protein or protein assembly, more particularly selected from a Coronavirus trimeric spike protein, an Orthomyxovirus trimeric HA protein, a Paramyxovirus trimeric fusion protein, a Dengue fever virus or Zikavirus trimeric or higher order protein assembly, a Herpesvirus trimeric gB fusion protein or a HIV trimeric gp120 protein.

35. The homotrimeric VHH antibody of any one of embodiments 30-34 wherein the target structure is the SARS-CoV-2 spike protein S1 domain, particularly the receptor-binding domain (RBD) of the SARS-CoV-2 S1 domain.

36. The homotrimeric VHH antibody of embodiment 35 wherein the VHH antibody is selected from the VHH antibodies according to any one of embodiments 1-5.

37. The homotrimeric VHH antibody of any one of embodiments 30-36 wherein the target structure is a non-viral protein or protein assembly, particularly a member of the TNF Ligand superfamily or a member of the TNF Receptor superfamily.

38. The homotrimeric VHH antibody of any one of embodiments 30-37 wherein each subunit comprises a VHH antibody linked via a spacer to a trimerization module.

39. The homotrimeric VHH antibody of embodiment 38 wherein the spacer has a length of about 5 to about 100 amino acids, particularly of about 10 to about 50 amino acids.

40. The homotrimeric VHH antibody of embodiment 38 or 39 wherein the spacer comprises amino acids selected from Gly, Ser, Ala, Thr, Pro, Asp and Glu, particularly in an amount of at least about 50%, about 60%, about 70%, about 80%, about 90% or 100%.

41. The homotrimeric VHH antibody of any one of embodiments 38-40 wherein the spacer comprises a succession of tripeptides, wherein the first and the second amino acid are selected from Gly, Ser, Ala, Thr, and Pro, and the third amino acid is selected from Asp and Glu.

42. The homotrimeric VHH antibody of any one of embodiments 38-41 wherein the spacer is substantially free or free of amino acids selected from Lys, Arg, Trp, Tyr, Phe, Met, Leu, Ile, Val, His and Cys.

43. The homotrimeric VHH antibody of any one of embodiments 30-42 wherein the trimerization module is selected from:

(a) a collagen trimerization domain, e.g. the NC1 domain of collagen XV, the NC1 domain of collagen XVIII or the NC1 domain of collagen X, or the lung surfactant protein D.

(b) a trimerization domain which has an identity of at least 80%, particularly at least 90% to a trimerization domain of (a).

44. A nucleic acid molecule encoding a homotrimeric VHH antibody according to any one of embodiments 30-43, preferably in operative linkage with a heterologous expression control sequence.

45. A vector comprising a nucleic acid molecule according to embodiment 44.

46. A recombinant cell or non-human organism transformed or transfected with a nucleic acid molecule according to embodiment 44 or a vector according to 45.

46. The homotrimeric VHH antibody of any one of embodiments 30-47, which is non-glycosylated.

47. The homotrimeric VHH antibody of any one of embodiments 30-47, which is conjugated to one or several polymer moieties, preferably hydrophilic polymer moieties, such as polyethylene glycol (PEG).

48. The homotrimeric VHH antibody of any one of embodiments 30-43, 46 or 47 for use in medicine, e.g. human medicine, particularly for use in therapy or diagnostics.

**Detailed description of the invention**

According to the present invention, SARS-CoV-2-neutralising VHH antibodies are provided, which are suitable for the effective treatment of COVID-19 patients. Specifically, the invention provides VHH antibodies that neutralize virus already at a very low concentration. Further, the invention provides multimeric, particularly trimeric VHH antibodies for enhancing the potency of VHH antibodies, which may also be directed against other target structures, e.g. for blocking infections by other viruses.

**SARS-CoV-2-neutralising VHH antibodies**

The primary aim of this aspect of the invention has been to generate, select, engineer and optimize SARS-CoV-2-neutralising VHH antibodies for effective treatment of COVID-19 patients. Specifically, we aimed for VHH antibodies that are capable of virus neutralization already at a very low concentration.

The present invention relates to a VHH antibody, which is a monovalent heavy chain-only antibody comprising a CDR1 domain, a CDR2 domain and a CDR3 domain linked by framework regions including, but not being limited to, whole VHH antibodies, e.g. native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention. The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing. According to the present invention, sequences related to the above sequences are encompassed. These related sequences are defined by having a minimum identity to a specifically indicated amino acid sequence, e.g. a CDR or VHH sequence. This identity is indicated over the whole length of the respective reference sequence and may be determined by using well known algorithms such as BLAST.

Further, the invention refers to a VHH antibody, which competes with a specific VHH antibody disclosed herein for the binding to the SARS-CoV-2 spike protein S1 domain. In certain embodiments, a competing VHH antibody binds the same or an overlapping epitope on the SARS-CoV-2 spike protein S1 domain, particularly the receptor-binding domain (RBD) of the SARS-CoV-2 S1 domain as a specific VHH antibody disclosed herein. For example, the invention refers to a VHH antibody, which competes either with the VHH antibodies Re5D06, Re6D06, Re6H06, Re9B09, Re9C08, or Re9H01, or with VHH antibodies Re6B07 or Re7E02.

In certain embodiments, the invention relates to a VHH antibody competing with VHH antibody Re5D06, such as any one of the VHH antibodies Re6B02, Re7B01, Re10B10, or Re10F10. In certain embodiments, the invention relates to a VHH antibody competing with VHH antibody Re7E02, such as the VHH antibody Re6B07.

Competition is measured either as an at least 90% loss of fluorescence staining of the SARS-CoV-2 spike protein by a fluorophore-labelled candidate VHH antibody in the presence of a 100-fold molar excess of an unlabelled competitor VHH antibody e.g. selected from Re5D06, Re6D06, Re6H06, Re9B09, Re9C08, Re9H01, or Re7E02, or conversely as an at least 90% loss of spike protein-staining by a fluorophore-labelled VHH antibody e.g. selected from Re5D06, Re6D06, Re6H06, Re9B09, Re9C08, Re9H01, or Re7E02 in the presence of a 100-fold molar excess of the unlabelled candidate VHH antibody as a competitor.

Further, the invention refers to a VHH antibody, which competes with a specific VHH antibody disclosed herein for the binding to the SARS-CoV-2 spike protein S1 domain. In certain embodiments, a competing VHH antibody binds the same or an overlapping epitope on the SARS-CoV-2 spike protein S1 domain, particularly the receptor-binding domain (RBD) of the SARS-CoV-2 S1 domain as a specific VHH antibody disclosed herein. For example, the invention refers to a VHH antibody, which competes either with the VHH antibodies Re5D06 and/or Re6D06 or with VHH antibodies Re6B07 and/or Re7E02.

In particular embodiments, an amino acid of a reference sequence, including an amino acid in a CDR1, CDR2 or CDR3 sequence, is replaced by a conservative amino acid substitution, i.e. a substitution of an amino acid by another amino acid with similar biochemical properties, for example a substitution of an aliphatic amino acid, e.g. Gly, Ala, Val, Leu or Ile, for another aliphatic amino acid; a substitution of a basic amino acid, e.g. His, Lys or Arg, against another basic amino acid or against Met; a substitution of an acidic amino acid or an amide thereof, e.g. Asp, Glu, Asn or Gln, against another acidic amino acid or an amide thereof; a substitution of an aromatic amino acid, e.g. Phe, Tyr or Trp, against another aromatic amino acid, or a substitution of a hydroxy or sulfur containing amino acid, e.g. Ser, Thr, Met or Cys, against another hydroxy or sulfur containing amino acid.

In further particular embodiments, the amino acid Lys of a reference sequence including an amino acid in a CDR1, CDR2 or CDR3 sequence, e.g. the amino acid Lys in the CDR3 sequence of any one of the VHH antibodies Re5D06, Re6B02, Re7B01, Re10B10 Re10F10 or of another VHH antibody may be replaced by a different amino acid, e.g. Arg or Met. The VHH antibody of the present invention is binding to the S1 domain, particularly to the RBD of the S1 domain of SARS-CoV-2. In certain embodiments, a VHH antibody is capable of virus neutralization at a concentration of about 10 nM or less, of about 1 nM or less, of about 500 pM or less, of about 100 pM or less or even at about 50 pM or less.

Further, the present invention relates to a nucleic acid molecule, e.g. a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well known in the art. The cell may be known host cell for producing antibodies or antibody fragments, e.g. a prokaryotic cell such as an E. coli or a Bacillus sp. cell, a yeast cell, an insect cell or a mammalian cell, e.g. a CHO cell, or a plant.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

The inventors' target structure has been the S1 fragment of the SARS-CoV-2 spike protein (Wrapp et al., 2020), which contains two main globular domains: A large N-terminal, lectin-like domain (residues 27-290) as well as a receptor-binding domain (RBD, residues 330-521). The RBD mediates the interaction with the ACE2 host cell receptor, and is thus essential for infectivity.

The inventors applied five subcutaneous immunizations in weekly intervals such that the S1 fragment and in particular the RBD were presented in multiple contexts to the animals' immune systems. As antigens were used: transfected HEK293 cells expressing the complete spike protein on its surface ($10^7$ cells per immunization) and up to 100 μg of the following purified proteins: fusions of the RBD and the entire S1 domain to mouse and human Fc fragments as well as an RBD fusion to the *E.coli* DsbG dimer. Four days after the last immunization, blood samples were collected, RNA from ~$10^8$ lymphocytes each was isolated, VHH-coding regions were amplified by nested RT-PCRs, cloned as cDNAs into a phagemid to yield three separate immune libraries with complexities of around $10^9$ independent clones each. These libraries were subjected to phage display, using as baits either an immobilized RBD (yielding anti-RBD VHH) or an immobilized S1 fragment with competition by an excess of free RBD (to yield anti-S1ΔRBD VHH antibodies). Bovine Serum Albumin, as well as human and murine serum, were included as non-specific competitors.

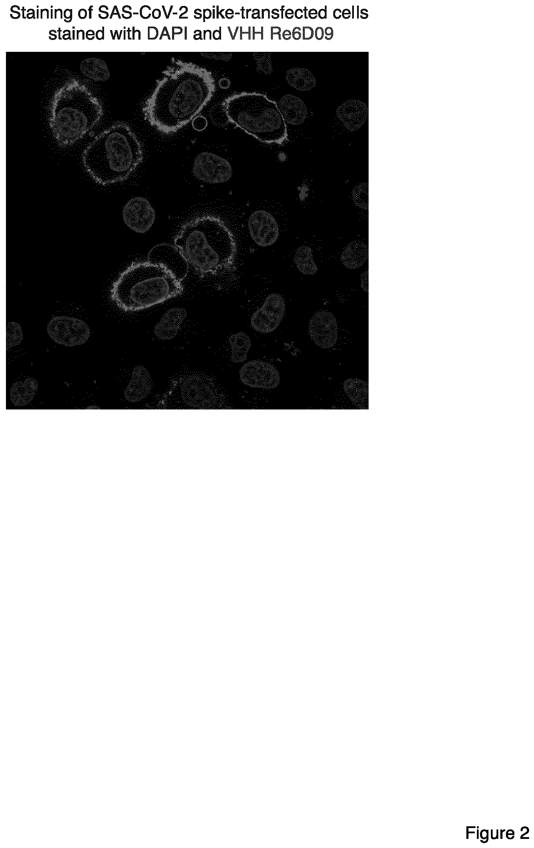

A total of 672 selected clones were so far sequenced and classified according to sequence similarity. Representatives of all VHH classes were then expressed in *E.coli* and purified (see Figure 1 for specific sequences of VHHs pursued in this invention). One set of nanobodies was expressed with ectopic cysteines for labelling with fluorophore-maleimides (Pleiner et al., 2015). These labelled nanobodies were used for immunofluorescence on HeLa cells that had been transfected with a SARS CoV2 spike expression construct. Because only a fraction of cells gets transfected, a typical field of view contains non-transfected cells (serving as internal negative control) as well as transfected ones. Several of the labelled nanobodies stained the expressed spike protein very brightly. Figure 2 shows VHH Re6D09 labelled with Alexa568 as an example. Table 1 (supra) lists the VHHs that have been tested positive for anti-Spike fluorescence.

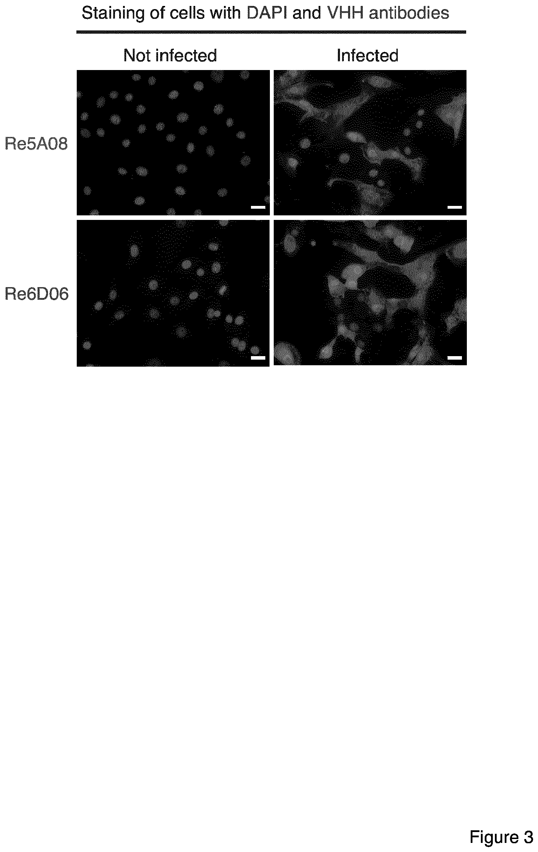

In a second set of experiments, the inventors used cultured cells infected with SARS-CoV-2 isolated from a patient's sample. It was observed that numerous VHH antibodies (e.g. Re5A08, Re5B06, Re5D05, Re6D06, and Re6D09) stained viral structures brightly and specifically as judged by a lack of signal in non-infected cells (see Figure 3 for example). Thus, VHH antibodies of this invention are suitable for detecting SARS-CoV-2 spike components in biological samples.

In the third set of experiments, the inventors performed tests if any of the obtained nanobodies can neutralize SARS-CoV-2 and prevent the virus from infecting a target cell. In these experiments, the Vero E6 cell line was used, which expresses high levels of the SARS-CoV-2 receptor ACE2 (Li et al., 2003) but is deficient in its interferon response (Emeny and Morgan, 1979); it is therefore extremely susceptible to viral infection. Moreover, a SARS-CoV-2 patient derived virus strain was used carrying the infection-enhancing D614G spike mutation (Zhang et al., 2020). This together therefore combines into a highly stringent test system for anti-viral inhibition. Without VHH antibodies, the addition of virus caused a pronounced cytopathic effect (CPE), and the amount of virus increased within three days of incubation approximately 10 000-fold.

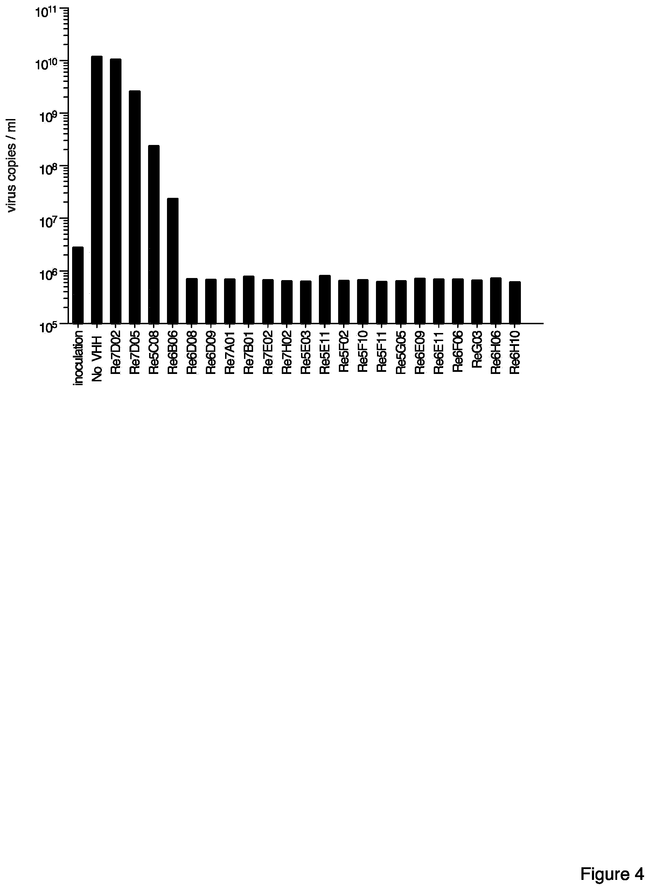

When tested at a concentration of 500 nM, 30 out of a first set of 35 VHHs were able to protect cells from the CPE and prevented virus replication completely (see Figure 4 for examples). These and neutralizing VHH antibodies discovered in later rounds of testings (see Tables 1-4) are now excellent candidates for treating SARS-CoV-2-infected patients.

## Homotrimeric VHH antibodies

An important issue in pharmaceutical development is the lower limit of concentration at which an anti-viral antibody is still effective. Indeed, it makes a great difference if grams (as in the case of rabies post-exposure prophylaxis) or one milligram are required for an effective therapeutic dose. This determines the costs per treatment and, if many patients need to be treated, it matters how many therapeutic doses can be produced at all. Likewise, adverse side effects by the antibody itself will scale with the applied dose. Finally, contaminants, such as endotoxins, can cause side effects, and the absolute amount of contaminant will scale-up with the administered dose. It is thus highly desirable to obtain VHH antibodies that effectively block viral infection at the lowest possible concentration.

The binding strength of an antibody or any other binder is in the first instance ruled by the Kd of the relevant interaction (dissociation constant or the ratio of off and on rates). If the free concentration of the binder equals the Kd, then half of the binding sites are blocked. As a SARS-CoV-2 viral particle contains >100 spike trimers (Bar-On et al., 2020), efficient neutralization probably only occurs if their majority, i.e. 99% or even 99.9% are blocked. This requires the binder to be present at concentrations that are 100-fold or a 1000-fold higher than the Kd.

A solution to this problem is to fuse the monovalent VHH antibody to the Fc part of an immunoglobulin, e.g. a human IgG and thus make the VHH bivalent. This could impose a stronger binding by avidity effects, but only if the distance of binding sites fits the geometry of the antibody. Apart from this reservation, Fc fusions are associated with additional potential drawbacks: First, production of such Fc fusions should be carried out in human cells to ensure proper folding and a human glycosylation pattern. Second, the Fc fragment is an immune effector domain that might aggravate the problem of inappropriate complement activation and cytokine storms, which are hallmarks of the most severe SARS-CoV-2 pathologies. Third, a dimeric IgG might well dock to two of the spike molecules of a homotrimeric spike, but this would leave the third one in an unbound state.

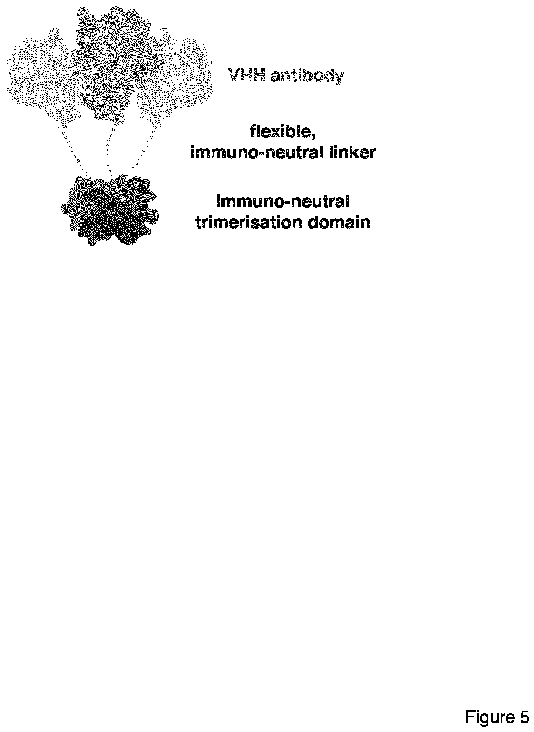

Thus, the inventors have considered that a homo-trimeric ligand would be far more appropriate for blocking a homotrimeric target (Figure 5). This principle of 'symmetry-matching' is a central element of this approach.

Homotrimerization of a VHH antibody requires an appropriate polypeptide fusion partner as trimerization module. According to the present invention, a fusion partner is preferred, comprising at least one of the following features:

(1) it has a length of about 250 amino acids or less, of about 200 amino acids or less or of about 150 amino acids or less;
(2) it is capable of folding properly in commonly used recombinant expression systems (bacteria, yeast, insect and human cells), and assemble into trimers with a very low Kd of e.g. less than 1nM in the picomolar range - to avoid disassembly at low concentrations; and
(3) it is of mammalian, particularly of human origin to be as non-immunogenic as possible.

Preferred trimerization modules include domains derived from collagen, the most abundant protein of the human body, including the trimerization (NC1) domains of collagen XV (PDB 3N3F, 54 residues) (Wirz et al., 2011), collagen XVIII (PDB 3HSH; 54 residues) (Boudko et al., 2009) and collagen X (PDB 1GR3; 160 amino acids) (Bogin et al., 2002). Another very appropriate homotrimeric fusion partner is the lung surfactant protein D (LSFPD) that recognizes sugars on viral and bacterial pathogens (see e.g. PDB 1B08, 3IKN, 3IKQ, 3IKR). As the SARS-CoV-2 spike protein is heavily glycosylated, an VHH-LSPD fusion could confer a bimodal inhibition of spike function.

The collagen XVIII NC1 module has been used for trimerizing VHHs before (Alvarez-Cienfuegos et al., 2016), however, not for the purpose of symmetry-matching and targeting homotrimeric targets. Further, this earlier study used expression in human cells.

The inventors now fused 12 different VHH antibodies through a flexible spacer to this trimerization module and found good expression and solubility in *E.coli,* and succeeded in just a single purification step to obtain an essentially pure fusion protein.

According to the present invention, it is preferred that the VHH antibody is linked to the trimerization domain via a spacer comprising at least one of the following features:

(1) it is long enough to allow a simultaneous binding of all three VHH copies to the same spike trimer, e.g. having a length of at least about 5 amino acids, particularly about 5 amino acids to about 100 amino acids, and more particularly of about 10 to about 50 amino acid, also depending on the specific target structure;

(2) it is substantially free (i.e. comprising an amount of 10% or less, 5% or less or 2.5% or less) or it is free from amino acid residues selected from Lys, Arg, Trp, Tyr, Phe, Met, Leu, Ile, Val, His and Cys;

(3) it does not contain a strong B-cell epitope;

(4) it is not particularly protease-sensitive, e.g. it does not contain a trypsin or chymotrypsin cleavage site;

(5) it does not contain repetitive sequence portions, e.g. repetitive sequence portions having a length of at least 3 amino acid residues;

(6) it comprises a negative net charge;

(7) it comprises amino acids selected from Gly, Ser, Ala, Thr, Pro, Asp and Glu, particularly in an amount of at least about 50%, at least about 60%, at least about 70%, at least about 80%, at least about 90% or 100%; and

(8) it comprises a succession of tripeptides, wherein the first and the second amino acid are selected from Gly, Ser, Ala, Thr, and Pro, particularly Gly, Ser, Ala and Thr, and more particularly Gly, Ser and Ala, and the third amino acid is selected from Asp and Glu; and particularly without direct repetitions of the concatenated tripeptide motifs.

It should be note that different arrangements for the subunits of homomultimeric VHH antibodies are possible. As just described, the trimerization module might be located N-terminally, followed by the spacer and the VHH antibody. Alternatively, the VHH antibody might be located N-terminally, followed by spacer and trimerization module.

The concept of providing homomultimeric, particularly homotrimeric VHH antibodies, is not limited to SARS-CoV-2. It is quite remarkable that many viruses rely on homotrimeric entry receptors and/ or fusion proteins with C3 rotational symmetry. The symmetry-mismatch to bivalent antibodies is perhaps of advantage for those viruses when it comes to delaying a neutralizing immune response. Thus, our symmetry-matching approach to homotrimeric neutralizing VHH antibodies is also suitable against any target structure having a threefold-rotationally symmetric subunit structure. Such a target structure is present on other viruses, such as other members of the *Coronaviridae, Orthomyxoviridae* (with their trimeric HA-proteins), many *Paramyxoviridae* (with their trimeric fusion proteins), Dengue fever- and Zika-viruses (3-fold or higher order protein assemblies on their surface), as well as *Herpesviridae* (trimeric gB fusion protein) or Human Immunodeficiency Virus (with its trimeric gp120 protein).

Moreover, the approach can even be extended to efficiently inhibit non-viral targets that also show a threefold rotational symmetry, such as the members of the TNF Ligand and TNF Receptor superfamilies, e.g. the death ligands and receptors that trigger the extrinsic pathway of apoptosis (e.g. CD95, TNFα, or Trail). Also here, a stable and non-immunogenic trimerization module as the ones derived from human collagens X, XV, or XVIII may be used.

The lengths of spacers between VHHs and trimerization unit may be dependent from the specific target structure and thus subject to optimization: If a spacer is too short, then it will not allow all three binding sites to be used at the same time or cause tension in the bound state, while a spacer that is too long might be entropically suboptimal in terms of increasing the local VHH concentration. Based on the above teaching, an optimal spacer may be developed, which will not only provide the maximum enhancement in binding strength but also a gain specificity as multivalent binding is most favored to objects of a complementary geometry.

**Therapeutic applications**

Still a further aspect of the present invention is the use of an active agent selected from the VHH antibody, the set of at least 2 different VHH antibodies or the multimeric trimeric VHH antibody in medicine, particularly for therapeutic and/or in vitro or vivo diagnostic use. In certain embodiments, the VHH antibody, the set of VHH antibodies or the multimeric, particularly trimeric VHH antibody is used in human medicine.

In particular embodiments, a therapeutic VHH antibody is selected from (i) a VHH antibody comprising the CDR3 sequence of VHH antibody Re5D06 or a related sequence, (ii) a VHH antibody comprising the CDR1, CDR2 and CDR3 sequences of VHH antibody Re5D06 or related sequences, (iii) a VHH antibody comprising the VHH sequence of VHH antibody Re5D06 or a related sequence, or (iv) a VHH antibody competing with VHH antibody Re5D06.

In particular embodiments, a therapeutic VHH antibody is selected from (i) a VHH antibody comprising the CDR3 sequence of VHH antibody Re6H06 or a related sequence, (ii) a VHH antibody comprising the CDR1, CDR2 and CDR3 sequences of VHH antibody Re6H06 or related sequences, (iii) a VHH antibody comprising the VHH sequence of VHH antibody Re6H06 or a related sequence, or (iv) a VHH antibody competing with VHH antibody Re6H06.

In particular embodiments, a therapeutic VHH antibody is selected from (i) a VHH antibody comprising the CDR3 sequence of VHH antibody Re9H01 or a related sequence, (ii) a VHH antibody comprising the CDR1, CDR2 and CDR3 sequences of VHH antibody Re9H01 or related sequences, (iii) a VHH antibody comprising the VHH sequence of VHH antibody Re5D06 or a related sequence, or (iv) a VHH antibody competing with VHH antibody Re9H01.

In particular embodiments, a therapeutic VHH antibody is selected from (i) a VHH antibody comprising the CDR3 sequence of VHH antibody Re7E02 or a related sequence, (ii) a VHH antibody comprising the CDR1, CDR2 and CDR3 sequences of VHH antibody Re7E02 or related sequences, (iii) a VHH antibody comprising the VHH sequence of VHH antibody Re7E02 or a related sequence, or (iv) a VHH antibody competing with VHH antibody Re7E02.

Therapeutic applications include methods for preventing or treating a disorder caused by and/or associated with a Coronavirus infection, particularly a disorder caused by and/or associated with a SARS-CoV-2 infection and more particularly of preventing or treating COVID-19. As outlined, above, however, the trimeric VHH antibody may be directed to other target structures, and thus, is useful in methods for preventing or treating a disorder caused by and/or associated with a virus infection, particularly a disorder caused by and/or associated with a Coronavirus infection, an Orthomyxovirus, e.g. an Influenza A, B, or C virus, a Paramyxovirus, e.g. a Parainfluenza virus, Respiratory Syncytial virus, Measles virus, or Mumps virus, Dengue fever virus, Zikavirus, a Herpesvirus or HIV. Further, the trimeric VHH antibody is useful in methods for preventing or treating a non-viral disorder, particularly a disorder caused by and/or associated with a member of the TNF ligand superfamily and/or a member of the TNF Receptor superfamily.

In therapeutic applications, the active agent is administered in an effective amount to a subject in need thereof, particularly to a human subject. The dose will depend from the specific type of agent, e.g. monovalent VHH antibody or multimeric, particularly trimeric VHH antibody, and the type of disease.

In the prevention or treatment of COVID-19, a daily dose of a monomeric VHH antibody for a human subject is any suitable dose based on the severity of the disease, the specific type of agent, and the mode of administration, e.g. locally or systemically, particularly from about 5 μg to about 5000 mg per day.

In the prevention or the treatment of COVID-19, a daily dose of a trimeric VHH antibody for a human subject is any suitable dose based on the severity of the disease, the specific type of agent, and the mode of administration, e.g. locally or systemically, particularly from about 1 μg to about 2500 mg per day.

In the prevention or treatment of COVID-19, a unit dose of a monomeric VHH antibody for a human subject is any suitable dose based on the severity of the disease, the specific type of agent, and the mode of administration, e.g. locally or systemically, particularly from about 5 μg to about 5000 mg per unit.

In the prevention or the treatment of COVID-19, a unit dose of a trimeric VHH antibody for a human subject is any suitable dose based on the severity of the disease, the specific type of agent, and the mode of administration, e.g. locally or systemically, particularly from about 1 μg to about 2500 mg per unit.

Typically, the VHH antibody is administered as a pharmaceutical composition comprising the active agent and a pharmaceutically acceptable carrier or excipient. Examples of suitable carriers and excipients for formulating antibodies or antibody fragments are well known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g. by subcutaneous, intramuscular or intravenous injection or by infusion. In further embodiments, the pharmaceutical composition may be administered locally, e.g. orally, nasally or intrapulmonary, for example by inhalation as an aerosol.

In certain embodiments, the pharmaceutical composition is administered in an early infection stage, e.g. in an infection stage where the upper airways such as the oral cavity, the nasal cavity, the paranasal sinus, the pharynx and/or the throat are infected, but the lower airways such as the bronchi and/or the lung are not infected. In further embodiments, the pharmaceutical composition is administered in a late infection stage wherein the lower airways such as the bronchi and/or the lung are infected. Further, the pharmaceutical composition may be administered in an infection stage where the subject suffers from a respiratory dysfunction and optionally is ventilated. In still further embodiments, the pharmaceutical composition may be administered prophylactically to a subject being at risk of a Coronavirus infection, e.g. a subject, which has previously, for example, within 1, 2, 3, 4, or 5 days or even longer, been in close contact with an infected subject.

The VHH antibody may be administered alone or together with a further active agent, which may be selected from anti-viral agents such as remdesivir or methotrexate.

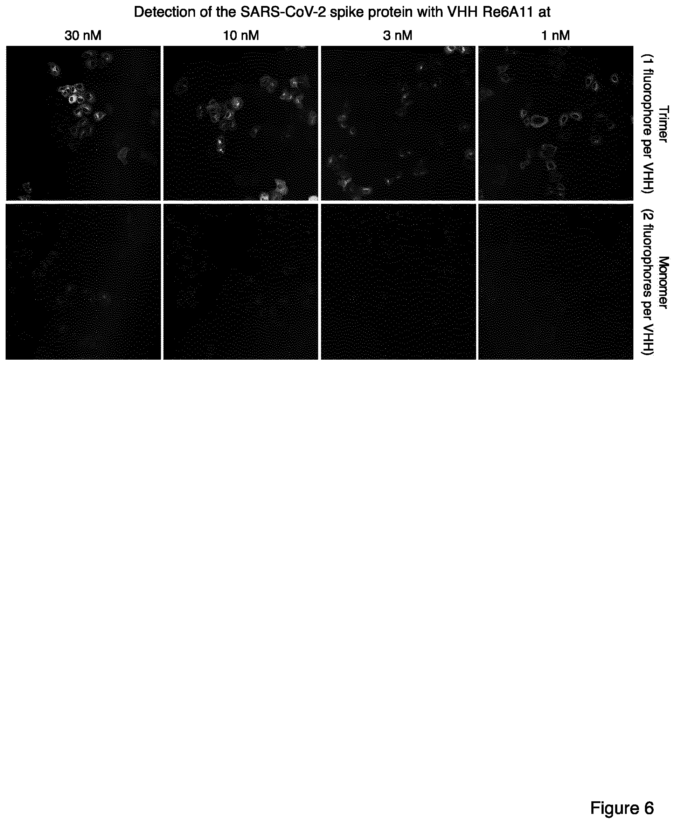

In order to determine suitable administration schemes, the inventors first tested the principle of avidity enhancement for the rather weakly binding Re6A11 variant (labelled with two Alexa488 fluorescence groups per VHH module), whose monomeric version is readily washed off its targets and hence showed only very weak staining of Spike molecules in transfected HeLa cells (Figure 6). The trimerized version, however, showed a strong signal even when used at lower concentrations and with just one Alexa488 per VHH module. This strong staining persisted also during very excessive washing steps and when the VHH trimer was used at a very low concentration (1 nM).

As second proof of principle for the avidity enhancement, the inventors used the virus neutralization assay as described above. Table 3 shows the minimum SARS-CoV-2-neutralizing concentrations of selected nanobody monomers and trimers. Virus neutralization experiments were performed as in Figure 4, with VHH concentrations being titrated down in factor $\sqrt{10}$ steps. Lowest SARS-CoV-2-neutralizing concentrations of selected VHH monomers and trimers are listed that fully suppressed infection over a period of three days. Concentrations refer to the respective VHH units.

The trimers were tandem fusions of VHH, a 39 residues spacer followed by the collagen XVIII NC1 domain.

**Table 3**

| VHH | Lowest neutralizing VHH concentration | |
|---|---|---|
| | As monomer | As trimer |
| Re5A08 | 5 nM | ≤ 0.017 nM |
| Re5C08 | 500 nM | 0.50 nM |
| Re6A11 | 50 nM | 0.050 nM |
| Re6B06 | 500 nM | 0.50 nM |
| Re6D06 | 0.5 nM | ≤ 0.017 nM |

The monomeric version of the VHH antibody Re5A08 blocked infection down to a concentration of 5 nM. In trimerized form, however, the block was still tight at the lowest tested concentration of 17 pM (calculated for the VHH concentration). As lower concentrations have not yet been tested, the enhancement by trimerization is most probably still underestimated. In fact, for the given geometry and length of spacers (39 residues in the present example), an increase in binding strength by a factor of $10^2$ or even more, e.g. up to $10^6$-$10^9$ (dependent on specific assumption) is possible. In such regime and for any reasonably strong binder, the binding constant will no longer be limiting, but only the on-rate for the binding reaction.

While the gain in virus-neutralizing power by the symmetry-matching trimerization is experimentally best demonstrated for weakly-binding VHHs, it should be noted that we isolated several VHHs that neutralize even as monomers at picomolar concentrations. Re5D06 (or other members of the same class) or Re6H06 are such examples: they fully suppressed infection already at the lowest tested concentration, 50 pM. This corresponds to ~ 0.7 μg VHH antibody per liter. When injected into a patient, and generously assuming that it gets diluted into an accessible volume of 50 l, then 100 μg should already be sufficient to reach a therapeutically effective concentration in the extracellular space. As also Re5D06 is being combined with avidity-enhancing trimerization module, there is a comfortable safety margin in this estimate. Following optimization, we usually obtain one gram of nanobody from 100 g *E. coli* biomass. Thus, an industry-standard 1000 l fermenter might yield >1 million therapeutic doses within a single run.

Table 4 depicts the lowest SARS-CoV-2-neutralizing concentrations of the most potent anti-viral VHH monomers. As described above, testings were under highly stringent conditions, and neutralization was only scored as such if the infection block remained tight over a period of three days, i.e. when no amplification of viral RNA, no cytopathic effect and no new synthesis of the viral Spike protein over this period of time occurred.

**Table 4**

| VHH | Class | Lowest SARS-CoV-2-neutralizing concentration |
|---|---|---|
| Re5D06 | Re5D06 | 0.050 nM |
| Re10B10 | Re5D06 | 0.050 nM |
| Re6B02 | Re5D06 | 0.050 nM |
| Re10F10 | Re5D06 | 0.050 nM |
| Re7B01 | Re5D06 | 0.050 nM |
| Re6H06 | | 0.050 nM |
| Re9H01 | | 0.050 nM |
| Re9B10 | Re6D06 | 0.500 nM |
| Re6D06 | Re6D06 | 0.500 nM |
| Re9B09 | | 0.500 nM |
| Re9C08 | | 0.500 nM |
| Re7E02 | Re6B07 | 0.500 nM |

From this data, it can be gathered that several VHH antibodies belong to a certain class of VHH antibodies, e.g. class Re5D6, class Re6B07 or Re6D06, i.e. they compete with the respective VHH antibody Re5D6, Re6B07 or Re6D06 for the binding to the RBD. Thus, the VHH antibodies of an individual class compete with each other, e.g. they recognize the same or an overlapping epitope on the RBD.

A further aspect that needs to be considered is the plasma half-life of a VHH antibody. Proteins smaller than 40kDa are subject to considerable renal clearance, and an isolated VHH antibody (~15 kDa) shows, therefore, a plasma half-life of only 4 hours (Hoefman et al., 2015). Thus, the administration might comprise repeated daily injections or continuous perfusion during anti-viral therapy that might last for days to weeks. One solution to this problem is to increase the size of the protein. Fusion to spacer and trimerization module increases the size to ≥70kDa, and such fusion should then have a far longer plasma half-life that is no longer limited by renal clearance. One can, however, also imagine alternative application routes. In fact, inhalation of an anti-SARS-CoV-2 VHH antibody formulation would bring the therapeutic agent directly to the site of the highest and most problematic virus load, namely the airway epithelia and/or the lung.

The here outlined antiviral clinical strategy will become more robust if not just one but an entire set of several VHH antibodies is deployed. First, one should expect synergistic effects for neutralizing antibodies that bind to non-overlapping sites. Second, patients might develop anti-idiotypic antibodies against a given therapeutic VHH antibody and render it ineffective in treatment. A switch to another VHH would solve the problem. Third, and perhaps most importantly there is the clear risk that SARS-CoV2 evolves further, that escape mutations in its RBD might destroy the epitope of a given VHH antibody and render such VHH inefficient in therapy. This risk can be minimized by utilizing a panel of neutralizing antibodies that recognize non-identical and possibly even non-overlapping epitopes, or by at least switching to another VHH that still neutralizes a given escape mutant.

It will be a critical clinical decision when, during an anti-viral therapy, the treatment regime needs to be adjusted and switched from one VHH antibody to another. It is therefore important to reiterate that the here described therapeutic VHH antibodies are also suitable for diagnostic purposes and can thus guide such decision. When, for example, a mutation in the RBD abolishes the binding and thus impairs the neutralizing power of a given VHH antibody, this can be reported through a diagnostic test using the same VHH. Conversely, if a mutant virus isolate can still be stained with a fluorescent VHH antibody that neutralizes the prototypic SARS-CoV2, then this VHH antibody will also be likely to neutralize the mutant one. The dual therapeutic and diagnostic use of anti-SARS-CoV-2 VHHs is, therefore, central to this invention.

### Diagnostic applications

Diagnostic applications include in vitro methods wherein a VHH antibody is used for detecting Coronavirus spike protein in a sample, e.g. in a body fluid such as saliva, blood, serum or plasma, stool samples or in a tissue or biopsy sample. Diagnostic applications further include in vivo methods wherein a VHH antibody is used for detecting Coronavirus spike protein in a subject, particularly in a human patient. For diagnostic applications the VHH antibody carry a label for direct detection or used in combination with secondary detection reagents, e.g. antibodies, including conventional antibodies or VHH antibodies, for indirect detection according to established techniques in the art.

In particular embodiments, a diagnostic VHH antibody is selected from (i) a VHH antibody comprising the CDR3 sequence of VHH antibody Re6D06 or a related sequence, (ii) a VHH antibody comprising the CDR1, CDR2 and CDR3 sequences of VHH antibody Re6D06 or related sequences, (iii) a VHH antibody comprising the VHH sequence of VHH antibody Re6D06 or a related sequence, or (iv) a VHH antibody competing with VHH antibody Re6D06.

In particular embodiments, a diagnostic VHH antibody is selected from (i) a VHH antibody comprising the CDR3 sequence of VHH antibody Re8E12 or a related sequence, (ii) a VHH antibody comprising the CDR1, CDR2 and CDR3 sequences of VHH antibody Re8E12 or related sequences, (iii) a VHH antibody comprising the VHH sequence of VHH antibody Re8E12 or a related sequence, or (iv) a VHH antibody competing with VHH antibody Re8E12.

Further, the present invention is explained in more detail by the following Figures and Examples.

### Figures

**Figure 1: Sequence alignment and highlighting of variable regions**
Figure shows an alignment of VHH sequences from Table 1. Residues that deviate from the consensus are shown in colour. The three variable CDR regions are indicated.

**Figure 2: Staining of transfected cells transiently expressing the SARS-CoV-2 spike protein.**
Hela cells where transfected with a plasmid carrying the humanized coding sequence of the SARS-CoV-2 spike protein under the control of a CMV promoter. 36 hours post-transfection, cells were fixed for 5 minutes with 4% paraformaldehyde (PFA), permeabilized, blocked with 5% BSA, incubated with 30 nM VHH Re6D09 carrying two Alexa568 fluorophores per molecule, extensively washed and finally imaged with an LSM780 confocal laser scanning microscope using the 405 nm and 568 nm laser lines for excitation. Image shows the overlaid DAPI and 568 channels, detecting DNA and VHH antibody in blue and red, respectively. Note that the transfection efficiency was only ~30%. The bright red signal corresponds to transfected cells, while non-transfected ones served as negative control.

**Figure 3: Fluorophore-labelled anti-SARS-CoV-2 VHH antibodies specifically detect the spike protein and assembling viruses in infected cells**
Vero E6 cells were infected by SARS-CoV-2 for three days, fixed for two hours with 4% paraformaldehyde, and stained as described above with 30 nM of the indicated Alexa4888-labelled VHH antibodies. Imaging was with a standard epifluorescence microscope.

**Figure 4: SARS-CoV-2 neutralizing VHH antibodies.** At day 0, Vero E6 cells were inoculated with SARS-CoV-2, in the presence or absence of the indicated VHH antibodies. Three days later, the virus-load increased ~10 000-fold when the antibodies had been omitted (compare "inoculation" and "no Nb"). One nanobody (Re7D02) had no effect. Three (Re7D05-Re6B06) had a weak impact. The other 18 VHH antibodies of this experiment blocked viral infection completely. Quantitation of viral RNA in the supernatants of infected cells was by quantitative reverse transcription (RT) PCR. Note the Log10-scale of the figure.

**Figure 5: Scheme of a homotrimeric VHH fusion to match the C3 rotational symmetry of the Spike of SARS-CoV-2 and other viruses.**

**Figure 6: Trimerization caused a strong avidity effect for the VHH-spike interaction**
Hela cells were transfected as described in Figure 2 to transiently express the SARS-CoV2 spike protein. Following fixation, they were stained with Alexa488-labelled VHH Re6A11. In monomeric form, the staining was very weak even at 30 nM and with 2 fluorophores per VHH. In contrast, staining was strong with the trimerized version, even at a much lower concentration of 1 nM VHH and with only one fluorophore per VHH.

**VHH antibody sequences**

>Re5A08

GSQVQLVESGGGLVQAGGSLRLSCTASGHTFTANRMGWFRQAPGKEREFVAAIN
WGGDSTNYVDSVKGRFTISRDIAKNTVYLQMNSLKPEDTAVYFCAARNHVTGEFD
SWGQGTQVTVSSTS

>Re5B06

GSQVQLVESGGGLVQPGGSLRLSCAASGSIRSIYATVWFRQAPGKEHEWVGSITS
SNVTTYADSVKGRFTISRDNAKKTVYLQMNSLKPEDTALYYCNVHFASEYSDYAQI
QGQGTQVTVSSTS

>Re5C01

GSQVQLVESGGGLVQAGGSLRLSCGVSGRTFSSYAMGWFRQAPGKEREFVATIS
WSGGTTNYAHSVKGRFTISRDNAKNTVYLQMNSLKVEDTAVYYCYAVSSGSDYDG
GMDYWGKGTQVTVSSTS

>Re5C08

GSQVQLVESGGGSVEAGGSLRLSCAASGRTFNDYNMVWFRQAPGKEREFVAAIK
WNGGNTSYADSVKGRFAVSRDNAKNTVYLQMNNLKHEDTAEYLCYTVGPEGDYW
GQGTQVTVSSTS

>Re5D06

GSQVQLVESGGGLVQPGGSLRLSCAASGITLDYYAIGWFRQAPGKEREGVSRIRS
SDGSTNYADSVKGRFTMSRDNAKNTVYLQMNSLKPEDTAVYYCAYGPLTKYGSS
WYWPYEYDYWGQGTQVTVSSTS

>Re5E03

GSQVQLVESGGGLVQPGGSLRLSCAASGFTLDYYAIGWFRQAPGKEREGVSCISN
SDGSTRYADSVKGRFTISRDNAKNTVYLQMNSLKPEDTAVYYCAGGPQTYYSGSY
YYTCAEGAMDYWGKGTLVTVSSTS

>Re5E11

GSQVQLVESGGGLVQPGGSLRLSCAASGFTLDYYAIGWFRQAPGKEREGVSCISS
SDGRTYYADSVKGRFTISRDNAKNTVYLQMNSLKPEDTAVYYCATAPLTYYSGSW
YLTCNSDAMDYWGKGTLVTVSSTS

>Re5F10

GSQVQLVESGGGLVQPGGSLRLSCVASGFTFSSFAMGWYRQAPGKECEWVATITI
TGGSTNYADSVKGRFTISRDNAKNTVYLQMNSLKPEDTAVYYCNPDPGCRRGQGT
QVTVSSTS

>Re5F11

GSQVQLVESGGALVQPGGSLRLSCATSGSISSYRMGWYRQGPGKQRELVAFITIG
GITDYIDSVKGRFTISRDNAKNTMYLQMNSLKPEDTAVYYCNADPPLFNWGQGTQV
TVSSTS

>Re5G05

GSQVQLVESGGGLVQAGGSLRLSCAASGFTATSYAMGWYRQAPGKECEWVATIT
STGGNTNYADSVKGRFTISRDNAKNTVYLQMNSLKPEDTAVYYCNPDPGCDWGQ
GTQVTVSSTS

>Re6A11

GSQVQLVESGGGLVQAGGSLRLSCAASGRTFSNDALGWFRQAPRKEREFVAAIN
WNSGTYYADSVKGRFTISRDNAKNTVYLQMNSLKPEDTAVYSCAAASDYGLPRED
FLYDYWGQGTLVTVSSTS

>Re6B02

GSQVQLVESGGALVQPGGSLRLSCVASGFTLDYYAIGWFRQAPGKEREGVSRIRS
SDGSTNYADSVKGRFTISRDNAKNTVYLQMNSLKPEDTAVYYCAYGPLTKYGSSW
YWPYEYDYWGQGTQVTVSSTS

>Re6B06

GSQVQLVESGGGLVQAGGSLRLSCAASGRAFSSAPMSWFRQAPGKEREFVASVS
WSGDSTNYADSVKGRFTISRDNAKNTGYLQMNSLKPEDTAVYYCKRGPYWGQGT
QVTVSSTS

>Re6B07

GSQVQLVESGGGLVQPGGSLRLSCAASGFTLDYYAIGWFRQAPGKEREGVSYIRS
SDGTTYYADSVKGRFTISRDNAKNTVYLQMNSLKPEDTAVYYCAADEAYYSELGW
ESPWGWSYWGQGTRVTVSSTS

>Re6D06

GSQVQLVESGGGLVQAGASLRLSCAASGRMFGVYRMGWFRQAPGKEREFVAGIS
TSVGTTNYADSVKGRFTISRDNAKNTVYLQMNSLKPEDTAVYYCAARDPTTYEYDY
WGQGTQVTVSSTS

>Re6D08

GSQVQLVESGGGLVQAGGSLRLSCAASGRTFSSYAMGWFHQAPGKEREFVATIN
WSGDSTYYADSVKGRFTISRDNAKNTVYLQMNSLKPEDTAVYYCAAVVDPSPTYY
SGKYYPPRVEYWGKGTQVTVSSTS

>Re6D09

GSQVQLVESGGGSVEAGGSLRLSCAASGRTFNNYNMVWFRQAPGKEREFVAAIN
WNGGSTSYAASVKGRFAVSIDNAKNTLYLQMNNLKHEDTAEYLCYTVGPEGDYW
GQGTQVTVSSTS

>Re6E11

GSQVQLVESGGGLVQPGGSLRLSCAASGLTLDYYAIGWFRQAPGKEREGVSCISS
RDGSTMYADSVKGRFTISRDNAKNTVYLQMNSLKPEDTAVYYCAATPTTYYSGSY
YYTCSPEGYDYWGQGTQVTVSSTS

>Re6F06

GSQVQLVESGGGLVQAGGSLRLSCAASGFTFSNYAMGWYRQAPGKECEFVAVITI
TGSNTNYADSVKGRFTISRDNAKNTVYLQMNSLKPEDTAVYYCNPDPGCESQGQG
TRVTVSSTS

>Re6G03

GSQVQLVESGGGLVQAGGSLRLSCAASGRTFSTYRMAWFRLAPGKEREFVAGIN
WSDGTTSYKDSVKGRFTISRDNAKNTVYLQMDSLKPEDTAVYYCNAHLSTGQEGP
GEYFGMDYWGKGTQVTVSSTS

>Re6H06

GSQVQLVESGGGLVQPGGSLRLSCAASGVTLDYYAIGWFRQAPGKEREGVSCTS
SSDGSTYYADSVKGRFTISRDNAKNTVYLQMNSLKPEDTGVYYCAVVPQTYYGGK
YYSQCTANGMDYWGKGTLVTVSSTS

>Re6H10

GSQVQLVESGGGLVQPGGSLRLSCAASGFTFSSYAMGWYRQAPGKECEFVAVITI
TGGSTNYADSVKGRFTISRDNAKNTVYLQMNSLKPEDTAVYYCNPDPGCRGGGQ
GTLVTVSSTS

>Re7A01

GSQVQLVESGGGLVQAGGSLRLSCAASGRTFSSYAMGWFRQAPGKEREFVATIS
FSGSTSYAGHVKGRFTISRDNAKNTVYLQMNSLKPEDTAVYYCHAVTRASDQDGG
MDYWGQGTQVTVSSTS

>Re7B01

GSQVQLVESGGGLVQPGGSLRLSCGASGFTLDYYAIGWFRQAPGKEREGVSRIRS
NDGSTDYADSVKGRFTISRDNAKNTVYLQMNSLKPEDTAVYYCAYGPLTKYGSSW
YWPYEYDYWGQGTQVTVSSTS

>Re7D05

GSQVQLVESGGGLVQAGGSLRLSCAASGRTFSSYAMGWFRQAPGKEREFVATIS
WSGGSTSYADSVKGRFTISRDNAKNTVYLQMNSLKPEDTAVYYCNAVTHHSDQD
GGMDYWGKGTLVTVSSTS

>Re7E02

GSQVQLVESGGGLVQAGGSLRLSCAASGFTLDYYAIGWFRQAPGKEREGVSYIRS
SDGTTYYADSVKGRFTISRDNAKNTVYLQMNSLKPEDTAVYYCAADEAYYSELGW
ESPWGWSYWGQGTQVTVSSTS

>Re7H02

GSQVQLVESGGGLVQAGGSLRLSCAASGRAFESAPMSWFRQAPGKEREFVASVS
WSGDSTNYADSVKGRFTISRDNAENTGYLQMNSLKPEDTAVYYCKRGPYWGQGT
QVTVSSTS

>Re8A03

GSQVQLVESGGGLVQPGGSLRLSCAASGRITGFNGMGWYRQTPGKQRELVASIT
NGGITKYADSVKGRFTISRDNAKNTVYLQMNSLKPEDTAVYLCYFWRPEFPNLYW
GQGTQVTVSSTS

>Re8A06

GSQVQLVESGGGLVQAGGSLRLSCAASGSIFSINAMGWYRQAPGKERELVAAMG
SSGWINYADSVKGRLTISRDNAKNTLYLQMNSLKPEDTAVYYCRGTGGVGPTSAD
YWGQGTQVTVSSTS

>Re8C06

GSQVQLVESGGGLVQAGGSLRLSCAASGRTDTIYNMGWFRQAPGKEREFVAAIS
WSDGKTTFADSVKGRFTISRDNAKNTVYLQMNSLKPEDTANYYCAAKAFLVAGRS
LEEYDYSGQGTQVTVSSTS

>Re8E12

GSQVQLVESGGGSVQPGGSLRLSCKVSGFTSDVDLRNYLVSWNRQAPGKERELV
AAITPTVISGGNTNYADSVKGRFTISRDYSKSTVYLQMNSLNPEDTAVYYCKVGVY
WGQGTQVTVSSTS

>Re8F03

GSQVQLVESGGGLVQPGGSLTLSCKVSGLTSYVDLRNYLVSWYRQGPGKERELV
AAITPTAITGGSTNYADSVKGRFTISRDYSKSTVYLQMNSLNPEDTAVYSCKVGVY
WGQGTQVTVSSTS

>Re9B09

GSQVQLVESGGGLVQPGGSLRLSCAASGFTLDYYAIGWFRQAPGKEREGVSRISS
SDGSTDYADSVKGRFTISRDNAKNTVYLQMNSLKPEDTAVYYCATVPGTYYSGNW
YYTWHPEAVDYWGKGTQVTVSSTS

>Re9B10

GSQVQLVESGGGLVQPGGSLRLSCAASGRMFGVYRMGWFRQAPGKEREFVAGI
STSVGTTNYADSVKGRFTISRDNAKNTVYLQMNSLKPEDTAVYYCAARDPTTYEYD
YWGQGTQVTVSSTS

>Re9C07

GSQVQLVESGGGLVQAGGSLRLSCAASGRTFSRYAMGWFRQAPGKEREFVAAIT
WNADTTYYADSVKGRFTISRDNAKNTVYLQMNSLKPEDTAVYYCAAGGNHYYSRS
YYSSLEYDHWGQGTQVTVSSTS

>Re9C08

GSQVQLVESGGGLVQPGGSLRLSCAVSGNIFGITAWDWHRQAPGKQRELVAHITS
RGDTYYLDSVKGRFAISRDHAKNTLSLQMNSLKPEDTAVYYCYLRTFGPPNDHWG
QGTQVTVSSTS

>Re9D02

GSQVQLVESGGGLVQAGGSLRLSCAASGRTFSNYAMGWFRQAPGKEREFVAAIS
WGGDTTYYADSLKGRFTISRDNAKNTVYLQMNSLKPEDTAVYYCAADRGLSYYYD
RVTEYDYWGQGTQVTVSSTS

>Re9G05

GSQVQLVESGGGLVQPGGSLRLSCAVSGNISSITAWDWHRQAPGKQRELVAHITS
RGDTMYLDSVKGRFAISRDHAKNTLSLQMNSLKPEDTAVYYCYLRTFGPPYDYWG
QGTQVTVSSTS

>Re9G12

GSQVQLVESGGGLVQAGGSLRLSCAASGRTFSSYVMGWFRQAPGKEREFVAHIS
WSGDSTYYADSVKGRFTIFRDNAKNTAYLQMNSLKPEDTAVYYCAADRGASYYYT
WASEYNYWGQGTQVTVSSTS

>Re9H01

GSQVQLVESGGGLVQAGDSLRLSCAASGNIFSINAMGWYRQAPGKQRELVAFITS
RGSTNYTDSVKGRFTISRDTAKDTVYLQMNSLKPEDTAVYFCRGGYSDYDIYFGS
WGQGTQVTVSSTS

>Re10B02

GSQVQLVESGGGLVQPGGSLRLSCATSGSISSYRMGWYRQGPGKQRELVAFITG
GITDYIDSVKGRFTISRDNAKNTMYLQMNSLKPEDTAVYYCNADPPLFNWGQGTQV
TVSSTS

>Re10B10

GSQVQLVESGGGLVQPGGSLRLSCAASGFTLDYYAIGWFRQAPGKEREGVSRIRS
SDGSTTYADSVKGRFTISRDNAKNTVYLQMNSLKPEDTAVYYCAYGPLTKYGSSW
YWPYEYDYWGQGTQVTVSSTS

>Re10F10

GSQVQLVESGGGLVQPGGSLRLSCAASGFTLDYYAIGWFRQAPGKEREGVSRIRN
NDGSTDYADSVKGRFTISRDNAKNTVYLQMNSLKPEDTAVYYCAYGPLTKYGSSW
YWPYEYDYWGQGTQVTVSSTS

>Re11C10

GSQVQLVESGGGSVEAGGSLRLSCAASGRTLDNYNAVWFRQAPGKEREFVAAIN
WNGSNTSYGNSVKGRFAVSRDNAKNTVYLQMNNLKHEDTAEYLCYTVGPEGDYW
GQGTQVTVSSTS

>Re11E11

GSQVQLVESGGGSVEAGGSLRLSCAASGRTFNNYNIVWFRQAPGKEREFVAAIN
WNGGSTSYANSVKGRFAVSRDNAKNTVYLQMNNLKHEDTAEYLCYTVGPEGDYW
GQGTQVTVSSTS

>Re11F07

GSQVQLVESGGGLVQAGGSLRLSCAASGRAFSSGTMGWFRQAPGKEREFVATIS
WSGGSTSYARSVKGRFTISGDNAENTVYLQMNSLKPEDTAVYYCYAVSSGSDYDG
GMDYWGKGTLVTVSSTS

>Re11F11

GSQVQLVESGGGLVQPGGSLRLSCAASGFTFSNYHMSWYRQAPGKGRELVADIT
SGGDYTHYADSVKGRFTVSRDNPKNTLYLQMNSLKPEDTAVYHCHVRIFGPGFPV
DYRGQGTQVTVSSTS

>Re11G09

GSQVQLVESGGGLVHTGGSLRLSCAASGSIFNIYRMAWYRQAPGKQREKVAIITTY
GLTDYADSVKGRFTISRDNAKNTTYLQMNSLKPDDTAVYYCNTDPPDLGPGYWGQ
GTQVTVSSTS

>Re11H04

GSQVQLVESGGGLVQPGGSLRLSCAASGFTLDYYTIAWFRQAPGKEREGVSCISG
NDGSTYYADSVKGRFTISRDNAKNTVYLQMNSLKPEDTAVYYCAADRGESYYPFR
PSEYHYWGQGTQVTVSSTS

## Examples

### Example 1 - Periplasmic expression and purification of VHH antibody monomers

VHH antibody Re6H06 was expressed with an N-terminal pelB signal sequence and a C-terminal His10 tag from a Kan-ColE1 plasmid harboring a T5/lac promoter in *E. coli* NEBExpress (New England Biolabs). A 125-ml preculture in Terrific Broth (TB) containing 50 $\mu$g/ml Kanamycin was grown overnight at 28 °C to early stationary phase. The culture was then diluted with fresh medium (500 ml, pre-warmed to 37 °C). After 30 minutes of growth at 37 °C, protein expression was induced with 0.05 mM IPTG and growth was continued for 2 hours at 37 °C, whereby the culture reached a final $OD_{600}$ of ~8. Bacteria were harvested by centrifugation and lysed by osmotic shock lysis: cell pellets were resuspended in 14ml 130 mM Tris/HCl pH 8.0, 10 mM EDTA, and sucrose was immediately added to 20% (w/v). After gentle mixing at 23 °C for 30 min, four volumes of ice-cold water were added and mixing was continued at 4 °C for 30 min. 20 mM Tris/HCl pH 7.5, 50 mM NaCl and 20 mM imidazole were added to the cell suspension. Periplasmic extract was then recovered as the supernatant of two consecutive centrifugation steps at 4 °C: a low-speed spin at 4000 xg (20 min, F13 rotor, Thermo Fisher Scientific) and a high-speed spin at 38000 rpm (~1 hour, T647.5 rotor). The VHH antibody was purified at 4 °C via $Ni^{2+}$ EDTA-amide chelate affinity chromatography (1ml matrix). Beads were washed with ten column volumes of 50 mM Tris/HCl pH 7.5, 300 mM NaCl, 20 mM imidazole, 0.2% (w/v) Triton X-100 and ten column volumes of buffer lacking detergent. After elution with 50 mM Tris/HCl pH 7.5, 300 mM NaCl, 500 mM imidazole, the buffer was exchanged to 50 mM Tris/HCl pH 7.5, 300 mM NaCl, 250 mM sucrose via a PD 10 desalting column (GE Healthcare). Aliquots were frozen in liquid nitrogen and stored at -80 °C. This expression/ purification strategy was used for all VHH antibodies containing four cysteines and thus two disulfide bonds (Re5E03, Re5E11, Re5G05, Re6E11, Re6F06, Re6G03, Re6H06, Re6H10) as well as for Re5F11.

**Example 2 - Cytoplasmic expression and purification of VHH monomers**

All other VHH antibodies comprising just two cysteines and thus a single disulfide bond were produced as His14-ScSUMO fusions by cytoplasmic expression in *E.coli* NEBExpress Shuffle, which allows forming disulfide bonds in the bacterial cytoplasm. In brief, 125 ml pre-cultures were grown overnight at 35°C in TB+50 µg/ml kanamycin in 5 liter flasks to early stationary phase. They were then diluted with 250ml fresh medium, shifted to 21°C and induced for 5 hours with 0.08 mM IPTG. 5 mM EDTA was added, bacteria were pelleted, resuspended in 50 mM Tris/HCl pH 7.5, 20 mM imidazole/HCl pH 7.5, 300 mM NaCl, frozen in liquid nitrogen and lysed by thawing plus sonication. Insoluble material was removed by ultracentrifugation at 38000 rpm (~1 hour, T647.5 rotor).

The supernatant was applied to a 1 ml Ni$^{2+}$ EDTA-amide chelate column. The matrix was sequentially washed in resuspension buffer, resuspension buffer + 0.2% TritonX100, resuspension buffer + 700 mM NaCl, low salt buffer (resuspension buffer minus NaCl), and protease buffer (resuspension buffer with an imidazole concentration lowered to 10mM and supplemented with 250 mM sucrose). The VHH antibody was finally eluted by cleaving the His14-SUMO-tag using 100 nM *S. cerevisiae* Ulp1 for 2 hours at room temperature. The eluted VHH antibodies were frozen in liquid nitrogen and stored an -80°C until further use.

**Example 3 - Cytoplasmic expression and purification of VHH trimers**

The VHH trimers listed in Table 3 were expressed and purified as described for the VHH monomers, the only difference being that the VHH sequence was C-terminally extended by a 31 residues long spacer (in single letter code: EGSEGPESSDGSDSTDPGEQGEGADASDGSE) (SEQ ID NO: 197) and by the 57 residues long human collagen XVIII NC trimerization domain (in single letter code: GASSGVRLWATRQAMLGQVHEVPEGWLIFVAEQEELYVRVQNGFRKVQLEARTP LPR) (SEQ ID NO:198).

**Example 4 - Spike protein-staining in transfected cells**

To prepare fluorophore-labelled probes, monomeric VHH antibodies were expressed with two additional cysteines, one at the N- and one at the C-terminus. These were then used for labelling with fluorophore-maleimides as described by Pleiner et al., 2015. The trimerized VHH antibody Re6A11 was labelled through a single N-terminal cysteine. Labelings were essentially quantitative as judged by ratiometric UV-VIS spectroscopy and electrophoretic size shifts.

HeLa cells were cultivated in DMEM+ 5% FCS, seeded in 10-well slides, and transiently transfected with a plasmid allowing expression of the SARS-CoV-2 spike protein from a CMV promoter and using the PolyJet transfection reagent according to the manufacturer's instruction (SignaGen). 2 days later, cells were fixed for 5 minutes with 4% paraformaldehyde (PFA) in PBS, washed in PBS, permeabilized with 0.5% saponin, blocked with 5% BSA in PBS, and incubated with gentle shaking for 60 min with fluorophore-labelled VHH antibodies (in PBS+ 1% BSA) at concentrations given in the figures. Following washes with PBS and counterstaining with DAPI, images were taken by confocal laser scanning microscopy.

**Example 5 - Virus infection and neutralization assays**

Virus stocks were prepared as supernatants from Vero E6 cells infected with SARS-CoV-2. The virus stocks contained between $10^{11}$ and $10^{12}$ copies of the virus genome per ml, as determined by reverse transcription and quantitative PCR (qRT-PCR) according to a standard protocol (Corman et al., 2020). $2*10^6$ virus copies were diluted in 100 µl of cell culture media, DMEM/ 2% FBS, in the presence or absence of varying concentrations of the VHH antibodies under study. After 60 min incubation at 37 °C, this was added to a monolayer of Vero E6 cells, i.e. 5000 cells in a well of a standard 96 well cell culture plate. After 48 or 72 hours of incubation, the supernatants of the cells were harvested. To quantify the amount of newly produced virus particles, the RNA from this material was isolated by the Trizol method, followed by qRT-PCR.

**Example 6 - Spike protein detection in SARS-CoV-2 infected cells**

Stainings were performed with Vero E6 cells infected for 2 days as described above. Fixation was with 4% paraformaldehyde (PFA) for 2 hours. This long period of fixation was due to safety reasons to make sure that no infectious

EP 3 944 877 A1

material remained. As a consequence, only highly fixation-resistant epitopes remain visible - explaining the difference between the staining of transfected and virus-infected cells in Table 1. The fluorescence staining themselves were performed as described above. Figure 3 shows epifluorescence images. The evaluations of Table 1 are based on confocal laser scans.

**References**

Alvarez-Cienfuegos, A., Nuñez-Prado, N., Compte, M., Cuesta, A. M., Blanco-Toribio, A., Harwood, S. L., Villate, M., Merino, N., Bonet, J., Navarro, R., Muñoz-Briones, C., Sørensen, K. M., Mølgaard, K., Oliva, B., Sanz, L., Blanco, F. J., and Alvarez-Vallina, L. (2016). Intramolecular trimerization, a novel strategy for making multispecific antibodies with controlled orientation of the antigen binding domains. Sci Rep 6, 28643.

Arbabi Ghahroudi, M., Desmyter, A., Wyns, L., Hamers, R., and Muyldermans, S. (1997). Selection and identification of single domain antibody fragments from camel heavy-chain antibodies. FEBS Lett 414, 521-526.

Bar-On, Y. M., Flamholz, A., Phillips, R., and Milo, R. (2020). SARS-CoV-2 (COVID-19) by the numbers. eLife 9,

Bogin, O., Kvansakul, M., Rom, E., Singer, J., Yayon, A., and Hohenester, E. (2002). Insight into Schmid metaphyseal chondrodysplasia from the crystal structure of the collagen X NC1 domain trimer. Structure 10, 165-173.

Boudko, S. P., Sasaki, T., Engel, J., Lerch, T. F., Nix, J., Chapman, M. S., and Bächinger, H. P. (2009). Crystal structure of human collagen XVIII trimerization domain: A novel collagen trimerization Fold. J Mol Biol 392, 787-802.

Chen, C., Zhang, Y., Huang, J., Yin, P., Cheng, Z., Wu, J., Chen, S., Zhang, Y., Chen, B., Lu, M., Luo, Y., Ju, L., Zhang, J., and Wang, X. (2020a). Favipiravir versus Arbidol for COVID-19: A Randomized Clinical Trial.

Chen, X., Zhao, B., Qu, Y., Chen, Y., Xiong, J., Feng, Y., Men, D., Huang, Q., Liu, Y., Yang, B., Ding, J., and Li, F. (2020b). Detectable serum SARS-CoV-2 viral load (RNAaemia) is closely correlated with drastically elevated interleukin 6 (IL-6) level in critically ill COVID-19 patients. Clin Infect Dis

Chug, H., Trakhanov, S., Hülsmann, B. B., Pleiner, T., and Gorlich, D. (2015). Crystal structure of the metazoan Nup62•Nup58•Nup54 nucleoporin complex. Science 350, 106-110.

Corman, V. M., Landt, O., Kaiser, M., Molenkamp, R., Meijer, A., Chu, D. K., Bleicker, T., Brünink, S., Schneider, J., Schmidt, M. L., Mulders, D. G., Haagmans, B. L., van der Veer, B., van den Brink, S., Wijsman, L., Goderski, G., Romette, J. L., Ellis, J., Zambon, M., Peiris, M., Goossens, H., Reusken, C., Koopmans, M. P., and Drosten, C. (2020). Detection of 2019 novel coronavirus (2019-nCoV) by real-time RT-PCR. Euro Surveill 25,

Duan, K., Liu, B., Li, C., Zhang, H., Yu, T., Qu, J., Zhou, M., Chen, L., Meng, S., Hu, Y., Peng, C., Yuan, M., Huang, J., Wang, Z., Yu, J., Gao, X., Wang, D., Yu, X., Li, L., Zhang, J., Wu, X., Li, B., Xu, Y., Chen, W., Peng, Y., Hu, Y., Lin, L., Liu, X., Huang, S., Zhou, Z., Zhang, L., Wang, Y., Zhang, Z., Deng, K., Xia, Z., Gong, Q., Zhang, W., Zheng, X., Liu, Y., Yang, H., Zhou, D., Yu, D., Hou, J., Shi, Z., Chen, S., Chen, Z., Zhang, X., and Yang, X. (2020). Effectiveness of convalescent plasma therapy in severe COVID-19 patients. Proceedings of the National Academy of Sciences 117, 9490-9496.

Emeny, J. M., and Morgan, M. J. (1979). Regulation of the interferon system: evidence that Vero cells have a genetic defect in interferon production. J Gen Virol 43, 247-252.

Hoefman, S., Ottevaere, I., Baumeister, J., and Sargentini-Maier, M. (2015). Pre-Clinical Intravenous Serum Pharmacokinetics of Albumin Binding and Non-Half-Life Extended Nanobodies®. Antibodies 4, 141-156.

Kliks, S. C., Nisalak, A., Brandt, W. E., Wahl, L., and Burke, D. S. (1989). Antibody-dependent enhancement of dengue virus growth in human monocytes as a risk factor for dengue hemorrhagic fever. Am J Trop Med Hyg 40, 444-451.

Li, W., Moore, M. J., Vasilieva, N., Sui, J., Wong, S. K., Berne, M. A., Somasundaran, M., Sullivan, J. L., Luzuriaga, K., Greenough, T. C., Choe, H., and Farzan, M. (2003). Angiotensin-converting enzyme 2 is a functional receptor for the SARS coronavirus. Nature 426, 450-454.

Littaua, R., Kurane, I., and Ennis, F. A. (1990). Human IgG Fc receptor II mediates antibody-dependent enhancement of dengue virus infection. J Immunol 144, 3183-3186.

Magro, C., Mulvey, J. J., Berlin, D., Nuovo, G., Salvatore, S., Harp, J., Baxter-Stoltzfus, A., and Laurence, J. (2020). Complement associated microvascular injury and thrombosis in the pathogenesis of severe COVID-19 infection: a report of five cases. Transl Res

Mehta, P., McAuley, D. F., Brown, M., Sanchez, E., Tattersall, R. S., Manson, J. J., and HLH Across Speciality Collaboration, U. K. (2020). COVID-19: consider cytokine storm syndromes and immunosuppression. Lancet 395, 1033-1034.

Murphy, K., and Weaver, C. (2016). Janeway's Immunobiology Garland Science).

Pleiner, T., Bates, M., and Gorlich, D. (2018). A toolbox of anti-mouse and anti-rabbit IgG secondary nanobodies. J Cell Biol 217, 1143-1154.

Pleiner, T., Bates, M., Trakhanov, S., Lee, C. T., Schliep, J. E., Chug, H., Böhning, M., Stark, H., Urlaub, H., and Gorlich, D. (2015). Nanobodies: site-specific labeling for super-resolution imaging, rapid epitope-mapping and native protein complex isolation. Elife 4, e11349.

Rasmussen, S. G., Choi, H. J., Fung, J. J., Pardon, E., Casarosa, P., Chae, P. S., Devree, B. T., Rosenbaum, D. M., Thian, F. S., Kobilka, T. S., Schnapp, A., Konetzki, I., Sunahara, R. K., Gellman, S. H., Pautsch, A., Steyaert, J., Weis, W. I., and Kobilka, B. K. (2011). Structure of a nanobody-stabilized active state of the $\beta$(2) adrenoceptor. Nature 469, 175-180.

Wang, C., Li, W., Drabek, D., Okba, N. M. A., van Haperen, R., Osterhaus, A. D. M. E., van Kuppeveld, F. J. M., Haagmans, B. L., Grosveld, F., and Bosch, B. J. (2020). A human monoclonal antibody blocking SARS-CoV-2 infection. Nat Commun 11, 2251.

Wirz, J. A., Boudko, S. P., Lerch, T. F., Chapman, M. S., and Bächinger, H. P. (2011). Crystal structure of the human collagen XV trimerization domain: A potent trimerizing unit common to multiplexin collagens. Matrix Biology 30, 9-15.

Wrapp, D., Wang, N., Corbett, K. S., Goldsmith, J. A., Hsieh, C. L., Abiona, O., Graham, B. S., and McLellan, J. S. (2020). Cryo-EM structure of the 2019-nCoV spike in the prefusion conformation. Science 367, 1260-1263.

Zhang, L., Jackson, C. B., Mou, H., Ojha, A., Rangarajan, E. S., Izard, T., Farzan, M., and Choe, H. (2020). The D614G mutation in the SARS-CoV-2 spike protein reduces S1 shedding and increases infectivity. bioRxiv https://doi.org/10.1101/2020.06.12.148726.

## SEQUENCE LISTING

<110> Max-Planck-Gesellschaft zur Förderung der Wissenschaften
e.V.

<120> Therapeutic and diagnostic VHH antibodies against SARS-CoV-2 and
methods for their enhancement

<130> 70956P EP

<160> 198

<170> PatentIn version 3.5

<210> 1
<211> 123
<212> PRT
<213> artificial sequence

<220>
<223> Re5A08

<400> 1

Gly Ser Gln Val Gln Leu Val Glu Ser Gly Gly Gly Leu Val Gln Ala
1               5                   10                  15

Gly Gly Ser Leu Arg Leu Ser Cys Thr Ala Ser Gly His Thr Phe Thr
            20                  25                  30

Ala Asn Arg Met Gly Trp Phe Arg Gln Ala Pro Gly Lys Glu Arg Glu
            35                  40                  45

Phe Val Ala Ala Ile Asn Trp Gly Gly Asp Ser Thr Asn Tyr Val Asp
        50                  55                  60

Ser Val Lys Gly Arg Phe Thr Ile Ser Arg Asp Ile Ala Lys Asn Thr
65                  70                  75                  80

Val Tyr Leu Gln Met Asn Ser Leu Lys Pro Glu Asp Thr Ala Val Tyr
                85                  90                  95

Phe Cys Ala Ala Arg Asn His Val Thr Gly Glu Phe Asp Ser Trp Gly
            100                 105                 110

Gln Gly Thr Gln Val Thr Val Ser Ser Thr Ser
            115                 120

<210> 2
<211> 9
<212> PRT
<213> artificial sequence

<220>
<223> Re5A08 CDR1

<400> 2

His Thr Phe Thr Ala Asn Arg Met Gly
1               5


<210> 3
<211> 15
<212> PRT
<213> artificial sequence

<220>
<223> Re5A08 CDR2

<400> 3

Phe Val Ala Ala Ile Asn Trp Gly Gly Asp Ser Thr Asn Tyr Val
1               5               10              15


<210> 4
<211> 13
<212> PRT
<213> artificial sequence

<220>
<223> Re5A08 CDR3

<400> 4

Ala Ala Arg Asn His Val Thr Gly Glu Phe Asp Ser Trp
1               5               10


<210> 5
<211> 124
<212> PRT
<213> artificial sequence

<220>
<223> Re5B06

<400> 5

Gly Ser Gln Val Gln Leu Val Glu Ser Gly Gly Gly Leu Val Gln Pro
1               5               10              15


Gly Gly Ser Leu Arg Leu Ser Cys Ala Ala Ser Gly Ser Ile Arg Ser
            20              25              30


Ile Tyr Ala Thr Val Trp Phe Arg Gln Ala Pro Gly Lys Glu His Glu
            35              40              45


Trp Val Gly Ser Ile Thr Ser Ser Asn Val Thr Thr Tyr Ala Asp Ser
        50              55              60


Val Lys Gly Arg Phe Thr Ile Ser Arg Asp Asn Ala Lys Lys Thr Val
65              70              75              80

```
Tyr Leu Gln Met Asn Ser Leu Lys Pro Glu Asp Thr Ala Leu Tyr Tyr
                85                  90                  95


Cys Asn Val His Phe Ala Ser Glu Tyr Ser Asp Tyr Ala Gln Ile Gln
            100                 105                 110


Gly Gln Gly Thr Gln Val Thr Val Ser Ser Thr Ser
            115                 120
```

<210> 6
<211> 9
<212> PRT
<213> artificial sequence

<220>
<223> Re5B06 CDR1

<400> 6

```
Ser Ile Arg Ser Ile Tyr Ala Thr Val
1               5
```

<210> 7
<211> 14
<212> PRT
<213> artificial sequence

<220>
<223> Re5B06 CDR2

<400> 7

```
Trp Val Gly Ser Ile Thr Ser Ser Asn Val Thr Thr Tyr Ala
1               5                   10
```

<210> 8
<211> 15
<212> PRT
<213> artificial sequence

<220>
<223> Re5B06 CDR3

<400> 8

```
Asn Val His Phe Ala Ser Glu Tyr Ser Asp Tyr Ala Gln Ile Gln
1               5                   10                  15
```

<210> 9
<211> 126
<212> PRT
<213> artificial sequence

<220>
<223> Re5C01
```

<400> 9

```
Gly Ser Gln Val Gln Leu Val Glu Ser Gly Gly Gly Leu Val Gln Ala
1               5                   10                  15

Gly Gly Ser Leu Arg Leu Ser Cys Gly Val Ser Gly Arg Thr Phe Ser
            20                  25                  30

Ser Tyr Ala Met Gly Trp Phe Arg Gln Ala Pro Gly Lys Glu Arg Glu
        35                  40                  45

Phe Val Ala Thr Ile Ser Trp Ser Gly Gly Thr Thr Asn Tyr Ala His
        50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Val Glu Asp Thr Ala Val Tyr
                85                  90                  95

Tyr Cys Tyr Ala Val Ser Ser Gly Ser Asp Tyr Asp Gly Gly Met Asp
            100                 105                 110

Tyr Trp Gly Lys Gly Thr Gln Val Thr Val Ser Ser Thr Ser
            115                 120                 125
```

<210> 10
<211> 9
<212> PRT
<213> artificial sequence

<220>
<223> Re5C01 CDR1

<400> 10

```
Arg Thr Phe Ser Ser Tyr Ala Met Gly
1               5
```

<210> 11
<211> 15
<212> PRT
<213> artificial sequence

<220>
<223> Re5C01 CDR2

<400> 11

```
Phe Val Ala Thr Ile Ser Trp Ser Gly Gly Thr Thr Asn Tyr Ala
1               5                   10                  15
```

33

<210> 12
<211> 16
<212> PRT
<213> artificial sequence

<220>
<223> Re5C01 CDR3

<400> 12

Tyr Ala Val Ser Ser Gly Ser Asp Tyr Asp Gly Gly Met Asp Tyr Trp
1               5                   10                  15


<210> 13
<211> 120
<212> PRT
<213> artificial sequence

<220>
<223> Re5C08

<400> 13

Gly Ser Gln Val Gln Leu Val Glu Ser Gly Gly Gly Ser Val Glu Ala
1               5                   10                  15


Gly Gly Ser Leu Arg Leu Ser Cys Ala Ala Ser Gly Arg Thr Phe Asn
            20                  25                  30


Asp Tyr Asn Met Val Trp Phe Arg Gln Ala Pro Gly Lys Glu Arg Glu
            35                  40                  45


Phe Val Ala Ala Ile Lys Trp Asn Gly Gly Asn Thr Ser Tyr Ala Asp
            50                  55                  60


Ser Val Lys Gly Arg Phe Ala Val Ser Arg Asp Asn Ala Lys Asn Thr
65                  70                  75                  80


Val Tyr Leu Gln Met Asn Asn Leu Lys His Glu Asp Thr Ala Glu Tyr
                85                  90                  95


Leu Cys Tyr Thr Val Gly Pro Glu Gly Asp Tyr Trp Gly Gln Gly Thr
            100                 105                 110


Gln Val Thr Val Ser Ser Thr Ser
            115                 120


<210> 14
<211> 9
<212> PRT
<213> artificial sequence

<220>
<223> Re5C08 CDR1

<400> 14

Arg Thr Phe Asn Asp Tyr Asn Met Val
1               5


<210> 15
<211> 15
<212> PRT
<213> artificial sequence

<220>
<223> Re5C08 CDR2

<400> 15

Phe Val Ala Ala Ile Lys Trp Asn Gly Gly Asn Thr Ser Tyr Ala
1               5                   10                  15


<210> 16
<211> 10
<212> PRT
<213> artificial sequence

<220>
<223> Re5C08 CDR3

<400> 16

Tyr Thr Val Gly Pro Glu Gly Asp Tyr Trp
1               5                   10


<210> 17
<211> 131
<212> PRT
<213> artificial sequence

<220>
<223> Re5D06

<400> 17

Gly Ser Gln Val Gln Leu Val Glu Ser Gly Gly Gly Leu Val Gln Pro
1               5                   10                  15

Gly Gly Ser Leu Arg Leu Ser Cys Ala Ala Ser Gly Ile Thr Leu Asp
                20                  25                  30

Tyr Tyr Ala Ile Gly Trp Phe Arg Gln Ala Pro Gly Lys Glu Arg Glu
                35                  40                  45

Gly Val Ser Arg Ile Arg Ser Ser Asp Gly Ser Thr Asn Tyr Ala Asp
                50                  55                  60

Ser Val Lys Gly Arg Phe Thr Met Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Tyr Gly Pro Leu Thr Lys Tyr Gly Ser Ser Trp Tyr Trp
            100                 105                 110

Pro Tyr Glu Tyr Asp Tyr Trp Gly Gln Gly Thr Gln Val Thr Val Ser
        115                 120                 125

Ser Thr Ser
    130

<210>  18
<211>  9
<212>  PRT
<213>  artificial sequence

<220>
<223>  Re5D06 CDR1

<400>  18

Ile Thr Leu Asp Tyr Tyr Ala Ile Gly
1               5

<210>  19
<211>  15
<212>  PRT
<213>  artificial sequence

<220>
<223>  Re5D06 CDR2

<400>  19

Gly Val Ser Arg Ile Arg Ser Ser Asp Gly Ser Thr Asn Tyr Ala
1               5                   10                  15

<210>  20
<211>  21
<212>  PRT
<213>  artificial sequence

<220>
<223>  Re5D06 CDR3

<400>  20

Ala Tyr Gly Pro Leu Thr Lys Tyr Gly Ser Ser Trp Tyr Trp Pro Tyr
1               5                   10                  15

Glu Tyr Asp Tyr Trp
            20

```
<210>  21
<211>  134
<212>  PRT
<213>  artificial sequence

<220>
<223>  Re5E03

<400>  21


Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
            20                  25                  30


Tyr Tyr Ala Ile Gly Trp Phe Arg Gln Ala Pro Gly Lys Glu Arg Glu
            35                  40                  45


Gly Val Ser Cys Ile Ser Asn Ser Asp Gly Ser Thr Arg Tyr Ala Asp
        50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95


Tyr Cys Ala Gly Gly Pro Gln Thr Tyr Tyr Ser Gly Ser Tyr Tyr Tyr
            100                 105                 110


Thr Cys Ala Glu Gly Ala Met Asp Tyr Trp Gly Lys Gly Thr Leu Val
            115                 120                 125


Thr Val Ser Ser Thr Ser
        130


<210>  22
<211>  9
<212>  PRT
<213>  artificial sequence

<220>
<223>  Re5E03 CDR1

<400>  22


Phe Thr Leu Asp Tyr Tyr Ala Ile Gly
1               5


<210>  23
<211>  15
```

```
<212>   PRT
<213>   artificial sequence

<220>
<223>   Re5E03 CDR2

<400>   23

Gly Val Ser Cys Ile Ser Asn Ser Asp Gly Ser Thr Arg Tyr Ala
1               5                   10                  15


<210>   24
<211>   24
<212>   PRT
<213>   artificial sequence

<220>
<223>   Re5E03 CDR3

<400>   24

Ala Gly Gly Pro Gln Thr Tyr Tyr Ser Gly Ser Tyr Tyr Tyr Thr Cys
1               5                   10                  15


Ala Glu Gly Ala Met Asp Tyr Trp
                20


<210>   25
<211>   134
<212>   PRT
<213>   artificial sequence

<220>
<223>   Re5E11

<400>   25

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
                20                  25                  30


Tyr Tyr Ala Ile Gly Trp Phe Arg Gln Ala Pro Gly Lys Glu Arg Glu
                35                  40                  45


Gly Val Ser Cys Ile Ser Ser Ser Asp Gly Arg Thr Tyr Tyr Ala Asp
                50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95
```

Tyr Cys Ala Thr Ala Pro Leu Thr Tyr Tyr Ser Gly Ser Trp Tyr Leu
100                 105                 110

Thr Cys Asn Ser Asp Ala Met Asp Tyr Trp Gly Lys Gly Thr Leu Val
115                 120                 125

Thr Val Ser Ser Thr Ser
130

<210> 26
<211> 9
<212> PRT
<213> artificial sequence

<220>
<223> Re5E11 CDR1

<400> 26

Phe Thr Leu Asp Tyr Tyr Ala Ile Gly
1               5

<210> 27
<211> 15
<212> PRT
<213> artificial sequence

<220>
<223> Re5E11 CDR2

<400> 27

Gly Val Ser Cys Ile Ser Ser Ser Asp Gly Arg Thr Tyr Tyr Ala
1               5               10                  15

<210> 28
<211> 24
<212> PRT
<213> artificial sequence

<220>
<223> Re5E11 CDR3

<400> 28

Ala Thr Ala Pro Leu Thr Tyr Tyr Ser Gly Ser Trp Tyr Leu Thr Cys
1               5               10                  15

Asn Ser Asp Ala Met Asp Tyr Trp
            20

<210> 29
<211> 118
<212> PRT
<213> artificial sequence

```
<220>
<223>  Re5F10

<400>  29

Gly Ser Gln Val Gln Leu Val Glu Ser Gly Gly Gly Leu Val Gln Pro
1               5                   10                  15


Gly Gly Ser Leu Arg Leu Ser Cys Val Ala Ser Gly Phe Thr Phe Ser
            20                  25                  30


Ser Phe Ala Met Gly Trp Tyr Arg Gln Ala Pro Gly Lys Glu Cys Glu
            35                  40                  45


Trp Val Ala Thr Ile Thr Ile Thr Gly Gly Ser Thr Asn Tyr Ala Asp
        50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95


Tyr Cys Asn Pro Asp Pro Gly Cys Arg Arg Gly Gln Gly Thr Gln Val
                100                 105                 110


Thr Val Ser Ser Thr Ser
            115


<210>  30
<211>  9
<212>  PRT
<213>  artificial sequence

<220>
<223>  Re5F10 CDR1

<400>  30

Phe Thr Phe Ser Ser Phe Ala Met Gly
1               5


<210>  31
<211>  15
<212>  PRT
<213>  artificial sequence

<220>
<223>  Re5F10 CDR2

<400>  31

Trp Val Ala Thr Ile Thr Ile Thr Gly Gly Ser Thr Asn Tyr Ala
```

```
                1               5                       10                      15


        <210>   32
        <211>   8
        <212>   PRT
        <213>   artificial sequence

        <220>
        <223>   Re5F10 CDR3

        <400>   32

        Asn Pro Asp Pro Gly Cys Arg Arg
        1                   5


        <210>   33
        <211>   117
        <212>   PRT
        <213>   artificial sequence

        <220>
        <223>   Re5F11

        <400>   33

        Gly Ser Gln Val Gln Leu Val Glu Ser Gly Gly Ala Leu Val Gln Pro
        1                   5                       10                      15


        Gly Gly Ser Leu Arg Leu Ser Cys Ala Thr Ser Gly Ser Ile Ser Ser
                    20                      25                      30


        Tyr Arg Met Gly Trp Tyr Arg Gln Gly Pro Gly Lys Gln Arg Glu Leu
                    35                      40                      45


        Val Ala Phe Ile Thr Ile Gly Gly Ile Thr Asp Tyr Ile Asp Ser Val
                    50                      55                      60


        Lys Gly Arg Phe Thr Ile Ser Arg Asp Asn Ala Lys Asn Thr Met Tyr
        65                      70                      75                      80


        Leu Gln Met Asn Ser Leu Lys Pro Glu Asp Thr Ala Val Tyr Tyr Cys
                    85                      90                      95


        Asn Ala Asp Pro Pro Leu Phe Asn Trp Gly Gln Gly Thr Gln Val Thr
                    100                     105                     110


        Val Ser Ser Thr Ser
                    115


        <210>   34
        <211>   8
        <212>   PRT
        <213>   artificial sequence
```

```
<220>
<223>  Re5F11 CDR1

<400>  34

Ser Ile Ser Ser Tyr Arg Met Gly
1               5


<210>  35
<211>  14
<212>  PRT
<213>  artificial sequence

<220>
<223>  Re5F11 CDR2

<400>  35

Leu Val Ala Phe Ile Thr Ile Gly Gly Ile Thr Asp Tyr Ile
1               5               10


<210>  36
<211>  9
<212>  PRT
<213>  artificial sequence

<220>
<223>  Re5F11 CDR3

<400>  36

Asn Ala Asp Pro Pro Leu Phe Asn Trp
1               5


<210>  37
<211>  118
<212>  PRT
<213>  artificial sequence

<220>
<223>  Re5G05

<400>  37

Gly Ser Gln Val Gln Leu Val Glu Ser Gly Gly Gly Leu Val Gln Ala
1               5               10                  15


Gly Gly Ser Leu Arg Leu Ser Cys Ala Ala Ser Gly Phe Thr Ala Thr
            20                  25                  30


Ser Tyr Ala Met Gly Trp Tyr Arg Gln Ala Pro Gly Lys Glu Cys Glu
            35                  40                  45


Trp Val Ala Thr Ile Thr Ser Thr Gly Gly Asn Thr Asn Tyr Ala Asp
        50                  55                  60
```

```
Ser Val Lys Gly Arg Phe Thr Ile Ser Arg Asp Asn Ala Lys Asn Thr
65              70              75                  80

Val Tyr Leu Gln Met Asn Ser Leu Lys Pro Glu Asp Thr Ala Val Tyr
                85              90                  95

Tyr Cys Asn Pro Asp Pro Gly Cys Asp Trp Gly Gln Gly Thr Gln Val
            100             105             110

Thr Val Ser Ser Thr Ser
            115
```

```
<210>   38
<211>   9
<212>   PRT
<213>   artificial sequence

<220>
<223>   Re5G05 CDR1

<400>   38

Phe Thr Ala Thr Ser Tyr Ala Met Gly
1               5
```

```
<210>   39
<211>   15
<212>   PRT
<213>   artificial sequence

<220>
<223>   Re5G05 CDR2

<400>   39

Trp Val Ala Thr Ile Thr Ser Thr Gly Gly Asn Thr Asn Tyr Ala
1               5                   10                  15
```

```
<210>   40
<211>   8
<212>   PRT
<213>   artificial sequence

<220>
<223>   Re5G05 CDR3

<400>   40

Asn Pro Asp Pro Gly Cys Asp Trp
1               5
```

```
<210>   41
<211>   127
<212>   PRT
<213>   artificial sequence
```

<220>
<223>  Re6A11

<400>  41

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Asn Asp Ala Leu Gly Trp Phe Arg Gln Ala Pro Arg Lys Glu Arg Glu
        35                  40                  45

Phe Val Ala Ala Ile Asn Trp Asn Ser Gly Thr Tyr Tyr Ala Asp Ser
    50                  55                  60

Val Lys Gly Arg Phe Thr Ile Ser Arg Asp Asn Ala Lys Asn Thr Val
65                  70                  75                  80

Tyr Leu Gln Met Asn Ser Leu Lys Pro Glu Asp Thr Ala Val Tyr Ser
                85                  90                  95

Cys Ala Ala Ala Ser Asp Tyr Gly Leu Pro Arg Glu Asp Phe Leu Tyr
            100                 105                 110

Asp Tyr Trp Gly Gln Gly Thr Leu Val Thr Val Ser Ser Thr Ser
        115                 120                 125

<210>  42
<211>  9
<212>  PRT
<213>  artificial sequence

<220>
<223>  Re6A11 CDR1

<400>  42

Arg Thr Phe Ser Asn Asp Ala Leu Gly
1               5

<210>  43
<211>  14
<212>  PRT
<213>  artificial sequence

<220>
<223>  Re6A11 CDR2

<400>  43

Phe Val Ala Ala Ile Asn Trp Asn Ser Gly Thr Tyr Tyr Ala

1               5                    10

<210> 44
<211> 18
<212> PRT
<213> artificial sequence

<220>
<223> Re6A11 CDR3

<400> 44

Ala Ala Ala Ser Asp Tyr Gly Leu Pro Arg Glu Asp Phe Leu Tyr Asp
1               5                   10                  15

Tyr Trp

<210> 45
<211> 131
<212> PRT
<213> artificial sequence

<220>
<223> Re6B02

<400> 45

Gly Ser Gln Val Gln Leu Val Glu Ser Gly Gly Ala Leu Val Gln Pro
1               5                   10                  15

Gly Gly Ser Leu Arg Leu Ser Cys Val Ala Ser Gly Phe Thr Leu Asp
            20                  25                  30

Tyr Tyr Ala Ile Gly Trp Phe Arg Gln Ala Pro Gly Lys Glu Arg Glu
            35                  40                  45

Gly Val Ser Arg Ile Arg Ser Ser Asp Gly Ser Thr Asn Tyr Ala Asp
            50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Tyr Gly Pro Leu Thr Lys Tyr Gly Ser Ser Trp Tyr Trp
            100                 105                 110

Pro Tyr Glu Tyr Asp Tyr Trp Gly Gln Gly Thr Gln Val Thr Val Ser
            115                 120                 125

Ser Thr Ser
130

<210> 46
<211> 9
<212> PRT
<213> artificial sequence

<220>
<223> Re6B02 CDR1

<400> 46

Phe Thr Leu Asp Tyr Tyr Ala Ile Gly
1               5

<210> 47
<211> 15
<212> PRT
<213> artificial sequence

<220>
<223> Re6B02 CDR2

<400> 47

Gly Val Ser Arg Ile Arg Ser Ser Asp Gly Ser Thr Asn Tyr Ala
1               5                   10                  15

<210> 48
<211> 21
<212> PRT
<213> artificial sequence

<220>
<223> Re6B02 CDR3

<400> 48

Ala Tyr Gly Pro Leu Thr Lys Tyr Gly Ser Ser Trp Tyr Trp Pro Tyr
1               5                   10                  15

Glu Tyr Asp Tyr Trp
            20

<210> 49
<211> 116
<212> PRT
<213> artificial sequence

<220>
<223> Re6B06

<400> 49

Gly Ser Gln Val Gln Leu Val Glu Ser Gly Gly Gly Leu Val Gln Ala
1               5                   10                  15

```
Gly Gly Ser Leu Arg Leu Ser Cys Ala Ala Ser Gly Arg Ala Phe Ser
            20                  25              30

Ser Ala Pro Met Ser Trp Phe Arg Gln Ala Pro Gly Lys Glu Arg Glu
            35                  40              45

Phe Val Ala Ser Val Ser Trp Ser Gly Asp Ser Thr Asn Tyr Ala Asp
            50                  55              60

Ser Val Lys Gly Arg Phe Thr Ile Ser Arg Asp Asn Ala Lys Asn Thr
65                  70              75                  80

Gly Tyr Leu Gln Met Asn Ser Leu Lys Pro Glu Asp Thr Ala Val Tyr
                85                  90                  95

Tyr Cys Lys Arg Gly Pro Tyr Trp Gly Gln Gly Thr Gln Val Thr Val
                100                 105                 110

Ser Ser Thr Ser
            115


<210>   50
<211>   9
<212>   PRT
<213>   artificial sequence

<220>
<223>   Re6B06 CDR1

<400>   50

Arg Ala Phe Ser Ser Ala Pro Met Ser
1               5


<210>   51
<211>   15
<212>   PRT
<213>   artificial sequence

<220>
<223>   Re6B06 CDR2

<400>   51

Phe Val Ala Ser Val Ser Trp Ser Gly Asp Ser Thr Asn Tyr Ala
1               5               10                  15


<210>   52
<211>   6
<212>   PRT
<213>   artificial sequence

<220>
```

<223>  Re6B06 CDR3

<400>  52

Lys Arg Gly Pro Tyr Trp
1               5


<210>  53
<211>  131
<212>  PRT
<213>  artificial sequence

<220>
<223>  Re6B07

<400>  53

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
            20                  25                  30


Tyr Tyr Ala Ile Gly Trp Phe Arg Gln Ala Pro Gly Lys Glu Arg Glu
            35                  40                  45


Gly Val Ser Tyr Ile Arg Ser Ser Asp Gly Thr Thr Tyr Tyr Ala Asp
            50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95


Tyr Cys Ala Ala Asp Glu Ala Tyr Tyr Ser Glu Leu Gly Trp Glu Ser
            100                 105                 110


Pro Trp Gly Trp Ser Tyr Trp Gly Gln Gly Thr Arg Val Thr Val Ser
            115                 120                 125


Ser Thr Ser
        130


<210>  54
<211>  9
<212>  PRT
<213>  artificial sequence

<220>
<223>  Re6B07 CDR1

<400>  54

```
Phe Thr Leu Asp Tyr Tyr Ala Ile Gly
1               5
```

```
<210>   55
<211>   15
<212>   PRT
<213>   artificial sequence

<220>
<223>   Re6B07 CDR2

<400>   55
```

```
Gly Val Ser Tyr Ile Arg Ser Ser Asp Gly Thr Thr Tyr Tyr Ala
1               5               10              15
```

```
<210>   56
<211>   21
<212>   PRT
<213>   artificial sequence

<220>
<223>   Re6B07 CDR3

<400>   56
```

```
Ala Ala Asp Glu Ala Tyr Tyr Ser Glu Leu Gly Trp Glu Ser Pro Trp
1               5               10              15
```

```
Gly Trp Ser Tyr Trp
            20
```

```
<210>   57
<211>   123
<212>   PRT
<213>   artificial sequence

<220>
<223>   Re6D06

<400>   57
```

```
Gly Ser Gln Val Gln Leu Val Glu Ser Gly Gly Gly Leu Val Gln Ala
1               5               10              15
```

```
Gly Ala Ser Leu Arg Leu Ser Cys Ala Ala Ser Gly Arg Met Phe Gly
            20              25              30
```

```
Val Tyr Arg Met Gly Trp Phe Arg Gln Ala Pro Gly Lys Glu Arg Glu
            35              40              45
```

```
Phe Val Ala Gly Ile Ser Thr Ser Val Gly Thr Thr Asn Tyr Ala Asp
        50              55              60
```

```
Ser Val Lys Gly Arg Phe Thr Ile Ser Arg Asp Asn Ala Lys Asn Thr
65          70              75                  80


Val Tyr Leu Gln Met Asn Ser Leu Lys Pro Glu Asp Thr Ala Val Tyr
                85              90                  95


Tyr Cys Ala Ala Arg Asp Pro Thr Thr Tyr Glu Tyr Asp Tyr Trp Gly
            100             105             110


Gln Gly Thr Gln Val Thr Val Ser Ser Thr Ser
        115             120
```

```
<210>  58
<211>  9
<212>  PRT
<213>  artificial sequence

<220>
<223>  Re6D06 CDR1

<400>  58
```

```
Arg Met Phe Gly Val Tyr Arg Met Gly
1               5
```

```
<210>  59
<211>  15
<212>  PRT
<213>  artificial sequence

<220>
<223>  Re6D06 CDR2

<400>  59
```

```
Phe Val Ala Gly Ile Ser Thr Ser Val Gly Thr Thr Asn Tyr Ala
1               5                10                  15
```

```
<210>  60
<211>  13
<212>  PRT
<213>  artificial sequence

<220>
<223>  Re6D06 CDR3

<400>  60
```

```
Ala Ala Arg Asp Pro Thr Thr Tyr Glu Tyr Asp Tyr Trp
1               5                10
```

```
<210>  61
<211>  133
<212>  PRT
<213>  artificial sequence
```

<220>
<223>   Re6D08

<400>   61

Gly Ser Gln Val Gln Leu Val Glu Ser Gly Gly Gly Leu Val Gln Ala
1               5               10              15

Gly Gly Ser Leu Arg Leu Ser Cys Ala Ala Ser Gly Arg Thr Phe Ser
            20              25              30

Ser Tyr Ala Met Gly Trp Phe His Gln Ala Pro Gly Lys Glu Arg Glu
            35              40              45

Phe Val Ala Thr Ile Asn Trp Ser Gly Asp Ser Thr Tyr Tyr Ala Asp
        50              55              60

Ser Val Lys Gly Arg Phe Thr Ile Ser Arg Asp Asn Ala Lys Asn Thr
65              70              75              80

Val Tyr Leu Gln Met Asn Ser Leu Lys Pro Glu Asp Thr Ala Val Tyr
                85              90              95

Tyr Cys Ala Ala Val Val Asp Pro Ser Pro Thr Tyr Tyr Ser Gly Lys
            100             105             110

Tyr Tyr Pro Pro Arg Val Glu Tyr Trp Gly Lys Gly Thr Gln Val Thr
            115             120             125

Val Ser Ser Thr Ser
        130


<210>   62
<211>   9
<212>   PRT
<213>   artificial sequence

<220>
<223>   Re6D08 CDR1

<400>   62

Arg Thr Phe Ser Ser Tyr Ala Met Gly
1               5


<210>   63
<211>   15
<212>   PRT
<213>   artificial sequence

<220>
<223>   Re6D08 CDR2

<400> 63

Phe Val Ala Thr Ile Asn Trp Ser Gly Asp Ser Thr Tyr Tyr Ala
1               5               10               15


<210> 64
<211> 23
<212> PRT
<213> artificial sequence

<220>
<223> Re6D08 CDR3

<400> 64

Ala Ala Val Val Asp Pro Ser Pro Thr Tyr Tyr Ser Gly Lys Tyr Tyr
1               5               10               15


Pro Pro Arg Val Glu Tyr Trp
                20


<210> 65
<211> 120
<212> PRT
<213> artificial sequence

<220>
<223> Re6D09

<400> 65

Gly Ser Gln Val Gln Leu Val Glu Ser Gly Gly Gly Ser Val Glu Ala
1               5               10               15


Gly Gly Ser Leu Arg Leu Ser Cys Ala Ala Ser Gly Arg Thr Phe Asn
                20               25               30


Asn Tyr Asn Met Val Trp Phe Arg Gln Ala Pro Gly Lys Glu Arg Glu
                35               40               45


Phe Val Ala Ala Ile Asn Trp Asn Gly Gly Ser Thr Ser Tyr Ala Ala
                50               55               60


Ser Val Lys Gly Arg Phe Ala Val Ser Ile Asp Asn Ala Lys Asn Thr
65               70               75               80


Leu Tyr Leu Gln Met Asn Asn Leu Lys His Glu Asp Thr Ala Glu Tyr
                85               90               95


Leu Cys Tyr Thr Val Gly Pro Glu Gly Asp Tyr Trp Gly Gln Gly Thr
                100               105               110


Gln Val Thr Val Ser Ser Thr Ser

115                    120

<210>   66
<211>   9
<212>   PRT
<213>   artificial sequence

<220>
<223>   Re6D09 CDR1

<400>   66

Arg Thr Phe Asn Asn Tyr Asn Met Val
1                   5


<210>   67
<211>   15
<212>   PRT
<213>   artificial sequence

<220>
<223>   Re6D09 CDR2

<400>   67

Phe Val Ala Ala Ile Asn Trp Asn Gly Gly Ser Thr Ser Tyr Ala
1                   5                   10                  15


<210>   68
<211>   10
<212>   PRT
<213>   artificial sequence

<220>
<223>   Re6D09 CDR3

<400>   68

Tyr Thr Val Gly Pro Glu Gly Asp Tyr Trp
1                   5                   10


<210>   69
<211>   134
<212>   PRT
<213>   artificial sequence

<220>
<223>   Re6E11

<400>   69

Gly Ser Gln Val Gln Leu Val Glu Ser Gly Gly Gly Leu Val Gln Pro
1                   5                   10                  15


Gly Gly Ser Leu Arg Leu Ser Cys Ala Ala Ser Gly Leu Thr Leu Asp
                20                  25                  30

```
Tyr Tyr Ala Ile Gly Trp Phe Arg Gln Ala Pro Gly Lys Glu Arg Glu
        35              40              45

Gly Val Ser Cys Ile Ser Ser Arg Asp Gly Ser Thr Met Tyr Ala Asp
        50              55              60

Ser Val Lys Gly Arg Phe Thr Ile Ser Arg Asp Asn Ala Lys Asn Thr
65              70              75              80

Val Tyr Leu Gln Met Asn Ser Leu Lys Pro Glu Asp Thr Ala Val Tyr
                85              90              95

Tyr Cys Ala Ala Thr Pro Thr Thr Tyr Tyr Ser Gly Ser Tyr Tyr Tyr
            100             105             110

Thr Cys Ser Pro Glu Gly Tyr Asp Tyr Trp Gly Gln Gly Thr Gln Val
            115             120             125

Thr Val Ser Ser Thr Ser
        130
```

<210> 70
<211> 9
<212> PRT
<213> artificial sequence

<220>
<223> Re6E11 CDR1

<400> 70

```
Leu Thr Leu Asp Tyr Tyr Ala Ile Gly
1               5
```

<210> 71
<211> 15
<212> PRT
<213> artificial sequence

<220>
<223> Re6E11 CDR2

<400> 71

```
Gly Val Ser Cys Ile Ser Ser Arg Asp Gly Ser Thr Met Tyr Ala
1               5               10              15
```

<210> 72
<211> 24
<212> PRT
<213> artificial sequence

<220>
<223> Re6E11 CDR3

<400>  72

Ala Ala Thr Pro Thr Thr Tyr Tyr Ser Gly Ser Tyr Tyr Tyr Thr Cys
1               5                   10                  15

Ser Pro Glu Gly Tyr Asp Tyr Trp
            20

<210>  73
<211>  119
<212>  PRT
<213>  artificial sequence

<220>
<223>  Re6F06

<400>  73

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Phe Thr Phe Ser
            20                  25                  30

Asn Tyr Ala Met Gly Trp Tyr Arg Gln Ala Pro Gly Lys Glu Cys Glu
            35                  40                  45

Phe Val Ala Val Ile Thr Ile Thr Gly Ser Asn Thr Asn Tyr Ala Asp
        50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Asn Pro Asp Pro Gly Cys Glu Ser Gln Gly Gln Gly Thr Arg
            100                 105                 110

Val Thr Val Ser Ser Thr Ser
            115

<210>  74
<211>  9
<212>  PRT
<213>  artificial sequence

<220>
<223>  Re6F06 CDR1

<400>  74

Phe Thr Phe Ser Asn Tyr Ala Met Gly
1               5

<210> 75
<211> 15
<212> PRT
<213> artificial sequence

<220>
<223> Re6F06 CDR2

<400> 75

Phe Val Ala Val Ile Thr Ile Thr Gly Ser Asn Thr Asn Tyr Ala
1               5                   10                  15

<210> 76
<211> 9
<212> PRT
<213> artificial sequence

<220>
<223> Re6F06 CDR3

<400> 76

Asn Pro Asp Pro Gly Cys Glu Ser Gln
1               5

<210> 77
<211> 130
<212> PRT
<213> artificial sequence

<220>
<223> Re6G03

<400> 77

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
                20                  25                  30

Thr Tyr Arg Met Ala Trp Phe Arg Leu Ala Pro Gly Lys Glu Arg Glu
                35                  40                  45

Phe Val Ala Gly Ile Asn Trp Ser Asp Gly Thr Thr Ser Tyr Lys Asp
                50                  55                  60

Ser Val Lys Gly Arg Phe Thr Ile Ser Arg Asp Asn Ala Lys Asn Thr
65                  70                  75                  80

Val Tyr Leu Gln Met Asp Ser Leu Lys Pro Glu Asp Thr Ala Val Tyr

                      85                    90                    95


        Tyr Cys Asn Ala His Leu Ser Thr Gly Gln Glu Gly Pro Gly Glu Tyr
                    100                 105                 110


        Phe Gly Met Asp Tyr Trp Gly Lys Gly Thr Gln Val Thr Val Ser Ser
                    115                 120                 125


        Thr Ser
            130


        <210> 78
        <211> 9
        <212> PRT
        <213> artificial sequence

        <220>
        <223> Re6G03 CDR1

        <400> 78

        Arg Thr Phe Ser Thr Tyr Arg Met Ala
        1               5


        <210> 79
        <211> 15
        <212> PRT
        <213> artificial sequence

        <220>
        <223> Re6G03 CDR2

        <400> 79

        Phe Val Ala Gly Ile Asn Trp Ser Asp Gly Thr Thr Ser Tyr Lys
        1               5                   10                  15


        <210> 80
        <211> 20
        <212> PRT
        <213> artificial sequence

        <220>
        <223> Re6G03 CDR3

        <400> 80

        Asn Ala His Leu Ser Thr Gly Gln Glu Gly Pro Gly Glu Tyr Phe Gly
        1               5                   10                  15


        Met Asp Tyr Trp
                    20


        <210> 81
        <211> 134

57

```
<212>    PRT
<213>    artificial sequence

<220>
<223>    Re6H06

<400>    81

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Val Thr Leu Asp
            20                  25                  30


Tyr Tyr Ala Ile Gly Trp Phe Arg Gln Ala Pro Gly Lys Glu Arg Glu
        35                  40                  45


Gly Val Ser Cys Thr Ser Ser Ser Asp Gly Ser Thr Tyr Tyr Ala Asp
        50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Gly Val Tyr
                85                  90                  95


Tyr Cys Ala Val Val Pro Gln Thr Tyr Tyr Gly Gly Lys Tyr Tyr Ser
            100                 105                 110


Gln Cys Thr Ala Asn Gly Met Asp Tyr Trp Gly Lys Gly Thr Leu Val
            115                 120                 125


Thr Val Ser Ser Thr Ser
        130


<210>    82
<211>    9
<212>    PRT
<213>    artificial sequence

<220>
<223>    Re6H06 CDR1

<400>    82

Val Thr Leu Asp Tyr Tyr Ala Ile Gly
1               5


<210>    83
<211>    15
<212>    PRT
<213>    artificial sequence
```

<220>
<223>   Re6H06 CDR2

<400>   83

Gly Val Ser Cys Thr Ser Ser Ser Asp Gly Ser Thr Tyr Tyr Ala
1                   5                   10                  15


<210>   84
<211>   24
<212>   PRT
<213>   artificial sequence

<220>
<223>   Re6H06 CDR3

<400>   84

Ala Val Val Pro Gln Thr Tyr Tyr Gly Gly Lys Tyr Tyr Ser Gln Cys
1                   5                   10                  15


Thr Ala Asn Gly Met Asp Tyr Trp
                20


<210>   85
<211>   119
<212>   PRT
<213>   artificial sequence

<220>
<223>   Re6H10

<400>   85

Gly Ser Gln Val Gln Leu Val Glu Ser Gly Gly Gly Leu Val Gln Pro
1                   5                   10                  15


Gly Gly Ser Leu Arg Leu Ser Cys Ala Ala Ser Gly Phe Thr Phe Ser
                20                  25                  30


Ser Tyr Ala Met Gly Trp Tyr Arg Gln Ala Pro Gly Lys Glu Cys Glu
                35                  40                  45


Phe Val Ala Val Ile Thr Ile Thr Gly Gly Ser Thr Asn Tyr Ala Asp
                50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95


Tyr Cys Asn Pro Asp Pro Gly Cys Arg Gly Gly Gly Gln Gly Thr Leu
                100                 105                 110

Val Thr Val Ser Ser Thr Ser
              115

<210>  86
<211>  9
<212>  PRT
<213>  artificial sequence

<220>
<223>  Re6H10 CDR1

<400>  86

Phe Thr Phe Ser Ser Tyr Ala Met Gly
1               5

<210>  87
<211>  15
<212>  PRT
<213>  artificial sequence

<220>
<223>  Re6H10 CDR2

<400>  87

Phe Val Ala Val Ile Thr Ile Thr Gly Gly Ser Thr Asn Tyr Ala
1               5                   10                  15

<210>  88
<211>  9
<212>  PRT
<213>  artificial sequence

<220>
<223>  Re6H10 CDR3

<400>  88

Asn Pro Asp Pro Gly Cys Arg Gly Gly
1               5

<210>  89
<211>  125
<212>  PRT
<213>  artificial sequence

<220>
<223>  Re7A01

<400>  89

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser

60

```
                   20                        25                        30


      Ser Tyr Ala Met Gly Trp Phe Arg Gln Ala Pro Gly Lys Glu Arg Glu
              35                      40                  45


      Phe Val Ala Thr Ile Ser Phe Ser Gly Ser Thr Ser Tyr Ala Gly His
              50                      55                  60


      Val Lys Gly Arg Phe Thr Ile Ser Arg Asp Asn Ala Lys Asn Thr Val
      65                      70                  75                  80


      Tyr Leu Gln Met Asn Ser Leu Lys Pro Glu Asp Thr Ala Val Tyr Tyr
                      85                  90                  95


      Cys His Ala Val Thr Arg Ala Ser Asp Gln Asp Gly Gly Met Asp Tyr
                  100                     105                 110


      Trp Gly Gln Gly Thr Gln Val Thr Val Ser Ser Thr Ser
              115                     120                 125


      <210>  90
      <211>  9
      <212>  PRT
      <213>  artificial sequence

      <220>
      <223>  Re7A01 CDR1

      <400>  90

      Arg Thr Phe Ser Ser Tyr Ala Met Gly
      1               5


      <210>  91
      <211>  14
      <212>  PRT
      <213>  artificial seq

      <220>
      <223>  Re7A01 CDR2

      <400>  91

      Phe Val Ala Thr Ile Ser Phe Ser Gly Ser Thr Ser Tyr Ala
      1               5                   10


      <210>  92
      <211>  16
      <212>  PRT
      <213>  artificial sequence

      <220>
      <223>  Re7A01 CDR3
```

<400> 92

His Ala Val Thr Arg Ala Ser Asp Gln Asp Gly Gly Met Asp Tyr Trp
1                   5                   10                  15


<210> 93
<211> 131
<212> PRT
<213> artificial sequence

<220>
<223> Re7B01

<400> 93

Gly Ser Gln Val Gln Leu Val Glu Ser Gly Gly Gly Leu Val Gln Pro
1                   5                   10                  15


Gly Gly Ser Leu Arg Leu Ser Cys Gly Ala Ser Gly Phe Thr Leu Asp
                20                  25                  30


Tyr Tyr Ala Ile Gly Trp Phe Arg Gln Ala Pro Gly Lys Glu Arg Glu
            35                  40                  45


Gly Val Ser Arg Ile Arg Ser Asn Asp Gly Ser Thr Asp Tyr Ala Asp
        50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
                85                  90                  95


Tyr Cys Ala Tyr Gly Pro Leu Thr Lys Tyr Gly Ser Ser Trp Tyr Trp
            100                 105                 110


Pro Tyr Glu Tyr Asp Tyr Trp Gly Gln Gly Thr Gln Val Thr Val Ser
            115                 120                 125


Ser Thr Ser
        130


<210> 94
<211> 9
<212> PRT
<213> artificial sequence

<220>
<223> Re7B01 CDR1

<400> 94

Phe Thr Leu Asp Tyr Tyr Ala Ile Gly

<210> 95
<211> 15
<212> PRT
<213> artificial sequence

<220>
<223> Re7B01 CDR2

<400> 95

Gly Val Ser Arg Ile Arg Ser Asn Asp Gly Ser Thr Asp Tyr Ala
1               5                   10                  15


<210> 96
<211> 21
<212> PRT
<213> artificial sequence

<220>
<223> Re7B01 CDR3

<400> 96

Ala Tyr Gly Pro Leu Thr Lys Tyr Gly Ser Ser Trp Tyr Trp Pro Tyr
1               5                   10                  15


Glu Tyr Asp Tyr Trp
                20


<210> 97
<211> 126
<212> PRT
<213> artificial sequence

<220>
<223> Re7D05

<400> 97

Gly Ser Gln Val Gln Leu Val Glu Ser Gly Gly Gly Leu Val Gln Ala
1               5                   10                  15


Gly Gly Ser Leu Arg Leu Ser Cys Ala Ala Ser Gly Arg Thr Phe Ser
                20                  25                  30


Ser Tyr Ala Met Gly Trp Phe Arg Gln Ala Pro Gly Lys Glu Arg Glu
                35                  40                  45


Phe Val Ala Thr Ile Ser Trp Ser Gly Gly Ser Thr Ser Tyr Ala Asp
                50                  55                  60


Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Asn Ala Val Thr His His Ser Asp Gln Asp Gly Gly Met Asp
            100                 105                 110

Tyr Trp Gly Lys Gly Thr Leu Val Thr Val Ser Ser Thr Ser
            115                 120                 125

```
<210>  98
<211>  9
<212>  PRT
<213>  artificial sequence

<220>
<223>  Re7D05 CDR1

<400>  98
```

Arg Thr Phe Ser Ser Tyr Ala Met Gly
1                   5

```
<210>  99
<211>  15
<212>  PRT
<213>  artificial sequence

<220>
<223>  Re7D05 CDR2

<400>  99
```

Phe Val Ala Thr Ile Ser Trp Ser Gly Gly Ser Thr Ser Tyr Ala
1                   5                   10                  15

```
<210>  100
<211>  16
<212>  PRT
<213>  artificial sequence

<220>
<223>  Re7D05 CDR3

<400>  100
```

Asn Ala Val Thr His His Ser Asp Gln Asp Gly Gly Met Asp Tyr Trp
1                   5                   10                  15

```
<210>  101
<211>  131
<212>  PRT
<213>  artificial sequence

<220>
<223>  Re7E02
```

<400> 101

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Phe Thr Leu Asp
            20                  25                  30

Tyr Tyr Ala Ile Gly Trp Phe Arg Gln Ala Pro Gly Lys Glu Arg Glu
            35                  40                  45

Gly Val Ser Tyr Ile Arg Ser Ser Asp Gly Thr Thr Tyr Tyr Ala Asp
            50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Glu Ala Tyr Tyr Ser Glu Leu Gly Trp Glu Ser
            100                 105                 110

Pro Trp Gly Trp Ser Tyr Trp Gly Gln Gly Thr Gln Val Thr Val Ser
            115                 120                 125

Ser Thr Ser
        130

<210> 102
<211> 9
<212> PRT
<213> artificial sequence

<220>
<223> Re7E02 CDR1

<400> 102

Phe Thr Leu Asp Tyr Tyr Ala Ile Gly
1               5

<210> 103
<211> 15
<212> PRT
<213> artificial sequence

<220>
<223> Re7E02 CDR2

<400> 103

Gly Val Ser Tyr Ile Arg Ser Ser Asp Gly Thr Thr Tyr Tyr Ala
1              5              10             15

<210>  104
<211>  21
<212>  PRT
<213>  artificial sequence

<220>
<223>  Re7E02 CDR3

<400>  104

Ala Ala Asp Glu Ala Tyr Tyr Ser Glu Leu Gly Trp Glu Ser Pro Trp
1              5              10             15

Gly Trp Ser Tyr Trp
            20

<210>  105
<211>  116
<212>  PRT
<213>  artificial sequence

<220>
<223>  Re7H02

<400>  105

Gly Ser Gln Val Gln Leu Val Glu Ser Gly Gly Gly Leu Val Gln Ala
1              5              10             15

Gly Gly Ser Leu Arg Leu Ser Cys Ala Ala Ser Gly Arg Ala Phe Glu
            20             25             30

Ser Ala Pro Met Ser Trp Phe Arg Gln Ala Pro Gly Lys Glu Arg Glu
            35             40             45

Phe Val Ala Ser Val Ser Trp Ser Gly Asp Ser Thr Asn Tyr Ala Asp
            50             55             60

Ser Val Lys Gly Arg Phe Thr Ile Ser Arg Asp Asn Ala Glu Asn Thr
65             70             75             80

Gly Tyr Leu Gln Met Asn Ser Leu Lys Pro Glu Asp Thr Ala Val Tyr
            85             90             95

Tyr Cys Lys Arg Gly Pro Tyr Trp Gly Gln Gly Thr Gln Val Thr Val
            100            105            110

Ser Ser Thr Ser
            115

```
<210>   106
<211>   9
<212>   PRT
<213>   artificial sequence

<220>
<223>   Re7H02 CDR1

<400>   106

Arg Ala Phe Glu Ser Ala Pro Met Ser
1               5


<210>   107
<211>   15
<212>   PRT
<213>   artificial sequence

<220>
<223>   Re7H02 CDR2

<400>   107

Phe Val Ala Ser Val Ser Trp Ser Gly Asp Ser Thr Asn Tyr Ala
1               5                   10                  15


<210>   108
<211>   6
<212>   PRT
<213>   artificial sequence

<220>
<223>   Re7H02 CDR3

<400>   108

Lys Arg Gly Pro Tyr Trp
1               5


<210>   109
<211>   121
<212>   PRT
<213>   artificial sequence

<220>
<223>   Re8A03

<400>   109

Gly Ser Gln Val Gln Leu Val Glu Ser Gly Gly Gly Leu Val Gln Pro
1               5                   10                  15

Gly Gly Ser Leu Arg Leu Ser Cys Ala Ala Ser Gly Arg Ile Thr Gly
                20                  25                  30

Phe Asn Gly Met Gly Trp Tyr Arg Gln Thr Pro Gly Lys Gln Arg Glu
                35                  40                  45
```

67

```
Leu Val Ala Ser Ile Thr Asn Gly Gly Ile Thr Lys Tyr Ala Asp Ser
    50                  55              60

Val Lys Gly Arg Phe Thr Ile Ser Arg Asp Asn Ala Lys Asn Thr Val
65              70              75                  80

Tyr Leu Gln Met Asn Ser Leu Lys Pro Glu Asp Thr Ala Val Tyr Leu
                85              90                  95

Cys Tyr Phe Trp Arg Pro Glu Phe Pro Asn Leu Tyr Trp Gly Gln Gly
                100             105             110

Thr Gln Val Thr Val Ser Ser Thr Ser
        115             120
```

```
<210>  110
<211>  9
<212>  PRT
<213>  artificial sequence

<220>
<223>  Re8A03 CDR1

<400>  110
```

```
Arg Ile Thr Gly Phe Asn Gly Met Gly
1               5
```

```
<210>  111
<211>  14
<212>  PRT
<213>  artificial sequence

<220>
<223>  Re8A03 CDR2

<400>  111
```

```
Leu Val Ala Ser Ile Thr Asn Gly Gly Ile Thr Lys Tyr Ala
1               5                   10
```

```
<210>  112
<211>  12
<212>  PRT
<213>  artificial sequence

<220>
<223>  Re8A03 CDR3

<400>  112
```

```
Tyr Phe Trp Arg Pro Glu Phe Pro Asn Leu Tyr Trp
1               5                   10
```

<210> 113
<211> 123
<212> PRT
<213> artificial sequence

<220>
<223> Re8A06

<400> 113

Gly Ser Gln Val Gln Leu Val Glu Ser Gly Gly Gly Leu Val Gln Ala
1               5               10              15

Gly Gly Ser Leu Arg Leu Ser Cys Ala Ala Ser Gly Ser Ile Phe Ser
            20              25              30

Ile Asn Ala Met Gly Trp Tyr Arg Gln Ala Pro Gly Lys Glu Arg Glu
            35              40              45

Leu Val Ala Ala Met Gly Ser Ser Gly Trp Ile Asn Tyr Ala Asp Ser
        50              55              60

Val Lys Gly Arg Leu Thr Ile Ser Arg Asp Asn Ala Lys Asn Thr Leu
65              70              75              80

Tyr Leu Gln Met Asn Ser Leu Lys Pro Glu Asp Thr Ala Val Tyr Tyr
                85              90              95

Cys Arg Gly Thr Gly Gly Val Gly Pro Thr Ser Ala Asp Tyr Trp Gly
            100             105             110

Gln Gly Thr Gln Val Thr Val Ser Ser Thr Ser
            115             120

<210> 114
<211> 9
<212> PRT
<213> artificial sequence

<220>
<223> Re8A06 CDR1

<400> 114

Ser Ile Phe Ser Ile Asn Ala Met Gly
1               5

<210> 115
<211> 14
<212> PRT
<213> artificial sequence

<220>

<223> Re8A06 CDR2

<400> 115

Leu Val Ala Ala Met Gly Ser Ser Gly Trp Ile Asn Tyr Ala
1               5                   10

<210> 116
<211> 14
<212> PRT
<213> artificial sequence

<220>
<223> Re8A06 CDR3

<400> 116

Arg Gly Thr Gly Gly Val Gly Pro Thr Ser Ala Asp Tyr Trp
1               5                   10

<210> 117
<211> 128
<212> PRT
<213> artificial sequence

<220>
<223> Re8C06

<400> 117

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Asp Thr
                20                  25                  30

Ile Tyr Asn Met Gly Trp Phe Arg Gln Ala Pro Gly Lys Glu Arg Glu
                35                  40                  45

Phe Val Ala Ala Ile Ser Trp Ser Asp Gly Lys Thr Thr Phe Ala Asp
        50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Asn Tyr
                85                  90                  95

Tyr Cys Ala Ala Lys Ala Phe Leu Val Ala Gly Arg Ser Leu Glu Glu
                100                 105                 110

Tyr Asp Tyr Ser Gly Gln Gly Thr Gln Val Thr Val Ser Ser Thr Ser
                115                 120                 125

```
<210>  118
<211>  9
<212>  PRT
<213>  artificial sequence

<220>
<223>  Re8C06 CDR1

<400>  118

Arg Thr Asp Thr Ile Tyr Asn Met Gly
1               5


<210>  119
<211>  15
<212>  PRT
<213>  artificial sequence

<220>
<223>  Re8C06 CDR2

<400>  119

Phe Val Ala Ala Ile Ser Trp Ser Asp Gly Lys Thr Thr Phe Ala
1               5                   10                  15


<210>  120
<211>  18
<212>  PRT
<213>  artificial sequence

<220>
<223>  Re8C06 CDR3

<400>  120

Ala Ala Lys Ala Phe Leu Val Ala Gly Arg Ser Leu Glu Glu Tyr Asp
1               5                   10                  15

Tyr Ser


<210>  121
<211>  123
<212>  PRT
<213>  artificial sequence

<220>
<223>  Re8E12

<400>  121

Gly Ser Gln Val Gln Leu Val Glu Ser Gly Gly Gly Ser Val Gln Pro
1               5                   10                  15

Gly Gly Ser Leu Arg Leu Ser Cys Lys Val Ser Gly Phe Thr Ser Asp
            20                  25                  30
```

```
Val Asp Leu Arg Asn Tyr Leu Val Ser Trp Asn Arg Gln Ala Pro Gly
        35              40              45

Lys Glu Arg Glu Leu Val Ala Ala Ile Thr Pro Thr Val Ile Ser Gly
        50              55              60

Gly Asn Thr Asn Tyr Ala Asp Ser Val Lys Gly Arg Phe Thr Ile Ser
65              70              75              80

Arg Asp Tyr Ser Lys Ser Thr Val Tyr Leu Gln Met Asn Ser Leu Asn
            85              90              95

Pro Glu Asp Thr Ala Val Tyr Tyr Cys Lys Val Gly Val Tyr Trp Gly
            100             105             110

Gln Gly Thr Gln Val Thr Val Ser Ser Thr Ser
        115             120
```

```
<210>   122
<211>   13
<212>   PRT
<213>   artificial sequence

<220>
<223>   Re8E12 CDR1

<400>   122

Phe Thr Ser Asp Val Asp Leu Arg Asn Tyr Leu Val Ser
1               5               10
```

```
<210>   123
<211>   18
<212>   PRT
<213>   artificial sequence

<220>
<223>   Re8E12 CDR2

<400>   123

Leu Val Ala Ala Ile Thr Pro Thr Val Ile Ser Gly Gly Asn Thr Asn
1               5               10              15

Tyr Ala
```

```
<210>   124
<211>   6
<212>   PRT
<213>   artificial sequence
```

<220>
<223>   Re8E12 CDR3

<400>   124

Lys Val Gly Val Tyr Trp
1               5


<210>   125
<211>   123
<212>   PRT
<213>   artificial sequence

<220>
<223>   Re8F03

<400>   125

Gly Ser Gln Val Gln Leu Val Glu Ser Gly Gly Gly Leu Val Gln Pro
1               5               10              15


Gly Gly Ser Leu Thr Leu Ser Cys Lys Val Ser Gly Leu Thr Ser Tyr
        20              25              30


Val Asp Leu Arg Asn Tyr Leu Val Ser Trp Tyr Arg Gln Gly Pro Gly
        35              40              45


Lys Glu Arg Glu Leu Val Ala Ala Ile Thr Pro Thr Ala Ile Thr Gly
        50              55              60


Gly Ser Thr Asn Tyr Ala Asp Ser Val Lys Gly Arg Phe Thr Ile Ser
65              70              75              80


Arg Asp Tyr Ser Lys Ser Thr Val Tyr Leu Gln Met Asn Ser Leu Asn
                85              90              95


Pro Glu Asp Thr Ala Val Tyr Ser Cys Lys Val Gly Val Tyr Trp Gly
        100             105             110


Gln Gly Thr Gln Val Thr Val Ser Ser Thr Ser
        115             120


<210>   126
<211>   13
<212>   PRT
<213>   artificial sequence

<220>
<223>   Re8F03 CDR1

<400>   126

Leu Thr Ser Tyr Val Asp Leu Arg Asn Tyr Leu Val Ser
1               5               10

<210> 127
<211> 18
<212> PRT
<213> artificial sequence

<220>
<223> Re8F03 CDR2

<400> 127

Leu Val Ala Ala Ile Thr Pro Thr Ala Ile Thr Gly Gly Ser Thr Asn
1                5                    10                  15

Tyr Ala


<210> 128
<211> 6
<212> PRT
<213> artificial sequence

<220>
<223> Re8F03 CDR3

<400> 128

Lys Val Gly Val Tyr Trp
1                5


<210> 129
<211> 134
<212> PRT
<213> artificial sequence

<220>
<223> Re9B09

<400> 129

Gly Ser Gln Val Gln Leu Val Glu Ser Gly Gly Gly Leu Val Gln Pro
1                5                    10                  15

Gly Gly Ser Leu Arg Leu Ser Cys Ala Ala Ser Gly Phe Thr Leu Asp
                20                   25                  30

Tyr Tyr Ala Ile Gly Trp Phe Arg Gln Ala Pro Gly Lys Glu Arg Glu
                35                   40                  45

Gly Val Ser Arg Ile Ser Ser Ser Asp Gly Ser Thr Asp Tyr Ala Asp
        50                   55                  60

Ser Val Lys Gly Arg Phe Thr Ile Ser Arg Asp Asn Ala Lys Asn Thr
65                   70                   75                  80

```
Val Tyr Leu Gln Met Asn Ser Leu Lys Pro Glu Asp Thr Ala Val Tyr
                85                  90                  95

Tyr Cys Ala Thr Val Pro Gly Thr Tyr Tyr Ser Gly Asn Trp Tyr Tyr
            100                 105                 110

Thr Trp His Pro Glu Ala Val Asp Tyr Trp Gly Lys Gly Thr Gln Val
            115                 120                 125

Thr Val Ser Ser Thr Ser
    130
```

```
<210> 130
<211> 9
<212> PRT
<213> artificial sequence

<220>
<223> Re9B09 CDR1

<400> 130
```

```
Phe Thr Leu Asp Tyr Tyr Ala Ile Gly
1               5
```

```
<210> 131
<211> 15
<212> PRT
<213> artificial sequence

<220>
<223> Re9B09 CDR2

<400> 131
```

```
Gly Val Ser Arg Ile Ser Ser Ser Asp Gly Ser Thr Asp Tyr Ala
1               5                   10                  15
```

```
<210> 132
<211> 24
<212> PRT
<213> artificial sequence

<220>
<223> Re9B09 CDR3

<400> 132
```

```
Ala Thr Val Pro Gly Thr Tyr Tyr Ser Gly Asn Trp Tyr Tyr Thr Trp
1               5                   10                  15

His Pro Glu Ala Val Asp Tyr Trp
                20
```

<210> 133
<211> 123
<212> PRT
<213> artificial sequence

<220>
<223> Re9B10

<400> 133

Gly Ser Gln Val Gln Leu Val Glu Ser Gly Gly Gly Leu Val Gln Pro
1               5                   10                  15

Gly Gly Ser Leu Arg Leu Ser Cys Ala Ala Ser Gly Arg Met Phe Gly
            20                  25                  30

Val Tyr Arg Met Gly Trp Phe Arg Gln Ala Pro Gly Lys Glu Arg Glu
        35                  40                  45

Phe Val Ala Gly Ile Ser Thr Ser Val Gly Thr Thr Asn Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Arg Asp Pro Thr Thr Tyr Glu Tyr Asp Tyr Trp Gly
            100                 105                 110

Gln Gly Thr Gln Val Thr Val Ser Ser Thr Ser
        115                 120

<210> 134
<211> 9
<212> PRT
<213> artificial sequence

<220>
<223> Re9B10 CDR1

<400> 134

Arg Met Phe Gly Val Tyr Arg Met Gly
1               5

<210> 135
<211> 15
<212> PRT
<213> artificial sequence

<220>
<223> Re9B10 CDR2

<400> 135

Phe Val Ala Gly Ile Ser Thr Ser Val Gly Thr Thr Asn Tyr Ala
1               5                   10                  15


<210> 136
<211> 13
<212> PRT
<213> artificial sequence

<220>
<223> Re9B10 CDR3

<400> 136

Ala Ala Arg Asp Pro Thr Thr Tyr Glu Tyr Asp Tyr Trp
1               5                   10


<210> 137
<211> 131
<212> PRT
<213> artificial sequence

<220>
<223> Re9C07

<400> 137

Gly Ser Gln Val Gln Leu Val Glu Ser Gly Gly Gly Leu Val Gln Ala
1               5                   10                  15


Gly Gly Ser Leu Arg Leu Ser Cys Ala Ala Ser Gly Arg Thr Phe Ser
            20                  25                  30


Arg Tyr Ala Met Gly Trp Phe Arg Gln Ala Pro Gly Lys Glu Arg Glu
        35                  40                  45


Phe Val Ala Ala Ile Thr Trp Asn Ala Asp Thr Thr Tyr Tyr Ala Asp
    50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
            85                  90                  95


Tyr Cys Ala Ala Gly Gly Asn His Tyr Tyr Ser Arg Ser Tyr Tyr Ser
            100                 105                 110


Ser Leu Glu Tyr Asp His Trp Gly Gln Gly Thr Gln Val Thr Val Ser
            115                 120                 125

Ser Thr Ser
130

<210> 138
<211> 9
<212> PRT
<213> artificial sequence

<220>
<223> Re9C07 CDR1

<400> 138

Arg Thr Phe Ser Arg Tyr Ala Met Gly
1               5

<210> 139
<211> 15
<212> PRT
<213> artificial sequence

<220>
<223> Re9C07 CDR2

<400> 139

Phe Val Ala Ala Ile Thr Trp Asn Ala Asp Thr Thr Tyr Tyr Ala
1               5                   10                  15

<210> 140
<211> 21
<212> PRT
<213> artificial sequence

<220>
<223> Re9C07 CDR3

<400> 140

Ala Ala Gly Gly Asn His Tyr Tyr Ser Arg Ser Tyr Tyr Ser Ser Leu
1               5                   10                  15

Glu Tyr Asp His Trp
                20

<210> 141
<211> 121
<212> PRT
<213> artificial sequence

<220>
<223> Re9C08

<400> 141

Gly Ser Gln Val Gln Leu Val Glu Ser Gly Gly Gly Leu Val Gln Pro
1               5                   10                  15

```
Gly Gly Ser Leu Arg Leu Ser Cys Ala Val Ser Gly Asn Ile Phe Gly
            20                  25                  30

Ile Thr Ala Trp Asp Trp His Arg Gln Ala Pro Gly Lys Gln Arg Glu
            35                  40                  45

Leu Val Ala His Ile Thr Ser Arg Gly Asp Thr Tyr Tyr Leu Asp Ser
            50                  55                  60

Val Lys Gly Arg Phe Ala Ile Ser Arg Asp His Ala Lys Asn Thr Leu
65                  70                  75                  80

Ser Leu Gln Met Asn Ser Leu Lys Pro Glu Asp Thr Ala Val Tyr Tyr
                85                  90                  95

Cys Tyr Leu Arg Thr Phe Gly Pro Pro Asn Asp His Trp Gly Gln Gly
            100                 105                 110

Thr Gln Val Thr Val Ser Ser Thr Ser
        115                 120


<210>   142
<211>   9
<212>   PRT
<213>   artificial sequence

<220>
<223>   Re9C08 CDR1

<400>   142

Asn Ile Phe Gly Ile Thr Ala Trp Asp
1               5


<210>   143
<211>   14
<212>   PRT
<213>   artificial sequence

<220>
<223>   Re9C08 CDR2

<400>   143

Leu Val Ala His Ile Thr Ser Arg Gly Asp Thr Tyr Tyr Leu
1               5                   10


<210>   144
<211>   12
<212>   PRT
<213>   artificial sequence

<220>
```

<223> Re9C08 CDR3

<400> 144

Tyr Leu Arg Thr Phe Gly Pro Pro Asn Asp His Trp
1               5                   10


<210> 145
<211> 129
<212> PRT
<213> artificial sequence

<220>
<223> Re9D02

<400> 145

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Asn Tyr Ala Met Gly Trp Phe Arg Gln Ala Pro Gly Lys Glu Arg Glu
            35                  40                  45

Phe Val Ala Ala Ile Ser Trp Gly Gly Asp Thr Thr Tyr Tyr Ala Asp
            50                  55                  60

Ser Leu Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Arg Gly Leu Ser Tyr Tyr Tyr Asp Arg Val Thr
            100                 105                 110

Glu Tyr Asp Tyr Trp Gly Gln Gly Thr Gln Val Thr Val Ser Ser Thr
            115                 120                 125

Ser


<210> 146
<211> 9
<212> PRT
<213> artificial sequence

<220>
<223> Re9D02 CDR1

<400> 146

Arg Thr Phe Ser Asn Tyr Ala Met Gly
1               5


<210> 147
<211> 15
<212> PRT
<213> artificial sequence

<220>
<223> Re9D02 CDR2

<400> 147

Phe Val Ala Ala Ile Ser Trp Gly Gly Asp Thr Thr Tyr Tyr Ala
1               5               10              15


<210> 148
<211> 19
<212> PRT
<213> artificial sequence

<220>
<223> Re9D02 CDR3

<400> 148

Ala Ala Asp Arg Gly Leu Ser Tyr Tyr Asp Arg Val Thr Glu Tyr
1               5               10              15


Asp Tyr Trp


<210> 149
<211> 121
<212> PRT
<213> artificial sequence

<220>
<223> Re9G05

<400> 149

Gly Ser Gln Val Gln Leu Val Glu Ser Gly Gly Gly Leu Val Gln Pro
1               5               10              15


Gly Gly Ser Leu Arg Leu Ser Cys Ala Val Ser Gly Asn Ile Ser Ser
            20              25              30


Ile Thr Ala Trp Asp Trp His Arg Gln Ala Pro Gly Lys Gln Arg Glu
            35              40              45


Leu Val Ala His Ile Thr Ser Arg Gly Asp Thr Met Tyr Leu Asp Ser
            50              55              60

Val Lys Gly Arg Phe Ala Ile Ser Arg Asp His Ala Lys Asn Thr Leu
65                  70              75                  80


Ser Leu Gln Met Asn Ser Leu Lys Pro Glu Asp Thr Ala Val Tyr Tyr
                85              90                  95


Cys Tyr Leu Arg Thr Phe Gly Pro Pro Tyr Asp Tyr Trp Gly Gln Gly
                100             105             110


Thr Gln Val Thr Val Ser Ser Thr Ser
        115             120


<210>   150
<211>   9
<212>   PRT
<213>   artificial sequence

<220>
<223>   Re9G05 CDR1

<400>   150

Asn Ile Ser Ser Ile Thr Ala Trp Asp
1                   5


<210>   151
<211>   14
<212>   PRT
<213>   artificial sequence

<220>
<223>   Re9G05 CDR2

<400>   151

Leu Val Ala His Ile Thr Ser Arg Gly Asp Thr Met Tyr Leu
1                   5                   10


<210>   152
<211>   12
<212>   PRT
<213>   artificial sequence

<220>
<223>   Re9G05 CDR3

<400>   152

Tyr Leu Arg Thr Phe Gly Pro Pro Tyr Asp Tyr Trp
1                   5                   10


<210>   153
<211>   129
<212>   PRT
<213>   artificial sequence

<220>
<223>  Re9G12

<400>  153

Gly Ser Gln Val Gln Leu Val Glu Ser Gly Gly Gly Leu Val Gln Ala
1                   5                   10                  15


Gly Gly Ser Leu Arg Leu Ser Cys Ala Ala Ser Gly Arg Thr Phe Ser
            20                  25                  30


Ser Tyr Val Met Gly Trp Phe Arg Gln Ala Pro Gly Lys Glu Arg Glu
            35                  40                  45


Phe Val Ala His Ile Ser Trp Ser Gly Asp Ser Thr Tyr Tyr Ala Asp
        50                  55                  60


Ser Val Lys Gly Arg Phe Thr Ile Phe Arg Asp Asn Ala Lys Asn Thr
65                  70                  75                  80


Ala Tyr Leu Gln Met Asn Ser Leu Lys Pro Glu Asp Thr Ala Val Tyr
                85                  90                  95


Tyr Cys Ala Ala Asp Arg Gly Ala Ser Tyr Tyr Tyr Thr Trp Ala Ser
            100                 105                 110


Glu Tyr Asn Tyr Trp Gly Gln Gly Thr Gln Val Thr Val Ser Ser Thr
            115                 120                 125


Ser


<210>  154
<211>  9
<212>  PRT
<213>  artificial sequence

<220>
<223>  Re9G12 CDR1

<400>  154

Arg Thr Phe Ser Ser Tyr Val Met Gly
1                   5


<210>  155
<211>  15
<212>  PRT
<213>  artificial sequence

<220>
<223>  Re9G12 CDR2

<400> 155

Phe Val Ala His Ile Ser Trp Ser Gly Asp Ser Thr Tyr Tyr Ala
1               5               10                  15

<210> 156
<211> 19
<212> PRT
<213> artificial sequence

<220>
<223> Re9G12 CDR3

<400> 156

Ala Ala Asp Arg Gly Ala Ser Tyr Tyr Tyr Thr Trp Ala Ser Glu Tyr
1               5               10                  15

Asn Tyr Trp

<210> 157
<211> 123
<212> PRT
<213> artificial sequence

<220>
<223> Re9H01

<400> 157

Gly Ser Gln Val Gln Leu Val Glu Ser Gly Gly Gly Leu Val Gln Ala
1               5               10                  15

Gly Asp Ser Leu Arg Leu Ser Cys Ala Ala Ser Gly Asn Ile Phe Ser
            20              25                  30

Ile Asn Ala Met Gly Trp Tyr Arg Gln Ala Pro Gly Lys Gln Arg Glu
            35              40                  45

Leu Val Ala Phe Ile Thr Ser Arg Gly Ser Thr Asn Tyr Thr Asp Ser
            50              55                  60

Val Lys Gly Arg Phe Thr Ile Ser Arg Asp Thr Ala Lys Asp Thr Val
65              70              75                  80

Tyr Leu Gln Met Asn Ser Leu Lys Pro Glu Asp Thr Ala Val Tyr Phe
                85              90                  95

Cys Arg Gly Gly Tyr Ser Asp Tyr Asp Ile Tyr Phe Gly Ser Trp Gly
            100             105                 110

Gln Gly Thr Gln Val Thr Val Ser Ser Thr Ser

115                    120

<210> 158
<211> 9
<212> PRT
<213> artificial sequence

<220>
<223> Re9H01 CDR1

<400> 158

Asn Ile Phe Ser Ile Asn Ala Met Gly
1               5


<210> 159
<211> 14
<212> PRT
<213> artificial sequence

<220>
<223> Re9H01 CDR2

<400> 159

Leu Val Ala Phe Ile Thr Ser Arg Gly Ser Thr Asn Tyr Thr
1               5                   10


<210> 160
<211> 14
<212> PRT
<213> artificial sequence

<220>
<223> Re9H01 CDR3

<400> 160

Arg Gly Gly Tyr Ser Asp Tyr Asp Ile Tyr Phe Gly Ser Trp
1               5                   10


<210> 161
<211> 117
<212> PRT
<213> artificial sequence

<220>
<223> Re10B02

<400> 161

Gly Ser Gln Val Gln Leu Val Glu Ser Gly Gly Gly Leu Val Gln Pro
1               5                   10                  15


Gly Gly Ser Leu Arg Leu Ser Cys Ala Thr Ser Gly Ser Ile Ser Ser
            20                  25                  30

```
Tyr Arg Met Gly Trp Tyr Arg Gln Gly Pro Gly Lys Gln Arg Glu Leu
        35                  40                  45

Val Ala Phe Ile Thr Ile Gly Gly Ile Thr Asp Tyr Ile Asp Ser Val
        50                  55                  60

Lys Gly Arg Phe Thr Ile Ser Arg Asp Asn Ala Lys Asn Thr Met Tyr
65                  70                  75                  80

Leu Gln Met Asn Ser Leu Lys Pro Glu Asp Thr Ala Val Tyr Tyr Cys
                85                  90                  95

Asn Ala Asp Pro Pro Leu Phe Asn Trp Gly Gln Gly Thr Gln Val Thr
            100                 105                 110

Val Ser Ser Thr Ser
            115


<210>   162
<211>   8
<212>   PRT
<213>   artificial sequence

<220>
<223>   Re10B02 CDR1

<400>   162

Ser Ile Ser Ser Tyr Arg Met Gly
1                   5


<210>   163
<211>   14
<212>   PRT
<213>   artificial sequence

<220>
<223>   Re10B02 CDR2

<400>   163

Leu Val Ala Phe Ile Thr Ile Gly Gly Ile Thr Asp Tyr Ile
1                   5                   10


<210>   164
<211>   9
<212>   PRT
<213>   artificial sequence

<220>
<223>   Re10B02 CDR3

<400>   164

Asn Ala Asp Pro Pro Leu Phe Asn Trp
```

1                    5

<210>   165
<211>   131
<212>   PRT
<213>   artificial sequence

<220>
<223>   Re10B10

<400>   165

Gly Ser Gln Val Gln Leu Val Glu Ser Gly Gly Gly Leu Val Gln Pro
1                   5                   10                  15

Gly Gly Ser Leu Arg Leu Ser Cys Ala Ala Ser Gly Phe Thr Leu Asp
            20                  25                  30

Tyr Tyr Ala Ile Gly Trp Phe Arg Gln Ala Pro Gly Lys Glu Arg Glu
            35                  40                  45

Gly Val Ser Arg Ile Arg Ser Ser Asp Gly Ser Thr Thr Tyr Ala Asp
        50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Tyr Gly Pro Leu Thr Lys Tyr Gly Ser Ser Trp Tyr Trp
            100                 105                 110

Pro Tyr Glu Tyr Asp Tyr Trp Gly Gln Gly Thr Gln Val Thr Val Ser
            115                 120                 125

Ser Thr Ser
        130

<210>   166
<211>   9
<212>   PRT
<213>   artificial sequence

<220>
<223>   Re10B10 CDR1

<400>   166

Phe Thr Leu Asp Tyr Tyr Ala Ile Gly
1                   5

```
<210>  167
<211>  15
<212>  PRT
<213>  artificial sequence

<220>
<223>  Re10B10 CDR2

<400>  167

Gly Val Ser Arg Ile Arg Ser Ser Asp Gly Ser Thr Thr Tyr Ala
1               5                   10                  15


<210>  168
<211>  21
<212>  PRT
<213>  artificial sequence

<220>
<223>  Re10B10 CDR3

<400>  168

Ala Tyr Gly Pro Leu Thr Lys Tyr Gly Ser Ser Trp Tyr Trp Pro Tyr
1               5                   10                  15


Glu Tyr Asp Tyr Trp
                20


<210>  169
<211>  131
<212>  PRT
<213>  artificial sequence

<220>
<223>  Re10F10

<400>  169

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
            20                  25                  30


Tyr Tyr Ala Ile Gly Trp Phe Arg Gln Ala Pro Gly Lys Glu Arg Glu
            35                  40                  45


Gly Val Ser Arg Ile Arg Asn Asn Asp Gly Ser Thr Asp Tyr Ala Asp
        50                  55                  60


Ser Val Lys Gly Arg Phe Thr Ile Ser Arg Asp Asn Ala Lys Asn Thr
65                  70                  75                  80


Val Tyr Leu Gln Met Asn Ser Leu Lys Pro Glu Asp Thr Ala Val Tyr
```

```
                        85                    90                    95


           Tyr Cys Ala Tyr Gly Pro Leu Thr Lys Tyr Gly Ser Ser Trp Tyr Trp
                   100                 105                 110


           Pro Tyr Glu Tyr Asp Tyr Trp Gly Gln Gly Thr Gln Val Thr Val Ser
                   115                 120                 125


           Ser Thr Ser
               130


           <210>  170
           <211>  9
           <212>  PRT
           <213>  artificial sequence

           <220>
           <223>  Re10F10 CDR1

           <400>  170

           Phe Thr Leu Asp Tyr Tyr Ala Ile Gly
           1               5


           <210>  171
           <211>  15
           <212>  PRT
           <213>  artificial sequence

           <220>
           <223>  Re10F10 CDR2

           <400>  171

           Gly Val Ser Arg Ile Arg Asn Asn Asp Gly Ser Thr Asp Tyr Ala
           1               5                   10                  15


           <210>  172
           <211>  21
           <212>  PRT
           <213>  artificial sequence

           <220>
           <223>  Re10F10 CDR3

           <400>  172

           Ala Tyr Gly Pro Leu Thr Lys Tyr Gly Ser Ser Trp Tyr Trp Pro Tyr
           1               5                   10                  15


           Glu Tyr Asp Tyr Trp
                       20


           <210>  173
           <211>  120
```

<212> PRT
<213> artificial sequence

<220>
<223> Re11C10

<400> 173

Gly Ser Gln Val Gln Leu Val Glu Ser Gly Gly Gly Ser Val Glu Ala
1               5                   10                  15

Gly Gly Ser Leu Arg Leu Ser Cys Ala Ala Ser Gly Arg Thr Leu Asp
            20                  25                  30

Asn Tyr Asn Ala Val Trp Phe Arg Gln Ala Pro Gly Lys Glu Arg Glu
            35                  40                  45

Phe Val Ala Ala Ile Asn Trp Asn Gly Ser Asn Thr Ser Tyr Gly Asn
        50                  55                  60

Ser Val Lys Gly Arg Phe Ala Val Ser Arg Asp Asn Ala Lys Asn Thr
65                  70                  75                  80

Val Tyr Leu Gln Met Asn Asn Leu Lys His Glu Asp Thr Ala Glu Tyr
                85                  90                  95

Leu Cys Tyr Thr Val Gly Pro Glu Gly Asp Tyr Trp Gly Gln Gly Thr
            100                 105                 110

Gln Val Thr Val Ser Ser Thr Ser
        115                 120

<210> 174
<211> 9
<212> PRT
<213> artificial sequence

<220>
<223> Re11C10 CDR1

<400> 174

Arg Thr Leu Asp Asn Tyr Asn Ala Val
1               5

<210> 175
<211> 15
<212> PRT
<213> artificial sequence

<220>
<223> Re11C10 CDR2

<400> 175

Phe Val Ala Ala Ile Asn Trp Asn Gly Ser Asn Thr Ser Tyr Gly
1               5               10                  15

<210> 176
<211> 10
<212> PRT
<213> artificial sequence

<220>
<223> Re11C10 CDR3

<400> 176

Tyr Thr Val Gly Pro Glu Gly Asp Tyr Trp
1               5               10

<210> 177
<211> 120
<212> PRT
<213> artificial sequence

<220>
<223> Re11E11

<400> 177

Gly Ser Gln Val Gln Leu Val Glu Ser Gly Gly Gly Ser Val Glu Ala
1               5               10                  15

Gly Gly Ser Leu Arg Leu Ser Cys Ala Ala Ser Gly Arg Thr Phe Asn
            20              25              30

Asn Tyr Asn Ile Val Trp Phe Arg Gln Ala Pro Gly Lys Glu Arg Glu
            35              40              45

Phe Val Ala Ala Ile Asn Trp Asn Gly Gly Ser Thr Ser Tyr Ala Asn
            50              55              60

Ser Val Lys Gly Arg Phe Ala Val Ser Arg Asp Asn Ala Lys Asn Thr
65              70              75              80

Val Tyr Leu Gln Met Asn Asn Leu Lys His Glu Asp Thr Ala Glu Tyr
                85              90                  95

Leu Cys Tyr Thr Val Gly Pro Glu Gly Asp Tyr Trp Gly Gln Gly Thr
            100             105             110

Gln Val Thr Val Ser Ser Thr Ser
        115             120

<210> 178
<211> 9

```
<212>  PRT
<213>  artificial sequence

<220>
<223>  Re11E11 CDR1

<400>  178

Arg Thr Phe Asn Asn Tyr Asn Ile Val
1               5


<210>  179
<211>  15
<212>  PRT
<213>  artificial sequence

<220>
<223>  Re11E11 CDR2

<400>  179

Phe Val Ala Ala Ile Asn Trp Asn Gly Gly Ser Thr Ser Tyr Ala
1               5                   10                  15


<210>  180
<211>  10
<212>  PRT
<213>  artificial sequence

<220>
<223>  Re11E11 CDR3

<400>  180

Tyr Thr Val Gly Pro Glu Gly Asp Tyr Trp
1               5                   10


<210>  181
<211>  126
<212>  PRT
<213>  artificial sequence

<220>
<223>  Re11F07

<400>  181

Gly Ser Gln Val Gln Leu Val Glu Ser Gly Gly Gly Leu Val Gln Ala
1               5                   10                  15


Gly Gly Ser Leu Arg Leu Ser Cys Ala Ala Ser Gly Arg Ala Phe Ser
                20                  25                  30


Ser Gly Thr Met Gly Trp Phe Arg Gln Ala Pro Gly Lys Glu Arg Glu
                35                  40                  45


Phe Val Ala Thr Ile Ser Trp Ser Gly Gly Ser Thr Ser Tyr Ala Arg
```

Ser Val Lys Gly Arg Phe Thr Ile Ser Gly Asp Asn Ala Glu Asn Thr
65              70              75              80

Val Tyr Leu Gln Met Asn Ser Leu Lys Pro Glu Asp Thr Ala Val Tyr
            85              90              95

Tyr Cys Tyr Ala Val Ser Ser Gly Ser Asp Tyr Asp Gly Gly Met Asp
        100             105             110

Tyr Trp Gly Lys Gly Thr Leu Val Thr Val Ser Ser Thr Ser
    115             120             125

<210>   182
<211>   9
<212>   PRT
<213>   artificial sequence

<220>
<223>   Re11F07 CDR1

<400>   182

Arg Ala Phe Ser Ser Gly Thr Met Gly
1               5

<210>   183
<211>   15
<212>   PRT
<213>   artificial sequence

<220>
<223>   Re11F07 CDR2

<400>   183

Phe Val Ala Thr Ile Ser Trp Ser Gly Gly Ser Thr Ser Tyr Ala
1               5               10              15

<210>   184
<211>   16
<212>   PRT
<213>   artificial sequence

<220>
<223>   Re11F07 CDR3

<400>   184

Tyr Ala Val Ser Ser Gly Ser Asp Tyr Asp Gly Gly Met Asp Tyr Trp
1               5               10              15

<210>   185
<211>   124

<210> PRT
<213> artificial sequence

<220>
<223> Re11F11

<400> 185

Gly Ser Gln Val Gln Leu Val Glu Ser Gly Gly Gly Leu Val Gln Pro
1               5                   10                  15

Gly Gly Ser Leu Arg Leu Ser Cys Ala Ala Ser Gly Phe Thr Phe Ser
            20                  25                  30

Asn Tyr His Met Ser Trp Tyr Arg Gln Ala Pro Gly Lys Gly Arg Glu
        35                  40                  45

Leu Val Ala Asp Ile Thr Ser Gly Gly Asp Tyr Thr His Tyr Ala Asp
        50                  55                  60

Ser Val Lys Gly Arg Phe Thr Val Ser Arg Asp Asn Pro Lys Asn Thr
65                  70                  75                  80

Leu Tyr Leu Gln Met Asn Ser Leu Lys Pro Glu Asp Thr Ala Val Tyr
                85                  90                  95

His Cys His Val Arg Ile Phe Gly Pro Gly Phe Pro Val Asp Tyr Arg
                100                 105                 110

Gly Gln Gly Thr Gln Val Thr Val Ser Ser Thr Ser
            115                 120

<210> 186
<211> 9
<212> PRT
<213> artificial sequence

<220>
<223> Re11F11 CDR1

<400> 186

Phe Thr Phe Ser Asn Tyr His Met Ser
1               5

<210> 187
<211> 15
<212> PRT
<213> artificial sequence

<220>
<223> Re11F11 CDR2

<400> 187

Leu Val Ala Asp Ile Thr Ser Gly Gly Asp Tyr Thr His Tyr Ala
1           5           10              15

<210> 188
<211> 14
<212> PRT
<213> artificial sequence

<220>
<223> Re11F11 CDR3

<400> 188

His Val Arg Ile Phe Gly Pro Gly Phe Pro Val Asp Tyr Arg
1           5           10

<210> 189
<211> 121
<212> PRT
<213> artificial sequence

<220>
<223> Re11G09

<400> 189

Gly Ser Gln Val Gln Leu Val Glu Ser Gly Gly Gly Leu Val His Thr
1           5           10              15

Gly Gly Ser Leu Arg Leu Ser Cys Ala Ala Ser Gly Ser Ile Phe Asn
            20          25          30

Ile Tyr Arg Met Ala Trp Tyr Arg Gln Ala Pro Gly Lys Gln Arg Glu
            35          40          45

Lys Val Ala Ile Ile Thr Thr Tyr Gly Leu Thr Asp Tyr Ala Asp Ser
            50          55          60

Val Lys Gly Arg Phe Thr Ile Ser Arg Asp Asn Ala Lys Asn Thr Thr
65          70          75          80

Tyr Leu Gln Met Asn Ser Leu Lys Pro Asp Asp Thr Ala Val Tyr Tyr
                85          90          95

Cys Asn Thr Asp Pro Pro Asp Leu Gly Pro Gly Tyr Trp Gly Gln Gly
                100         105         110

Thr Gln Val Thr Val Ser Ser Thr Ser
            115         120

<210> 190
<211> 9

95

<212> PRT
<213> artificial sequence

<220>
<223> Re11G09 CDR1

<400> 190

Ser Ile Phe Asn Ile Tyr Arg Met Ala
1               5


<210> 191
<211> 14
<212> PRT
<213> artificial sequence

<220>
<223> Re11G09 CDR2

<400> 191

Lys Val Ala Ile Ile Thr Thr Tyr Gly Leu Thr Asp Tyr Ala
1               5                   10


<210> 192
<211> 12
<212> PRT
<213> artificial sequence

<220>
<223> Re11G09 CDR3

<400> 192

Asn Thr Asp Pro Pro Asp Leu Gly Pro Gly Tyr Trp
1               5                   10


<210> 193
<211> 129
<212> PRT
<213> artificial sequence

<220>
<223> Re11H04

<400> 193

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
                20                  25                  30


Tyr Tyr Thr Ile Ala Trp Phe Arg Gln Ala Pro Gly Lys Glu Arg Glu
            35                  40                  45


Gly Val Ser Cys Ile Ser Gly Asn Asp Gly Ser Thr Tyr Tyr Ala Asp

```
                50                      55                         60


        Ser Val Lys Gly Arg Phe Thr Ile Ser Arg Asp Asn Ala Lys Asn Thr
        65                  70              75                  80


        Val Tyr Leu Gln Met Asn Ser Leu Lys Pro Glu Asp Thr Ala Val Tyr
                        85              90                  95


        Tyr Cys Ala Ala Asp Arg Gly Glu Ser Tyr Tyr Pro Phe Arg Pro Ser
                100             105             110


        Glu Tyr His Tyr Trp Gly Gln Gly Thr Gln Val Thr Val Ser Ser Thr
                115             120             125


        Ser


        <210>  194
        <211>  9
        <212>  PRT
        <213>  artificial sequence

        <220>
        <223>  Re11H04 CDR1

        <400>  194

        Phe Thr Leu Asp Tyr Tyr Thr Ile Ala
        1               5


        <210>  195
        <211>  15
        <212>  PRT
        <213>  artificial sequence

        <220>
        <223>  Re11H04 CDR2

        <400>  195

        Gly Val Ser Cys Ile Ser Gly Asn Asp Gly Ser Thr Tyr Tyr Ala
        1               5               10                  15


        <210>  196
        <211>  19
        <212>  PRT
        <213>  artificial sequence

        <220>
        <223>  Re11H04 CDR3

        <400>  196

        Ala Ala Asp Arg Gly Glu Ser Tyr Tyr Pro Phe Arg Pro Ser Glu Tyr
        1               5               10                  15
```

```
His Tyr Trp


<210>   197
<211>   31
<212>   PRT
<213>   artificial sequence

<220>
<223>   Spacer

<400>   197

Glu Gly Ser Glu Gly Pro Glu Ser Ser Asp Gly Ser Asp Ser Thr Asp
1               5               10              15


Pro Gly Glu Gln Gly Glu Gly Ala Asp Ala Ser Asp Gly Ser Glu
        20              25              30


<210>   198
<211>   57
<212>   PRT
<213>   artificial sequence

<220>
<223>   human collagen XVIII NC trimerization domain

<400>   198

Gly Ala Ser Ser Gly Val Arg Leu Trp Ala Thr Arg Gln Ala Met Leu
1               5               10              15


Gly Gln Val His Glu Val Pro Glu Gly Trp Leu Ile Phe Val Ala Glu
        20              25              30


Gln Glu Glu Leu Tyr Val Arg Val Gln Asn Gly Phe Arg Lys Val Gln
        35              40              45


Leu Glu Ala Arg Thr Pro Leu Pro Arg
        50              55
```

## Claims

1. A VHH antibody recognizing the SARS-CoV-2 spike protein S1 domain, particularly the receptor-binding domain (RBD) of the SARS-CoV-2 S1 domain comprising

   (a) a CDR3 sequence as shown in SEQ ID NO. 20, 4, 8, 12, 16, 24, 28, 32, 36, 40, 44, 48, 52, 56, 60, 64, 68, 72, 76, 80, 84, 88, 92, 96, 100, 104, 108, 112, 116, 120, 124, 128, 132, 136, 140, 144, 148, 152, 156, 160, 164, 168, 172, 176, 180, 184, 188, 192 or 196,
   (b) a CDR3 sequence which has an identity of at least 80%, or at least 90% to a CDR3 sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.

2. The VHH antibody according to claim 1 comprising

(a) a combination of CDR1, CDR2 and CDR3 sequences as shown in SEQ ID NO. 18-20, 2-4, 6-8, 10-12, 14-16, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108, 110-112, 114-116, 118-120, 122-124, 126-128, 130-132, 134-136, 138-140, 142-144, 146-148, 150-152, 154-156, 158-160, 162-164, 166-168, 170-172, 174-176, 178-180, 182-184, 186-188, 190-192 or 194-196,
(b) a combination of CDR1, CDR2 and CDR3 sequences which has an identity of at least 80%, at least 90%, or at least 95% to a combination of CDR1, CDR2 and CDR3 sequences of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

3. The VHH antibody according to claim 1 or 2 comprising

(a) a VHH sequence as shown in SEQ ID NO. 17, 1, 5, 9, 13, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 177, 181, 185, 189 or 193,
(b) a sequence which has an identity of at least 70%, at least 80%, at least 90%, at least 95%, or at least 99% to a VHH sequence of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

4. The VHH antibody of any one of claims 1-3, which is capable of virus neutralization.

5. The VHH antibody of any one of claims 1-4, which is covalently or non-covalently conjugated to a heterologous moiety, e.g. a labeling group, a capture group or an effector group.

6. The VHH antibody of any one of claims 1-5, which is fused to a heterologous polypeptide moiety, e.g. a multimerization module, e.g. dimerization, trimerization or tetramerization module.

7. The VHH antibody of claim 6, which is a homotrimerized VHH antibody fused to a trimerization module, e.g. a collagen trimerization moiety, particularly a human collagen moiety, and a lung surfactant protein D moiety.

8. The VHH antibody of claim 6 or 7, which is fused to a heterologous polypeptide moiety via a spacer, e.g. a spacer having a chain length of 5-100 amino acids, particularly selected from Gly, Ser, Ala, Thr, Pro, Glu and/or Asp.

9. A set of two or more different VHH antibodies of any of claims 1-8.

10. The set of claim 9, wherein the different VHH antibodies recognize different epitopes on the RBD, particularly non-overlapping epitopes on the RBD.

11. The set of claim 9 or 10 for the detection of SARS-CoV-2 virus comprising

(i) a set of at least two VHH antibodies recognizing different, particularly non-overlapping epitopes on the RBD,
(ii) a set of at least two VHH antibodies comprising a set of capturing antibodies conjugated to a capturing moiety and a set of labeling antibodies conjugated to a labeling moiety, and/or
(iii) a set of at least two VHH antibodies comprising a first set of labeling antibodies conjugated to a first labeling moiety, and a second set of labeling antibodies conjugated to a second labeling moiety, wherein the first labeling moiety is different from the second labeling moiety, wherein the first and the second labeling moieties are particularly selected from two spectrally different fluorescence labeling moieties.

12. A homotrimeric VHH antibody comprising 3 VHH antibody subunits, wherein each VHH antibody subunit comprises a VHH antibody directly linked or linked via a spacer to a trimerization module, particularly wherein the homotrimeric VHH antibody comprises three identical VHH antibody subunits.

13. The homotrimeric VHH antibody of claim 12, which is directed to a target structure selected from

(i) a viral protein or protein assembly, particularly a homo-trimeric viral protein or protein assembly, more particularly selected from a Coronavirus trimeric spike protein, an Orthomyxovirus trimeric HA protein, a Paramyxovirus trimeric fusion protein, a Dengue fever virus or Zikavirus trimeric or higher order protein assembly, a Herpesvirus trimeric gB fusion protein or a HIV trimeric gp120 protein, and even more the receptor-binding domain (RBD) of the SARS-CoV-2 S1 domain and

(ii) a non-viral protein or protein assembly, particularly a homo-trimeric non-viral protein or protein assembly, more particularly a member of the TNF Ligand superfamily or the TNF Receptor superfamily.

14. The VHH antibody of any one of claims 1-8 or the homotrimeric VHH antibody of claim 12 or 13, which is non-glycosylated.

15. The VHH antibody of any one of claims 1-8 or 14, the set of claim 9, 10, or 11, or the homotrimeric VHH antibody of claim 12, 13 or 14 for use in medicine, e.g. human medicine, particularly for use in therapy or diagnostics, more particularly for the prevention or treatment of a disorder caused by and/or associated with an infection with SARS-CoV-2.

CDR1　　　　　　　CDR2　　　　　　　　　　CDR3

Re5A08　GSQVQLVESGGGLVQAGGSLRLSCTASGHT----FTANRMGWFRQAPGKEREFVAAIN---WGGDSTNYVDSVRGRFTISRDIAKNTVYLQMNSLKPEDTAVYFCAARNEV-----------TGEFDSNGQGTQVTVSSTS
Re5B06　GSQVQLVESGGGLVQPGGSLRLSCAASGSI----RSIVATVWFRQAPGKEHEWVGSIT---SS-NVTTYADSVRGRFTISRDNAKKTVYLQMNSLKPEDTALYYCNVHFASEY---------SDYAQIQGQGTQVTVSSTS
Re5C01　GSQVQLVESGGGLVQAGGSLRLSCGVSGRT----FSSYAMGWFRQAPGKEREFVATIS---WSGGTTNYABSVRGRFTISRDNAKNTVYLQMNSLKVEDTAVYYCIAVSSG--------SDYDGGMDYWGKGTQVTVSSTS
Re5C08　GSQVQLVESGGGSVEAGGSLRLSCAASGRT----FNDYNMVWFRQAPGKEREFVAAIK---WNGGNTSYADSVRGRFAVGRDNAKNTVYLQMNNLKHEDTAEYLCVTVGP-----------EGDYWGQGTQVTVSSTS
Re5D06　GSQVQLVESGGGLVQPGGSLRLSCAASGIT----LDYYAIGWFRQAPGKEREGVSRIR---SSDGSTNYADSVRGRFTMSRDNAKNTVYLQMNSLKPEDTAVYYCAIGPLTKYGSSWIWPY---EYDYWGQGTQVTVSSTS
Re5E03　GSQVQLVESGGGLVQPGGSLRLSCAASGFT----LDYYAIGWFRQAPGKEREGVSCIS---NSDGSTRYADSVRGRFTISRDNAKNTVYLQMNSLKPEDTAVYYCAGGPQTYISGSIYYTCAEGAMDYWGKGTLVTVSSTS
Re5E11　GSQVQLVESGGGLVQPGGSLRLSCAASGFT----LDYYAIGWFRQAPGKEREGVSCIS---SSDGRTYYADSVRGRFTISRDNAKNTVYLQMNSLKPEDTAVYYCATAPLIYYSGSWYLTCNSDAMDYWGKGTLVTVSSTS
Re5F10　GSQVQLVESGGGLVQPGGSLRLSCVASGFT----FSSFAMGWFRQAPGKECEWWATIT---ITGGSTNYADSVRGRFTISRDNAKNTVYLQMNSLKPEDTAVYYCNPDPG-----------C-RRGQGTQVTVSSTS
Re5F11　GSQVQLVESGGALVQPGGSLRLSCATSGSI-----SSYRMGWYRQGPGKQRELVAFIT---IG-GITDYIDSVRGRFTISRDNAKNTMYLQMNSLKPEDTAVYYCADPPL-----------FNNGQGTQVTVSSTS
Re5G05　GSQVQLVESGGGLVQAGGSLRLSCAASGFT----ATSYAMGWYRQAPGKECEWWATIT---STGGNTNYADSVRGRFTISRDNAKNTVYLQMNSLKPEDTAVYYCNPDPG-----------C-DNGQGTQVTVSSTS
Re6A11　GSQVQLVESGGGLVQAGGSLRLSCAASGRT----FSNDALGWFRQAPRKEREFVAAIN---WNS-GTYYADSVRGRFTISRDNAKNTVYLQMNSLKPEDTAVYSCAAASDYGL------PREDFLYDYWGQGTLVTVSSTS
Re6B02　GSQVQLVESGGALVQPGGSLRLSCVASGFT----LDYYAIGWFRQAPGKEREGVSRIR---SSDGSTNYADSVRGRFTISRDNAKNTVYLQMNSLKPEDTAVYYCAIGPLTKYGSSWIWPY---EYDYWGQGTQVTVSSTS
Re6B06　GSQVQLVESGGGLVQAGGSLRLSCAASGRA----FSSAPMSWFRQAPGKEREFVASVS---WSGDSTNYADSVRGRFTISRDNAKNTGYLQMNSLKPEDTAVYYCKRG-----------------PYWGQGTQVTVSSTS
Re6B07　GSQVQLVESGGGLVQPGGSLRLSCAASGFT----LDYYAIGWFRQAPGKEREGVSYIR---SSDGTTNYADSVRGRFTISRDNAKNTVYLQMNSLKPEDTAVYYCAADEAYYSELGWESPW---GWSYWGQGTRVTVSSTS
Re6D06　GSQVQLVESGGGLVQAGASLRLSCAASGRM----FGVYRMGWFRQAPGKEREFVAGIS---TSVGTTNYADSVRGRFTISRDNAKNTVYLQMNSLKPEDTAVYYCAARDPTT-----------YEYDYWGQGTQVTVSSTS
Re6D08　GSQVQLVESGGGLVQAGGSLRLSCAASGRT----FSSYAMGWFHQAPGKEREFVATIN---WSGDSTYYADSVRGRFTISRDNAKNTVYLQMNSLKPEDTAVYYCAAVVDPSPTY-ISGKYYPPRVYWGKGTQVTVSSTS
Re6D09　GSQVQLVESGGGSVEAGGSLRLSCAASGRT----FNNYNMVWFRQAPGKEREFVAAIN---WNCGSTSYAASVRGRFAVGIDNAKNTVYLQMNNLKHEDTAEYLCVTVGP-----------EGDYWGQGTQVTVSSTS
Re6E11　GSQVQLVESGGGLVQPGGSLRLSCAASGLT----LDYYAIGWFRQAPGKEREGVSCIS---SRDGSTMYADSVRGRFTISRDNAKNTVYLQMNSLKPEDTAVYYCAATPTTYYSGSYYYTCSPEGYDYWGQGTQVTVSSTS
Re6F06　GSQVQLVESGGGLVQAGGSLRLSCAASGFT----FSNYAMGWFRQAPGKECEFVAVIT---ITGSNTNYADSVRGRFTISRDNAKNTVYLQMNSLKPEDTAVYYCNPDPG-----------CESQGQGTRVTVSSTS
Re6G03　GSQVQLVESGGGLVQAGGSLRLSCAASGRT----FSTYRMAWFRLAPGKEREFVAGIN---WSDGTTSYKDSVRGRFTISRDNAKNTVYLQMSLKPEDTAVYYCNAHLGTGQ----EGPGEYFGMDYWGKGTQVTVSSTS
Re6H06　GSQVQLVESGGGLVQPGGSLRLSCAASGVT----LDYYAIGWFRQAPGKEREGVSCIS---SSDGSTYYADSVRGRFTISRDNAKNTVYLQMNSLKPEDTGVYYCAVVPQTYYGGKYYSQCTANGMDYWGKGTLVTVSSTS
Re6H10　GSQVQLVESGGGLVQPGGSLRLSCAASGFT----FSSYAMGWYRQAPGKECEFVAVIT---ITGGSTNYADSVRGRFTISRDNAKNTVYLQMNSLKPEDTAVYYCNPDPG-----------CRGGGQGTLVTVSSTS
Re7A01　GSQVQLVESGGGLVQAGGSLRLSCAASGRT----FSSYAMGWFRQAPGKEREFVATIS---ESG-STSYAGHVRGRFTISRDNAKNTVYLQMNSLKPEDTAVYYCHAVTRA--------SDQDGGMDYWGKGTQVTVSSTS
Re7B01　GSQVQLVESGGGLVQPGGSLRLSCGASGFT----LDYYAIGWFRQAPGKEREGVSRIR---GNDGSTYYADSVRGRFTISRDNAKNTVYLQMNSLKPEDTAVYYCAIGPLTKYGSSWYWPY---EYDYWGQGTQVTVSSTS
Re7D05　GSQVQLVESGGGLVQAGGSLRLSCAASGRT----FSSYAMGWFRQAPGKEREFVATIS---WSGGSTSYADSVRGRFTISRDNAKNTVYLQMNSLKPEDTAVYYCNAVTHH--------SDQDGGMDYWGKGTLVTVSSTS
Re7E02　GSQVQLVESGGGLVQAGGSLRLSCAASGFT----LDYYAIGWFRQAPGKEREGVSYIR---SSDGTTNYADSVRGRFTISRDNAKNTVYLQMNSLKPEDTAVYYCAADEAYYSELGWESPW---GWSYWGQGTQVTVSSTS

Figure 1

```
                                    CDR1                        CDR2                                                      CDR3
Re7H02   GSQVQLVESGGGLVQAGGSLRLSCAASGRA----FSSAPMSWFRQAPGKEREFVASVS---WSGDSTNYADSVKGRFTISRDNAENTGYLQMNSLKPEDTAVYYCKRG-----------------PYKQGTQVTVSSTS
Re8A03   GSQVQLVESGGGLVQPGGSLRLSCAASGRI----TGFNGMGWYRQTPGKQRELVASIT---NG-GITKYADSVKGRFTISRDNAKNTVYLQMNSLKPEDTAVYLCTFWRPEP------PNLYWGQGTQVTVSSTS
Re8A06   GSQVQLVESGGGLVQAGGSLRLSCAASGSI----FSINAMGWYRQAPGKERELVAAMG---SSG-WINYADSVKGRLTISRDNAKNTLYLQMNSLKPEDTAVYYCRGTGGVG----------PTSADYWGQGTQVTVSSTS
Re8C06   GSQVQLVESGGGLVQAGGSLRLSCAASGRT----DTTYNMGWFRQAPGKEREFVAAIS---WSDGKTTFADSVKGRFTISRDNAKNTVYLQMNSLKPEDTANYYCAAKAPLV------AGRSLEEYDYSGQGTQVTVSSTS
Re8E12   GSQVQLVESGGGSVQPGGSLRLSCKVSGFTSDVDLRNYLVSWNRQAPGKERELVAAITPTVISGGNTNYADSVKGRFTISRDISRSTVYLQMNSLNPEDTAVYYCKVG-----------------VYKQGTQVTVSSTS
Re8F03   GSQVQLVESGGGLVQPGGSLTLSCKVSGLTSVVDLRNYLVSWYRQGPGKERELVAAITPTAITGGSTNYADSVKGRFTISRDTGKSTVYLQMNSLNPEDTAVYSCKVG-----------------VYKQGTQVTVSSTS
Re9B09   GSQVQLVESGGGLVQPGGSLRLSCAASGFT----LDYYAIGWFRQAPGKEREGVSRIS---SSDGSTDYADSVKGRFTISRDNAKNTVYLQMNSLKPEDTAVYYCATVPGTYYSGNWYYTWHPEAVYKCKGTQVTVSSTS
Re9B10   GSQVQLVESGGGLVQPGGSLRLSCAASGRM----FGVYRMGWFRQAPGKEREFVAGIS---TSVGTTNYADSVKGRFTISRDNAKNTVYLQMNSLKPEDTAVYYCAARDPTT-----------VEYDYWGQGTQVTVSSTS
Re9C07   GSQVQLVESGGGLVQAGGSLRLSCAASGRT----FSRYAMGWFRQAPGKEREFVAAIT---WNADTTYYADSVKGRFTISRDNAKNTVYLQMNSLKPEDTAVYYCAAGGNHYYSR---SVYSSLEYDHWGQGTQVTVSSTS
Re9C08   GSQVQLVESGGGLVQPGGSLRLSCAVSGNI----FGITAWDWHRQAPGKQRELVAHIT---SR-GDTMYLDSVKGRFAISRDHAKNTLGLQMNSLKPEDTAVYYCLLRTFGP-----------PNDHWGQGTQVTVSSTS
Re9D02   GSQVQLVESGGGLVQAGGSLRLSCAASGRT----FSNYAMGWFRQAPGKEREFVAAIS---WGGDTTYYADSLKGRFTISRDNAKNTVYLQMNSLKPEDTAVYYCAADRGLSY-----TYDRVTEYDYWGQGTQVTVSSTS
Re9G05   GSQVQLVESGGGLVQPGGSLRLSCAVSGNI----SSITAWDWHRQAPGKQRELVAHIT---SR-GDTMYLDSVKGRFAISRDHAKNTLGLQMNSLKPEDTAVYYCLLRTFGP-----------PYDYWGQGTQVTVSSTS
Re9G12   GSQVQLVESGGGLVQAGGSLRLSCAASGRT----FSSYVMGWFRQAPGKEREFVAHIS---WSGDSTNYADSVKGRFTIPRDNAKNTAYLQMNSLKPEDTAVYYCAADRGASY-----YYTWASEYNYWGQGTQVTVSSTS
Re9H01   GSQVQLVESGGGLVQAGDGLRLSCAASGNI----FSINAMGWYRQAPGKQRELVAFIT---SR-GSTNYTDSVKGRFTISRDTARDTVYLQMNSLKPEDTAVYFCRGGYSDY----DIYFGSKGQGTQVTVSSTS
Re10B02  GSQVQLVESGGGLVQPGGSLRLSCATSGSI----SSYRMGWYRQGPGKQRELVAFIT---NG-GITDYIDSVKGRFTISRDNAKNTMYLQMNSLKPEDTAVYYCNADPPI-----------ENNWGQGTQVTVSSTS
Re10B10  GSQVQLVESGGGLVQPGGSLRLSCAASGFT----LDYYAIGWFRQAPGKEREGVSRIR---SSDGSTTYADSVKGRFTISRDNAKNTVYLQMNSLKPEDTAVYYCAYGPLTKYGSSWYWPY---EYDYWGQGTQVTVSSTS
Re10F10  GSQVQLVESGGGLVQPGGSLRLSCAASGFT----LDYYAIGWFRQAPGKEREGVSRIR---NNDGSTDYADSVKGRFTISRDNAKNTVYLQMNSLKPEDTAVYYCAYGPLIKYGSSWYWPY---EYDYWGQGTQVTVSSTS
Re11C10  GSQVQLVESGGGSVEAGGSLRLSCAASGRT----LDNYNAVWFRQAPGKEREFVAAIN---WNGSNTSYGNSVKGRFAISRDNAKNTVYLQMNNLKHEDTAEYLCTTVGP--------------EGDYWGQGTQVTVSSTS
Re11E11  GSQVQLVESGGGSVEAGGSLRLSCAASGRT----FNNYNTUWFRQAPGKEREFVAAIN---WNGGSTSYANSVKGRFAISRDNAKNTVYLQMNNLKHEDTAEYLCTTVGP--------------EGDYWGQGTQVTVSSTS
Re11F07  GSQVQLVESGGGLVQAGGSLRLSCAASGRA----FSSGTMGWFRQAPGKEREFVATIS---WSGGSTSYARSVKGRFTISGDNAENTVYLQMNSLKPEDTAVYYCAUSSG---------SDYDGGMDYNCKGTLVTVSSTS
Re11F11  GSQVQLVESGGGLVQPGGSLRLSCAASGFT----FSNYHMSWYRQAPGKGRELVADIT---SGGDYTHYADSVKGRFTYSRDNPKNTLYLQMNSLKPEDTAVYHCBVRIPGP----------GFPNDYRGQGTQVTVSSTS
Re11G09  GSQVQLVESGGGLVHTGGSLRLSCAASGSI----FNIVRMAWYRQAPGKQREKVAIIT---TY-GLTDYADSVKGRFTISRDNAKNTTYLQMNSLKPDDTAVYYCNTDPPL-----------GPGYWGQGTQVTVSSTS
Re11H04  GSQVQLVESGGGLVQPGGSLRLSCAASGFT----LDYYTTAWFRQAPGKEREGVSCIS---GNDGSTYYADSVKGRFTISRDNAKNTVYLQMNSLKPEDTAVYYCAADRG-----ESYIPFRPSEYHYWGQGTQVTVSSTS
```

Figure 1 (continued)

Staining of SAS-CoV-2 spike-transfected cells
stained with DAPI and VHH Re6D09

Figure 2

Staining of cells with DAPI and VHH antibodies

Figure 3

Figure 4

VHH antibody

flexible,
immuno-neutral linker

Immuno-neutral
trimerisation domain

Figure 5

Detection of the SARS-CoV-2 spike protein with VHH Re6A11 at

Figure 6

Europäisches
Patentamt

European
Patent Office

Office européen
des brevets

# EUROPEAN SEARCH REPORT

Application Number

EP 20 18 8451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Thomas J. Esparza ET AL: "High Affinity Nanobodies Block SARS-CoV-2 Spike Receptor Binding Domain Interaction with Human Angiotensin Converting Enzyme", bioRxiv, 24 July 2020 (2020-07-24), XP055763653, DOI: 10.1101/2020.07.24.219857 Retrieved from the Internet: URL:https://www.biorxiv.org/content/10.1101/2020.07.24.219857v1.full.pdf [retrieved on 2021-01-11] | 1-5, 7-11,14, 15 | INV. A61P31/14 C07K16/10 |
| Y | * figures 4-7 * | 6-8 | |
| X | DANIEL WRAPP ET AL: "Structural Basis for Potent Neutralization of Betacoronaviruses by Single-Domain Camelid Antibodies", CELL, vol. 181, no. 5, 28 May 2020 (2020-05-28), pages 1004-1015.e15, XP055764639, Amsterdam NL ISSN: 0092-8674, DOI: 10.1016/j.cell.2020.04.031 | 1-5, 9-11,14, 15 | |
| | | | TECHNICAL FIELDS SEARCHED (IPC) |
| Y | * figures 4a,5,6 * | 6-8 | C07K A61P |
| X | Leo Hanke ET AL: "An alpaca nanobody neutralizes SARS-CoV-2 by blocking receptor interaction", bioRxiv, 2 June 2020 (2020-06-02), XP055764643, DOI: 10.1101/2020.06.02.130161 Retrieved from the Internet: URL:https://www.biorxiv.org/content/10.1101/2020.06.02.130161v1.full.pdf [retrieved on 2021-01-13] | 1-5, 9-11,14, 15 | |
| Y | * figures 1-3 * | 6-8 | |

-/--

The present search report has been drawn up for all claims

| Place of search | Date of completion of the search | Examiner |
|---|---|---|
| Munich | 13 January 2021 | Cilensek, Zoran |

EPO FORM 1503 03.82 (P04C01)

page 1 of 3

Europäisches
Patentamt

European
Patent Office

Office européen
des brevets

**EUROPEAN SEARCH REPORT**

Application Number

EP 20 18 8451

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WU YANLING ET AL: "Identification of Human Single-Domain Antibodies against SARS-CoV-2", CELL HOST & MICROBE, ELSEVIER, NL, vol. 27, no. 6, 14 May 2020 (2020-05-14), page 891, XP086178478, ISSN: 1931-3128, DOI: 10.1016/J.CHOM.2020.04.023 [retrieved on 2020-05-14] | 1-5, 9-11,14, 15 | |
| Y | * figures 2,3 * | 6-8 | |
| X | JIANBO DONG ET AL: "Development of multi-specific humanized llama antibodies blocking SARS-CoV-2/ACE2 interaction with high affinity and avidity", EMERGING MICROBES & INFECTIONS, no. 1, 22 May 2020 (2020-05-22), pages 1034-1036, XP055735314, ISSN: 2222-1751, DOI: 10.1080/22221751.2020.1768806 | 1-5, 9-11,14, 15 | |
| Y | * figure 1 * | 6-8 | TECHNICAL FIELDS SEARCHED (IPC) |
| Y,D | ANA ALVAREZ-CIENFUEGOS ET AL: "Intramolecular trimerization, a novel strategy for making multispecific antibodies with controlled orientation of the antigen binding domains", SCIENTIFIC REPORTS, vol. 6, 27 January 2016 (2016-01-27), page 28643, XP055312689, DOI: 10.1038/srep28643 * figure 1 * | 6-8 | |
| X | WO 2019/234187 A1 (LEADARTIS S L [ES]) 12 December 2019 (2019-12-12) | 12-15 | |
| Y | * figures 2,9 * | 6-8 | |

-/--

The present search report has been drawn up for all claims

| Place of search | Date of completion of the search | Examiner |
|---|---|---|
| Munich | 13 January 2021 | Cilensek, Zoran |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 18 8451

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 441 776 A1 (LEADARTIS S L [ES]) 18 April 2012 (2012-04-18) | 12,13 | |
| Y | * paragraphs [0020], [0024] * | 6-8 | |
| | ----- | | |

TECHNICAL FIELDS
SEARCHED    (IPC)

The present search report has been drawn up for all claims

| Place of search | Date of completion of the search | Examiner |
|---|---|---|
| Munich | 13 January 2021 | Cilensek, Zoran |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 18 8451

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2021

| Patent document cited in search report | | Publication date | Patent family member(s) | Publication date |
|---|---|---|---|---|
| WO 2019234187 | A1 | 12-12-2019 | NONE | |
| EP 2441776 | A1 | 18-04-2012 | NONE | |

EPO FORM P0459

For more details about this annex : see Official Journal of the European Patent Office, No. 12/82

## REFERENCES CITED IN THE DESCRIPTION

**Non-patent literature cited in the description**

- **ALVAREZ-CIENFUEGOS, A. ; NUÑEZ-PRADO, N. ; COMPTE, M. ; CUESTA, A. M. ; BLANCO-TORIBIO, A. ; HARWOOD, S. L. ; VILLATE, M. ; MERINO, N. ; BONET, J. ; NAVARRO, R.** Intramolecular trimerization, a novel strategy for making multispecific antibodies with controlled orientation of the antigen binding domains. *Sci Rep,* 2016, vol. 6, 28643
- **ARBABI GHAHROUDI, M. ; DESMYTER, A. ; WYNS, L. ; HAMERS, R. ; MUYLDERMANS, S.** Selection and identification of single domain antibody fragments from camel heavy-chain antibodies. *FEBS Lett,* 1997, vol. 414, 521-526
- **BAR-ON, Y. M. ; FLAMHOLZ, A. ; PHILLIPS, R. ; MILO, R.** SARS-CoV-2 (COVID-19) by the numbers. *eLife,* 2020, 9
- **BOGIN, O. ; KVANSAKUL, M. ; ROM, E. ; SINGER, J. ; YAYON, A. ; HOHENESTER, E.** Insight into Schmid metaphyseal chondrodysplasia from the crystal structure of the collagen X NC1 domain trimer. *Structure,* 2002, vol. 10, 165-173
- **BOUDKO, S. P. ; SASAKI, T. ; ENGEL, J. ; LERCH, T. F. ; NIX, J. ; CHAPMAN, M. S. ; BÄCHINGER, H. P.** Crystal structure of human collagen XVIII trimerization domain: A novel collagen trimerization Fold. *J Mol Biol,* 2009, vol. 392, 787-802
- **CHEN, C. ; ZHANG, Y. ; HUANG, J. ; YIN, P. ; CHENG, Z. ; WU, J. ; CHEN, S. ; ZHANG, Y. ; CHEN, B. ; LU, M.** Favipiravir versus Arbidol for COVID-19. *A Randomized Clinical Trial,* 2020
- **CHEN, X. ; ZHAO, B. ; QU, Y. ; CHEN, Y. ; XIONG, J. ; FENG, Y. ; MEN, D. ; HUANG, Q. ; LIU, Y. ; YANG, B.** Detectable serum SARS-CoV-2 viral load (RNAaemia) is closely correlated with drastically elevated interleukin 6 (IL-6) level in critically ill COVID-19 patients. *Clin Infect Dis,* 2020
- **CHUG, H. ; TRAKHANOV, S. ; HÜLSMANN, B. B. ; PLEINER, T. ; GORLICH, D.** Crystal structure of the metazoan Nup62•Nup58•Nup54 nucleoporin complex. *Science,* 2015, vol. 350, 106-110
- **CORMAN, V. M. ; LANDT, O. ; KAISER, M. ; MOLENKAMP, R. ; MEIJER, A. ; CHU, D. K. ; BLEICKER, T. ; BRÜNINK, S. ; SCHNEIDER, J. ; SCHMIDT, M. L.** Detection of 2019 novel coronavirus (2019-nCoV) by real-time RT-PCR. *Euro Surveill,* 2020, 25
- **DUAN, K. ; LIU, B. ; LI, C. ; ZHANG, H. ; YU, T. ; QU, J. ; ZHOU, M. ; CHEN, L. ; MENG, S. ; HU, Y.** Effectiveness of convalescent plasma therapy in severe COVID-19 patients. *Proceedings of the National Academy of Sciences,* 2020, vol. 117, 9490-9496
- **EMENY, J. M. ; MORGAN, M. J.** Regulation of the interferon system: evidence that Vero cells have a genetic defect in interferon production. *J Gen Virol,* 1979, vol. 43, 247-252
- **HOEFMAN, S. ; OTTEVAERE, I. ; BAUMEISTER, J. ; SARGENTINI-MAIER, M.** Pre-Clinical Intravenous Serum Pharmacokinetics of Albumin Binding and Non-Half-Life Extended Nanobodies®. *Antibodies,* 2015, vol. 4, 141-156
- **KLIKS, S. C. ; NISALAK, A. ; BRANDT, W. E. ; WAHL, L. ; BURKE, D. S.** Antibody-dependent enhancement of dengue virus growth in human monocytes as a risk factor for dengue hemorrhagic fever. *Am J Trop Med Hyg,* 1989, vol. 40, 444-451
- **LI, W. ; MOORE, M. J. ; VASILIEVA, N. ; SUI, J. ; WONG, S. K. ; BERNE, M. A. ; SOMASUNDARAN, M. ; SULLIVAN, J. L. ; LUZURIAGA, K. ; GREENOUGH, T. C.** Angiotensin-converting enzyme 2 is a functional receptor for the SARS coronavirus. *Nature,* 2003, vol. 426, 450-454
- **LITTAUA, R. ; KURANE, I. ; ENNIS, F. A.** Human IgG Fc receptor II mediates antibody-dependent enhancement of dengue virus infection. *J Immunol,* 1990, vol. 144, 3183-3186
- **MAGRO, C. ; MULVEY, J. J. ; BERLIN, D. ; NUOVO, G. ; SALVATORE, S. ; HARP, J. ; BAXTER-STOLTZFUS, A. ; LAURENCE, J.** Complement associated microvascular injury and thrombosis in the pathogenesis of severe COVID-19 infection: a report of five cases. *Transl Res,* 2020
- **MEHTA, P. ; MCAULEY, D. F. ; BROWN, M. ; SANCHEZ, E. ; TATTERSALL, R. S. ; MANSON, J. J. ; HLH ACROSS SPECIALITY COLLABORATION, U. K.** COVID-19: consider cytokine storm syndromes and immunosuppression. *Lancet,* 2020, vol. 395, 1033-1034
- **MURPHY, K. ; WEAVER, C.** Janeway's Immunobiology. Garland Science, 2016
- **PLEINER, T. ; BATES, M. ; GORLICH, D.** A toolbox of anti-mouse and anti-rabbit IgG secondary nanobodies. *J Cell Biol,* 2018, vol. 217, 1143-1154

- **PLEINER, T. ; BATES, M. ; TRAKHANOV, S. ; LEE, C. T. ; SCHLIEP, J. E. ; CHUG, H. ; BÖHNING, M. ; STARK, H. ; URLAUB, H. ; GORLICH, D.** Nanobodies: site-specific labeling for super-resolution imaging, rapid epitope-mapping and native protein complex isolation. *Elife,* 2015, vol. 4, e11349
- **RASMUSSEN, S. G. ; CHOI, H. J. ; FUNG, J. J. ; PARDON, E. ; CASAROSA, P. ; CHAE, P. S. ; DEVREE, B. T. ; ROSENBAUM, D. M. ; THIAN, F. S. ; KOBILKA, T. S.** Structure of a nanobody-stabilized active state of the β(2) adrenoceptor. *Nature,* 2011, vol. 469, 175-180
- **WANG, C. ; LI, W. ; DRABEK, D. ; OKBA, N. M. A. ; VAN HAPEREN, R. ; OSTERHAUS, A. D. M. E. ; VAN KUPPEVELD, F. J. M. ; HAAGMANS, B. L. ; GROSVELD, F. ; BOSCH, B. J.** A human monoclonal antibody blocking SARS-CoV-2 infection. *Nat Commun,* 2020, vol. 11, 2251
- **WIRZ, J. A. ; BOUDKO, S. P. ; LERCH, T. F. ; CHAPMAN, M. S. ; BÄCHINGER, H. P.** Crystal structure of the human collagen XV trimerization domain: A potent trimerizing unit common to multiplexin collagens. *Matrix Biology,* 2011, vol. 30, 9-15
- **WRAPP, D. ; WANG, N. ; CORBETT, K. S. ; GOLDSMITH, J. A. ; HSIEH, C. L. ; ABIONA, O. ; GRAHAM, B. S. ; MCLELLAN, J. S.** Cryo-EM structure of the 2019-nCoV spike in the prefusion conformation. *Science,* 2020, vol. 367, 1260-1263
- **ZHANG, L. ; JACKSON, C. B. ; MOU, H. ; OJHA, A. ; RANGARAJAN, E. S. ; IZARD, T. ; FARZAN, M. ; CHOE, H.** *The D614G mutation in the SARS-CoV-2 spike protein reduces S1 shedding and increases infectivity. bioRxiv,* 2020, https://doi.org/10.1101/2020.06.12.148726